(19) Europäisches Patentamt
European Patent Office
Office européen des brevets

(11) **EP 3 137 626 B1**

(12) **EUROPEAN PATENT SPECIFICATION**

(45) Date of publication and mention
of the grant of the patent:
**02.12.2020 Bulletin 2020/49**

(21) Application number: **15717179.4**

(22) Date of filing: **21.04.2015**

(51) Int Cl.:
***C12Q 1/6809*** (2018.01)     ***C12Q 1/6886*** (2018.01)

(86) International application number:
**PCT/EP2015/058614**

(87) International publication number:
**WO 2015/165779 (05.11.2015 Gazette 2015/44)**

(54) **SMALL NCRNAS AS BIOMARKERS**

KLEINE NCRNAS ALS BIOMARKER

PETITS ARNNC SERVANT DE BIOMARQUEURS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.05.2014 EP 14166802**

(43) Date of publication of application:
**08.03.2017 Bulletin 2017/10**

(73) Proprietors:
 • **Stichting VUmc
  1081 HV Amsterdam (NL)**
 • **Universidad de Granada
  18071 Granada (ES)**

(72) Inventors:
 • **PEGTEL, Dirk Michiel
  1081 HV Amsterdam (NL)**
 • **KOPPERS-LALIC, Danijela
  1081 HV Amsterdam (NL)**
 • **WURDINGER, Tom
  1081 HV Amsterdam (NL)**
 • **BIJNSDORP, Irene
  1081 HV Amsterdam (NL)**

 • **HACKENBERG, Michael
  18.071 Granada (ES)**

(74) Representative: **V.O.
  P.O. Box 87930
  Carnegieplein 5
  2508 DH Den Haag (NL)**

(56) References cited:
  **WO-A1-2011/024157**

 • **SUNG-CHOU LI ET AL: "miRNA arm selection and isomiR distribution in gastric cancer", BMC GENOMICS, BIOMED CENTRAL LTD, LONDON, UK, vol. 13, no. Suppl 1, 17 January 2012 (2012-01-17), page S13, XP021093261, ISSN: 1471-2164, DOI: 10.1186/1471-2164-13-S1-S13**
 • **LEUNG CHUNG-MAN ET AL: "Comprehensive microRNA profiling of prostate cancer cells after ionizing radiation treatment", ONCOLOGY REPORTS, vol. 31, no. 3, March 2014 (2014-03), pages 1067-1078, XP002728158, ISSN: 1021-335X**

Remarks:
  The file contains technical information submitted after the application was filed and not included in this specification

**Description**

FIELD OF THE INVENTION

The present disclosure provides small ncRNAs as biomarkers for classifying the health status of an individual. The disclosure also provides screening methods for identifying ncRNA biomarkers.

BACKGROUND OF THE INVENTION

The human genome encodes for a vast amount of small non-protein-coding RNA (ncRNAs) transcripts. Multiple ncRNA classes have been described including the highly abundant transfer RNAs (tRNAs), ribosomal RNAs (rRNAs), small nucleolar RNAs (snoRNAs), micro-RNAs (miRNAs), small interfering RNAs (siRNAs), small nuclear RNAs (snRNAs), and piwi-interacting RNAs (piRNAs) (Amaral et al., 2008; Martens-Uzunova et al., 2013). MiRNAs act as translational repressors by binding to target mRNAs at sites with adequate sequence complementary (Ameres et al., 2007), while the highly abundant cytoplasmic Y RNAs function in RNA quality control by affecting the subcellular location of Ro proteins (Sim et al., 2009). The repressive activity of mature miRNAs on mRNA translation is shared by other classes of ncRNAs, including siRNAs and endo-siRNAs, in addition to piRNAs that silence retrotransposons at defined subcellular locations (Chuma and Pillai, 2009). MiRNA activity relies on sufficient levels of abundance in the cytoplasm, and interaction with RNA-induced silencing complexes (RISC) localized at endosomal membranes (Gibbings et al., 2009; Lee et al., 2009a), whereas low abundant miRNAs have less impact on translational repression. As a consequence, subtle alterations in the levels of certain miRNA may already influence cellular processes, while strong perturbations can cause disease. Besides abundance, interactions with (RISC) proteins but also RNA partners and correct subcellular localization are interrelated factors that control miRNA physiology (Mullokandov et al., 2012; Wee et al., 2012).

MiRNAs are a class of small, 22-25 nucleotide, non-coding regulatory RNAs that control key aspects of post-translational gene regulation and function in a highly specific manner. Since miRNAs act as specific gene regulators and because their expression is frequently perturbed in cancer development, their use as biomarkers has been investigated. Overexpression of certain miRNA (oncomirs) such as miR-21 or lack of expression, such as the miR200 family, seem to correlate with clinically aggressive or metastatic disease outcome. In chronic lymphocytic leukemia (CLL), circulating miRNAs have been used for disease stratification and predicted the response to therapeutic intervention. Li et al. (BMC Genomics 2012 13:S1-S13) describes methods for determining isomiR types in gastric tissue. Leung et al. (Oncology Reports 2014 31:1067-1078) describes microRNA profiling in PC3 prostate cancer cells. WO2011024157 describes the sequencing of isomiRs from solid tumor samples.

Nevertheless, although miRNA profiling is a relatively standard technique, widespread clinical implementation of circulating miRNAs has been hampered due to conflicting data. There is a need for ncRNA biomarkers which can better predict the health status of an individual.

SUMMARY OF THE INVENTION

One aspect of the disclosure provides a method for identifying a small non-coding RNA (ncRNA) biomarker pair, the method comprising

- determining for an ncRNA the ratio of two different varieties of said ncRNA in a first bodily fluid sample obtained from a first individual reference and in a second bodily fluid sample obtained from a second individual reference, wherein said first individual reference has an altered health status from said second individual reference,
- comparing the ratios from said first and second bodily fluid sample, and
- identifying the two different varieties of said ncRNA as a biomarker pair

when the ratio is altered between said first and second bodily fluid sample, wherein the first variety is selected from the canonical ncRNA and the ncRNA with a 3' non-templated nucleotide addition and the second variety is the ncRNA with a 3' non-templated nucleotide addition, and
wherein when said ncRNA is miRNA, the first variety is selected from canonical ncRNA, the ncRNA trimmed at the 5' or 3' end, and the ncRNA with a 3' non-templated nucleotide addition, optionally trimmed at the 5' or 3' end and the second variety is selected from ncRNA trimmed at the 5' or 3' end and ncRNA with a 3' non-templated nucleotide addition, optionally trimmed at the 5' or 3' end.

Alternatively stated, wherein the first variety is selected from the canonical ncRNA, the ncRNA trimmed at the 5' or 3' end, and the ncRNA with a 3' non-templated nucleotide addition optionally trimmed at the 5' or 3' end; wherein the second variety is selected from the ncRNA trimmed at the 5' or 3' end and the ncRNA with a 3' non-templated nucleotide addition optionally trimmed at the 5' or 3' end;
wherein when said ncRNA is not an miRNA the first variety is selected from the canonical ncRNA and the ncRNA with a 3' non-templated nucleotide addition and the second variety is the ncRNA with a 3' non-templated nucleotide addition.

Preferably, the first and second varieties are selected from the canonical ncRNA; the ncRNA trimmed at the 5' or 3' end and/or extended at the 5' or 3' end; and the ncRNA with a 3' non-templated nucleotide addition, optionally trimmed at the 5' or 3' end or extended at the 5' or 3' end;

wherein when said ncRNA is not an miRNA the first variety is selected from the canonical ncRNA and the ncRNA with a 3' non-templated nucleotide addition and the second variety is the ncRNA with a 3' non-templated nucleotide addition.

Preferably, the method comprises determining the quantity of two different varieties of said ncRNA in a first bodily fluid sample and determining the quantity of two different varieties of said ncRNA in a second bodily fluid sample.

Preferably, the first individual reference is from one or more healthy individuals and said second individual reference is from one or more individuals having a disorder, preferably wherein said disorder is prostate cancer.

Preferably, the first individual reference is from an individual having a disorder and said second individual reference is from the same individual following treatment of the disorder.

In preferred aspects of the methods disclosed herein, the biomarker pair ratio is determined by quantifying the two different variety in each sample and determining the relationship between the two quantities. Preferably, the variety are quantified using deep sequencing (RNA-seq).

A further aspect of the disclosure provides for a method for collecting data for classifying the health status of an individual using a small non-coding RNA (ncRNA) biomarker pair. The method comprises determining the ratio of the biomarker pair in a bodily fluid sample from an individual and comparing the ratio to the ratio of the biomarker pair in a bodily fluid from a reference sample (such as the second individual reference described above),

wherein the presence or absence in a difference in the ratio between the individual and the reference sample assists in classifying the health status of said individual, wherein the biomarker pair consists of two different varieties of an ncRNA wherein the first variety is selected from the canonical ncRNA and the ncRNA with a 3' non-templated nucleotide addition and the second variety is the ncRNA with a 3' non-templated nucleotide addition, and

wherein when said ncRNA is miRNA, the first variety is selected from canonical ncRNA, the ncRNA trimmed at the 5' or 3' end, and the ncRNA with a 3' non-templated nucleotide addition, optionally trimmed at the 5' or 3' end and the second variety is selected from ncRNA trimmed at the 5' or 3' end and ncRNA with a 3' non-templated nucleotide addition, optionally trimmed at the 5' or 3' end.

Alternatively stated, wherein the first variety is selected from the canonical ncRNA, the ncRNA trimmed at the 5' or 3' end, and the ncRNA with a 3' non-templated nucleotide addition optionally trimmed at the 5' or 3' end; wherein the second variety is selected from the ncRNA trimmed at the 5' or 3' end and the ncRNA with a 3' non-templated nucleotide addition optionally trimmed at the 5' or 3' end;

wherein when said ncRNA is not an miRNA the first variety is selected from the canonical ncRNA and the ncRNA with a 3' non-templated nucleotide addition and the second variety is the ncRNA with a 3' non-templated nucleotide addition.

Preferably, the first and second varieties are selected from the canonical ncRNA; the ncRNA trimmed at the 5' or 3' end and/or extended at the 5' or 3' end; and the ncRNA with a 3' non-templated nucleotide addition, optionally trimmed at the 5' or 3' end or extended at the 5'or 3'end;

wherein when said ncRNA is not an miRNA the first variety is selected from the canonical ncRNA and the ncRNA with a 3' non-templated nucleotide addition and the second variety is the ncRNA with a 3' non-templated nucleotide addition.

The data obtained in such a method can be used either alone or in combination with other factors (e.g., the presence of additional biomarkers, clinical symptoms in a patient, etc.) to diagnose the individual as having a disorder.

A further aspect of the disclosure provides for a method for classifying the health status of an individual using a small non-coding RNA (ncRNA) biomarker pair, the method comprising determining the ratio of the biomarker pair in a bodily fluid sample from said individual and comparing the ratio to the ratio of the biomarker pair in a bodily fluid from a reference sample,

wherein the presence or absence in a difference in the ratio between the individual and the reference sample classifies the health status of said individual, wherein the biomarker pair consists of two different variety of an ncRNA wherein the first variety is selected from the canonical ncRNA and the ncRNA with a 3' non-templated nucleotide addition and the second variety is the ncRNA with a 3' non-templated nucleotide addition, and

wherein when said ncRNA is miRNA, the first variety is selected from canonical ncRNA, the ncRNA trimmed at the 5' or 3' end, and the ncRNA with a 3' non-templated nucleotide addition, optionally trimmed at the 5' or 3' end and the second variety is selected from ncRNA trimmed at the 5' or 3' end and ncRNA with a 3' non-templated nucleotide addition, optionally trimmed at the 5' or 3' end.

Alternatively stated, wherein the first variety is selected from the canonical ncRNA, the ncRNA trimmed at the 5' or 3' end, and the ncRNA with a 3' non-templated nucleotide addition optionally trimmed at the 5' or 3' end; wherein the second variety is selected from the ncRNA trimmed at the 5' or 3' end and the ncRNA with a 3' non-templated nucleotide addition optionally trimmed at the 5' or 3' end;

wherein when said ncRNA is not an miRNA the first variety is selected from the canonical ncRNA and the ncRNA with a 3' non-templated nucleotide addition and the second variety is the ncRNA with a 3' non-templated nucleotide addition.

Preferably, the first and second varieties are selected from the canonical ncRNA; the ncRNA trimmed at the 5' or 3' end and/or extended at the 5' or 3' end; and the ncRNA with a 3' non-templated nucleotide addition, optionally trimmed at the 5' or 3' end or extended at the 5'or 3'end;

wherein when said ncRNA is not an miRNA the first variety is selected from the canonical ncRNA and the ncRNA with a 3' non-templated nucleotide addition and the second variety is the ncRNA with a 3' non-templated nucleotide addition.

Preferably, the methods comprise determining the quantity of the biomarker pair in said individual sample.

Preferably, the reference sample is from one or more healthy individuals.

Preferably, the reference sample is from one or more individuals having a disorder. Preferably the disorder as disclosed herein is cancer, more preferably prostate cancer, breast cancer, cHL, testicular cancer or colorectal cancer. Preferably, the disorder is prostate cancer, breast cancer, or cHL. Preferably the disorder is Alzheimer's disease.

Preferably, the reference sample is from one or more individuals having a good response or poor response to treatment for a disorder.

The ncRNA is selected from transfer RNA (tRNA), microRNA (miRNA), and Y RNA, preferably wherein said ncRNA is selected from miRNA.

In preferred aspects of the methods disclosed herein, the bodily fluid is urine or blood.

In preferred aspects of the methods disclosed herein, the two different varieties are selected from canonical and 3' NTA-A; canonical and 3' NTA-G; canonical and 3' NTA-C; canonical and 3' NTA-U; 3' NTA-G and 3' NTA-C; 3' NTA-G and 3' NTA-U; 3' NTA-G and 3' NTA-A; 3' NTA-C and 3' NTA-U; 3' NTA-C and 3' NTA-A; and 3' NTA-U and 3' NTA-A; canonical and 5' trimmed; canonical and 3' trimmed; 5' trimmed and 3' NTA-C; 5' trimmed and 3' NTA-U; 5' trimmed and 3' NTA-A; 5' trimmed and 3' NTA-G; 3' trimmed and 3' NTA-C; 3' trimmed and 3' NTA-U; 3' trimmed and 3' NTA-A; 3' trimmed and 3' NTA-G; and 3' trimmed and 5' trimmed; extended and 3' NTA-A; extended and 3' NTA-G; extended and 3' NTA-C; extended and 3' NTA-U; extended and 5' trimmed; extended and 3' trimmed; 5' or 3' trimmed and 5'or 3'extended; (trimmed and/or extended) and 3' NTA-U; (trimmed and/or extended) and 3' NTA-A; (trimmed and/or extended) and 3' NTA-G; (trimmed and/or extended) and 3' NTA-C; (trimmed and/or extended) and canonical.

In preferred aspects of the methods disclosed herein, the each ncRNA of said biomarker pair comprises at least 16 nucleotides.

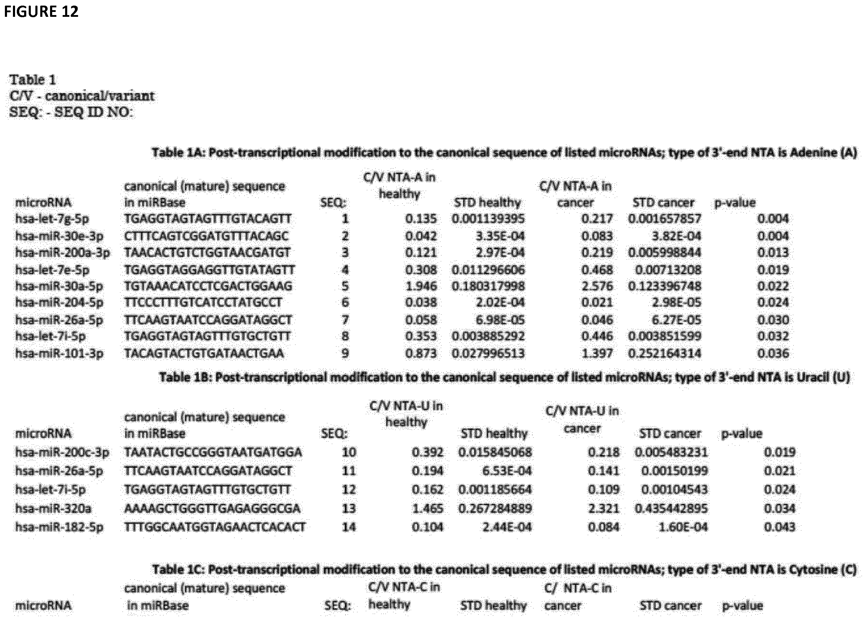

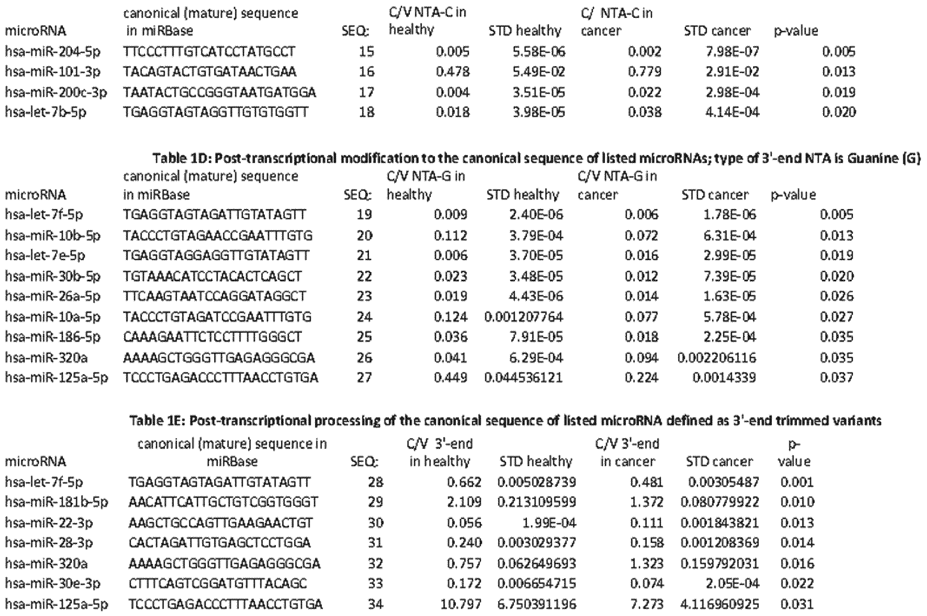

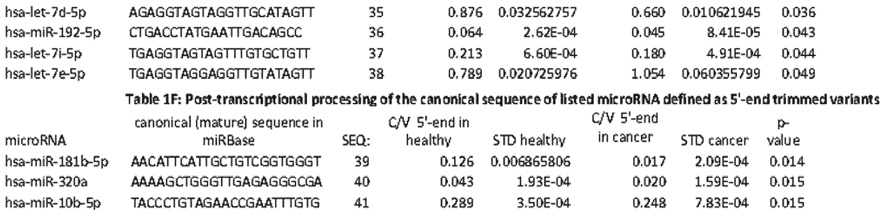

Figure 12 provides an exemplary embodiment of biomarker pairs of the invention. Table 1 is understood to disclose that one biomarker is the canonical sequence and the other biomarker is the respective 3'NTA-A variety, e.g., hsa-let-7g-5p canonical and hsa-let-7g-5p 3'NTA-A. Table 1A thus discloses 9 separate biomarker pairs. Table 1B discloses 5 separate biomarker pairs (e.g., hsa-miR-200c-3p canonical and hsa-miR-200c-3p 3'NTA-U ; Table 1C discloses 4 separate biomarker pairs (e.g., hsa-miR-204-5p canonical and hsa-miR-204-5p 3'NTA-C); Table 1D discloses 9 separate biomarker pairs (e.g., hsa-let-7f-5p canonical and hsa-let-7f-5p 3'NTA-G); Table 1E discloses 11 separate biomarker pairs (e.g., hsa-let-7f-5p canonical and hsa-let-7f-5p 3'trimmed ; Table 1F discloses 3 separate biomarker pairs (e.g., hsa-miR-181b-5p canonical and hsa-miR-181b-5p 5'trimmed).

In preferred aspects of the methods disclosed herein, the biomarker pair is selected from one of the biomarker pairs depicted in Figure 12. Preferably, the biomarker pair is selected from Table 1A. Preferably, the biomarker pair is selected from Table 1B. Preferably, the biomarker pair is selected from Table 1C. Preferably, the biomarker pair is selected from Table 1D. Preferably, the biomarker pair is selected from Table 1E. Preferably, the biomarker pair is selected from Table 1F.

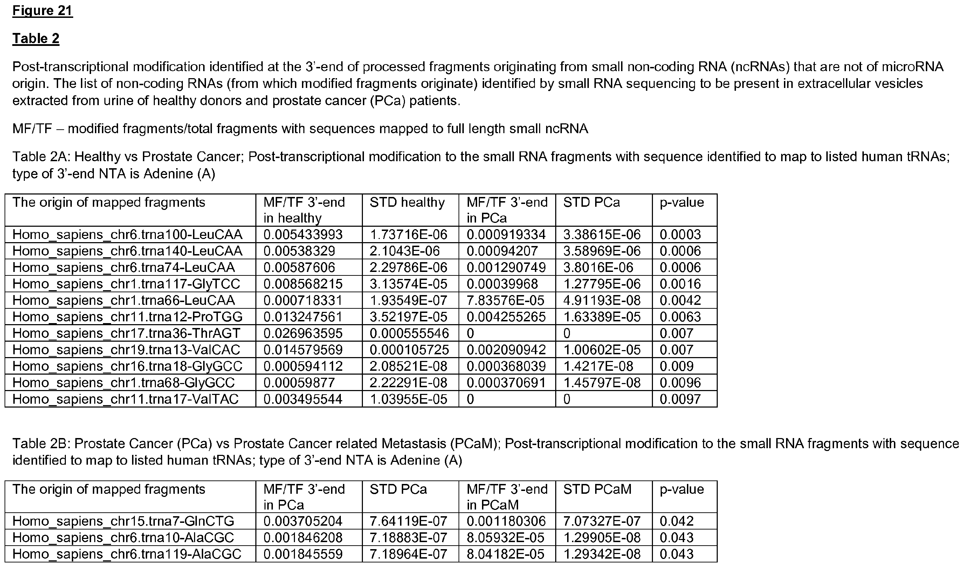

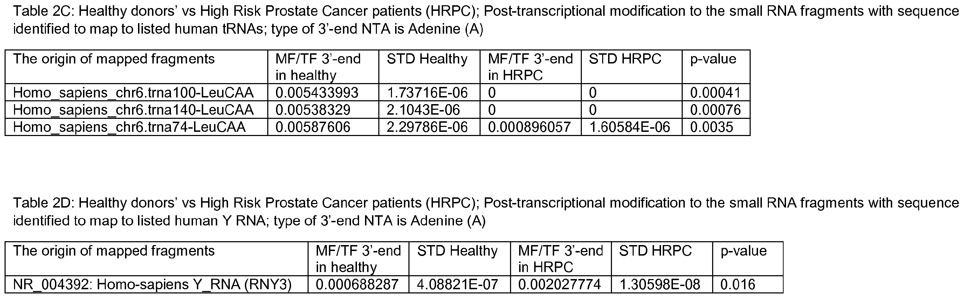

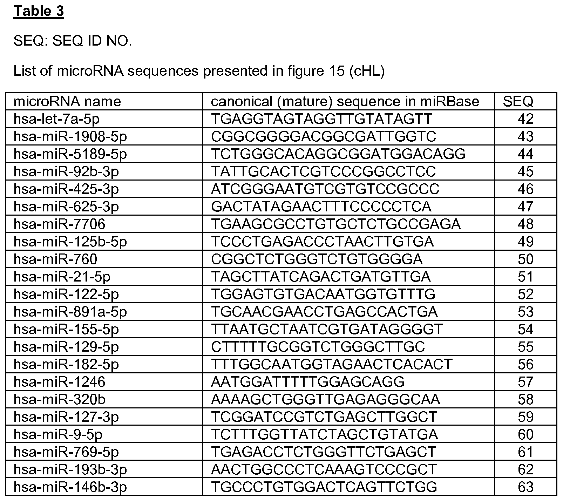

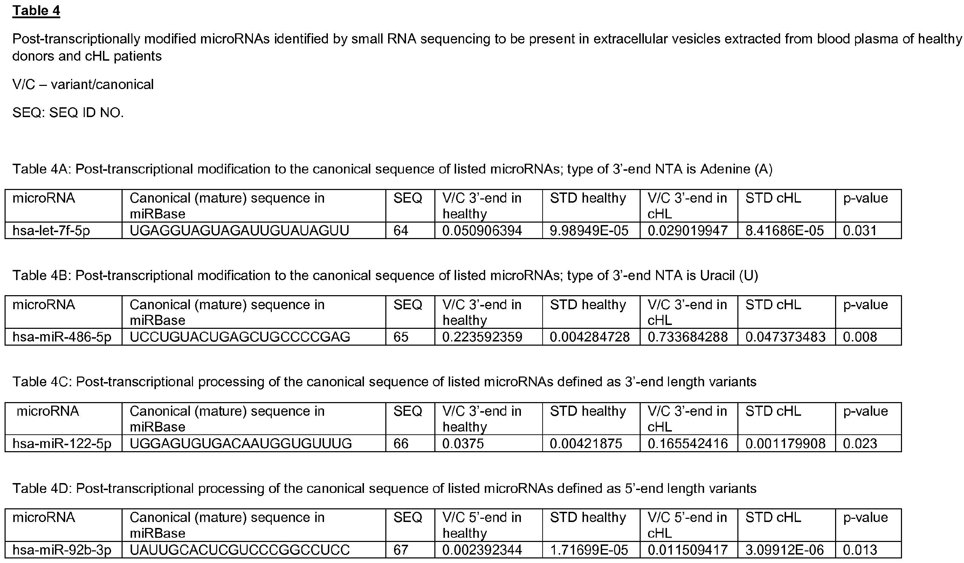

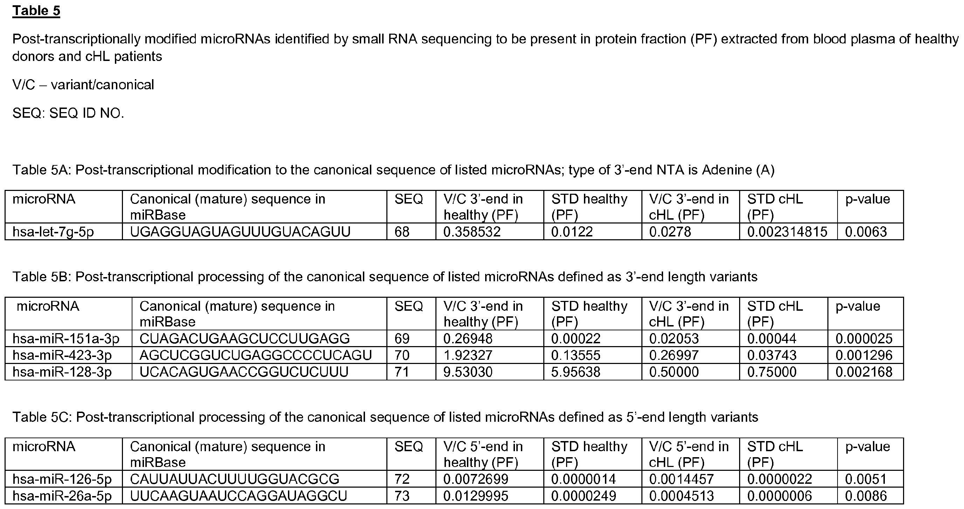

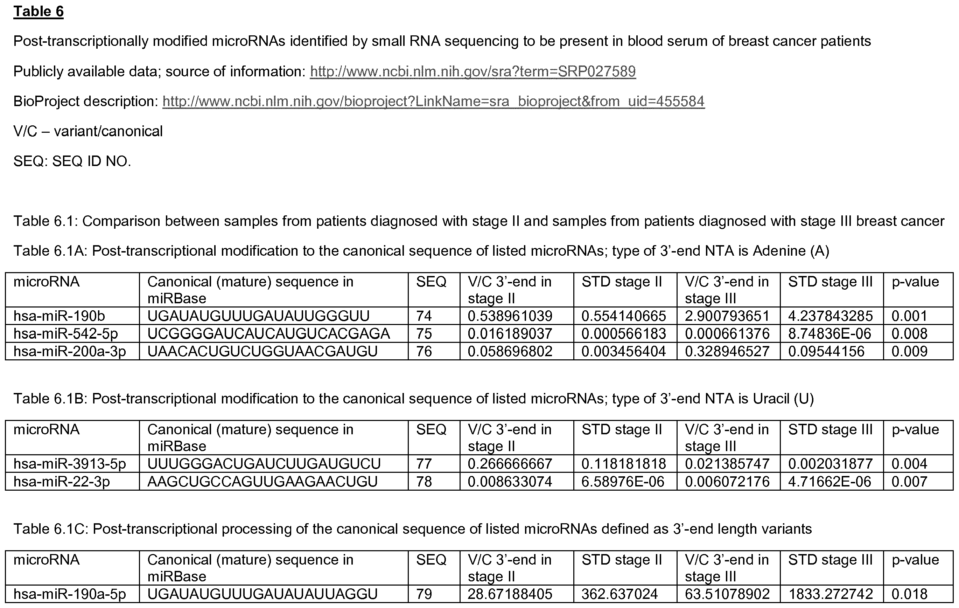

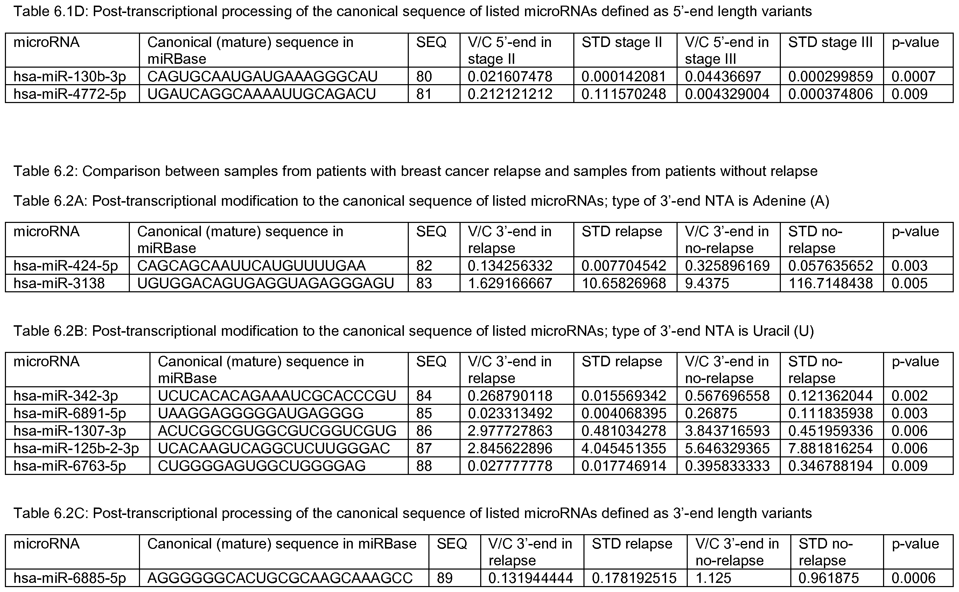

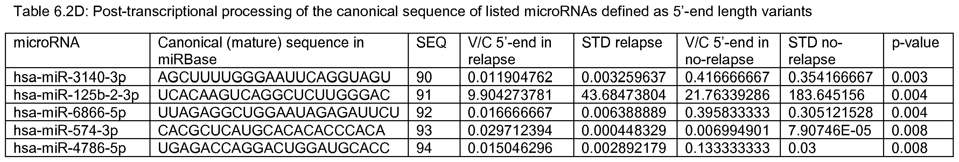

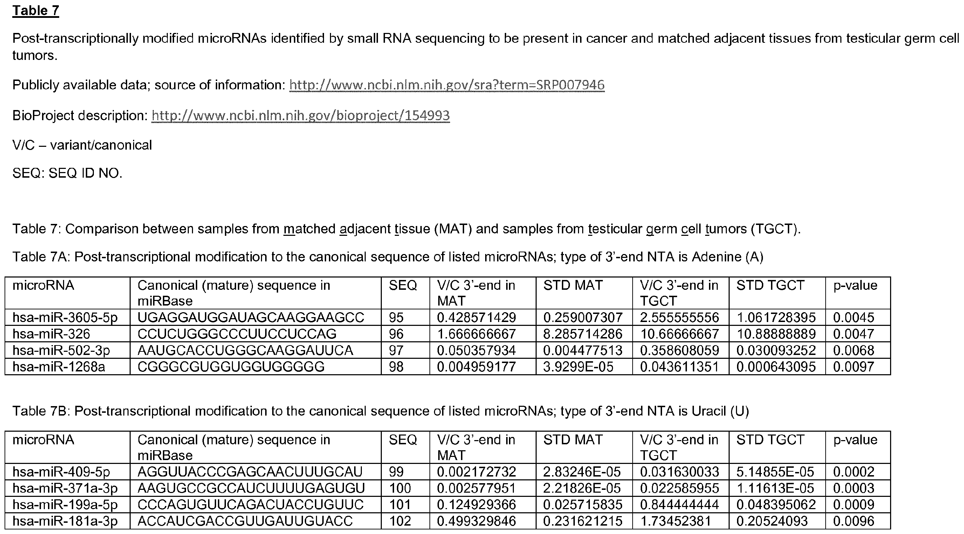

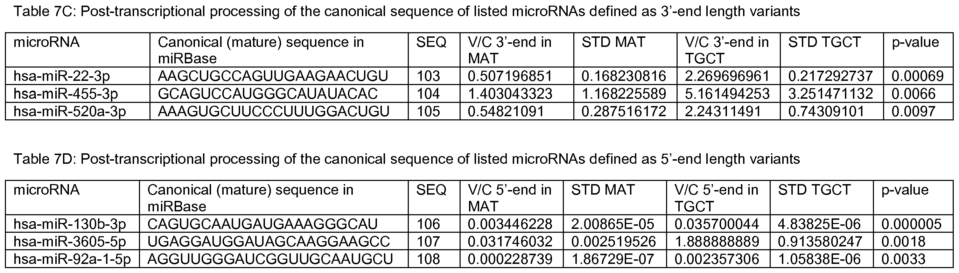

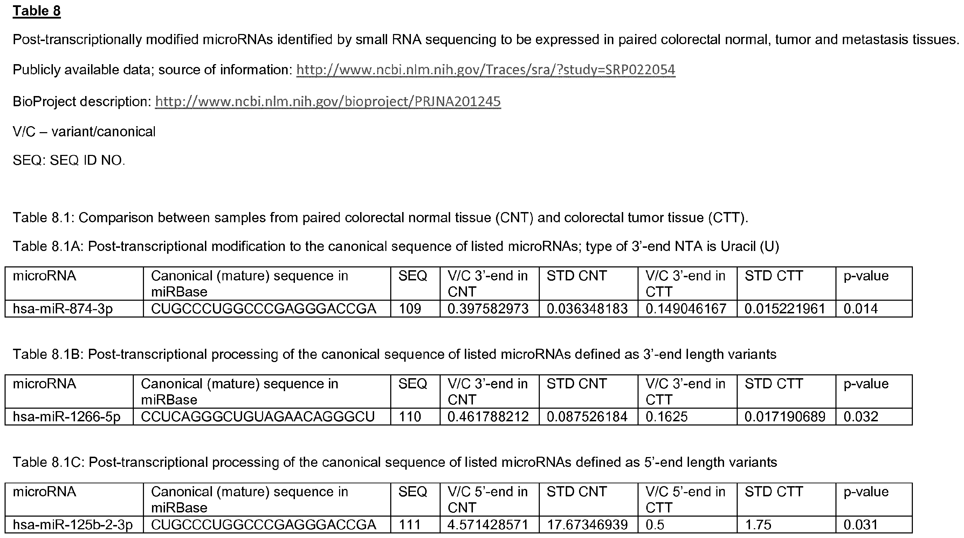

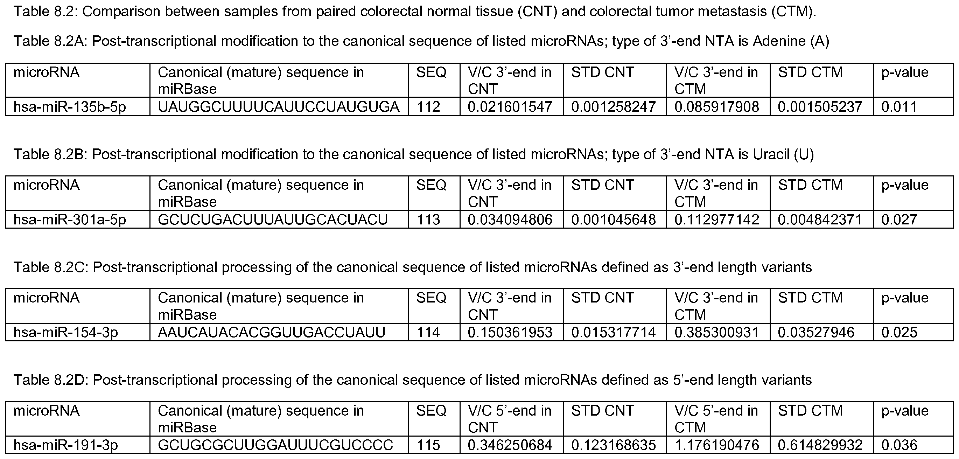

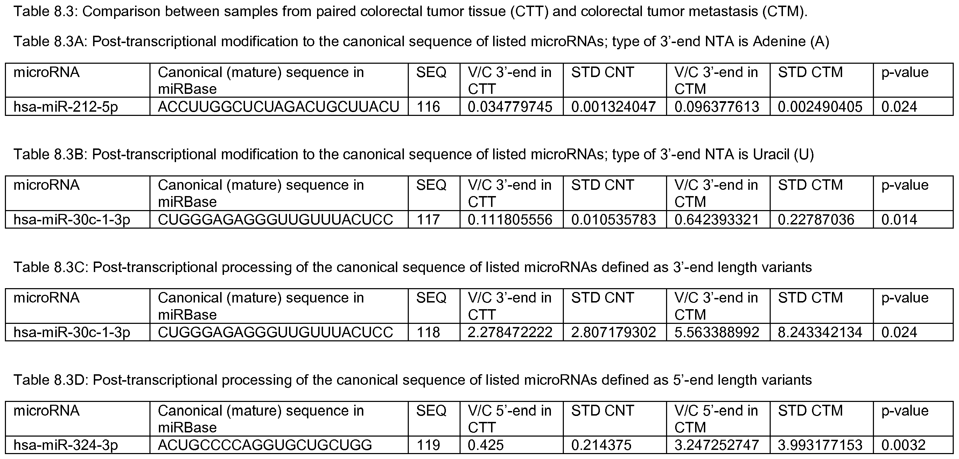

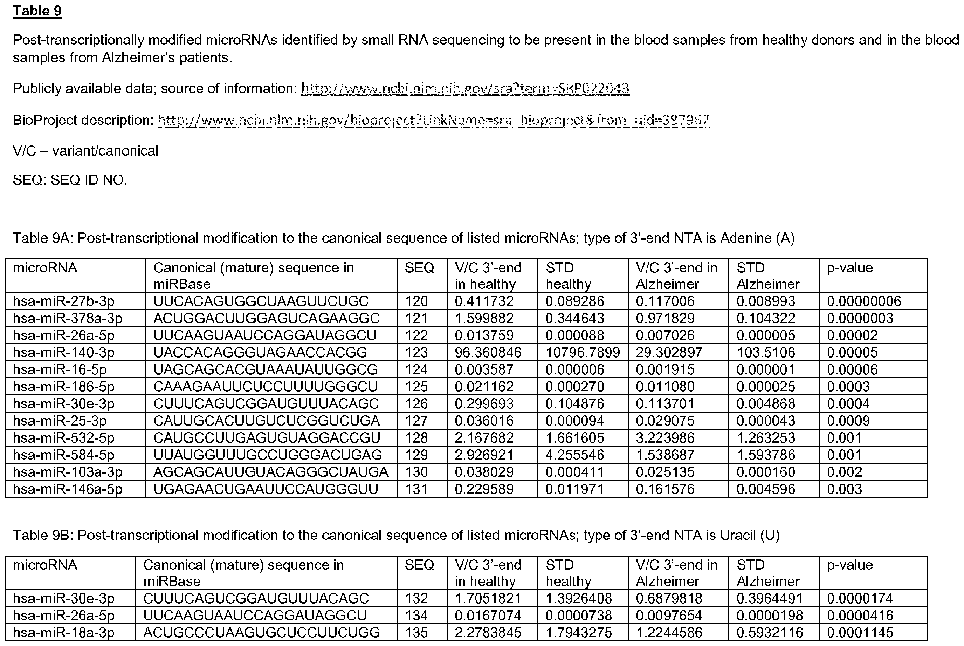

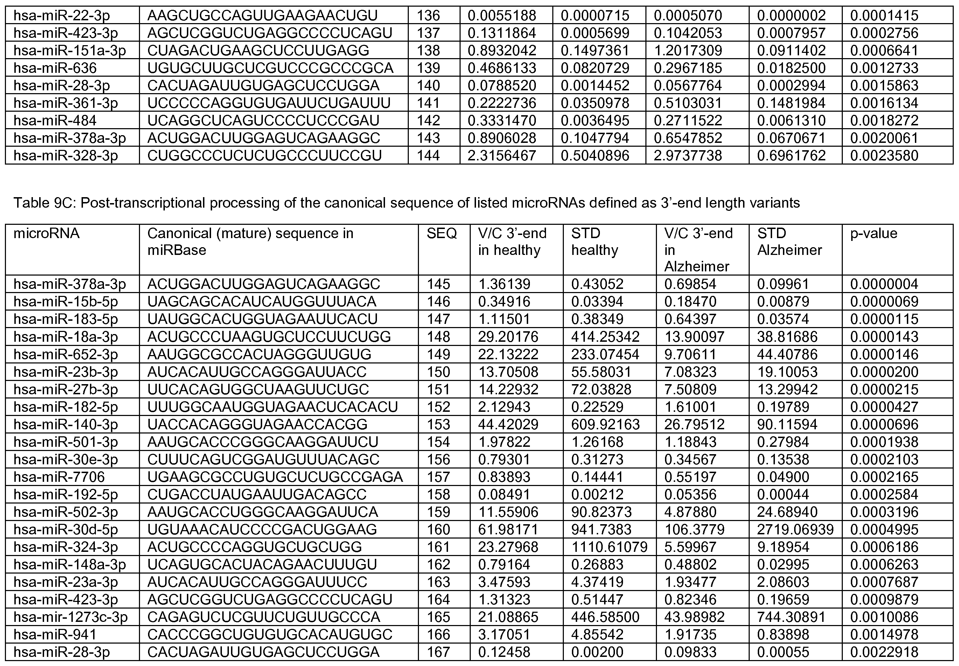

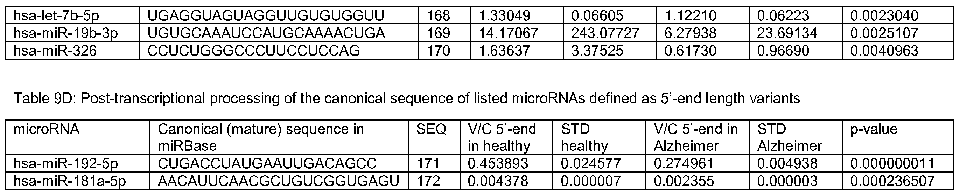

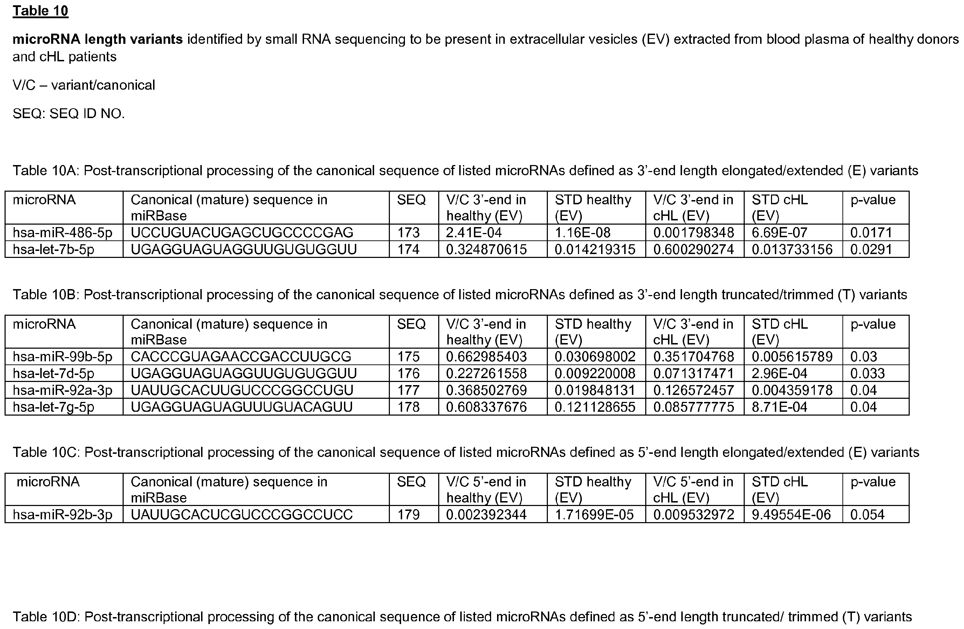

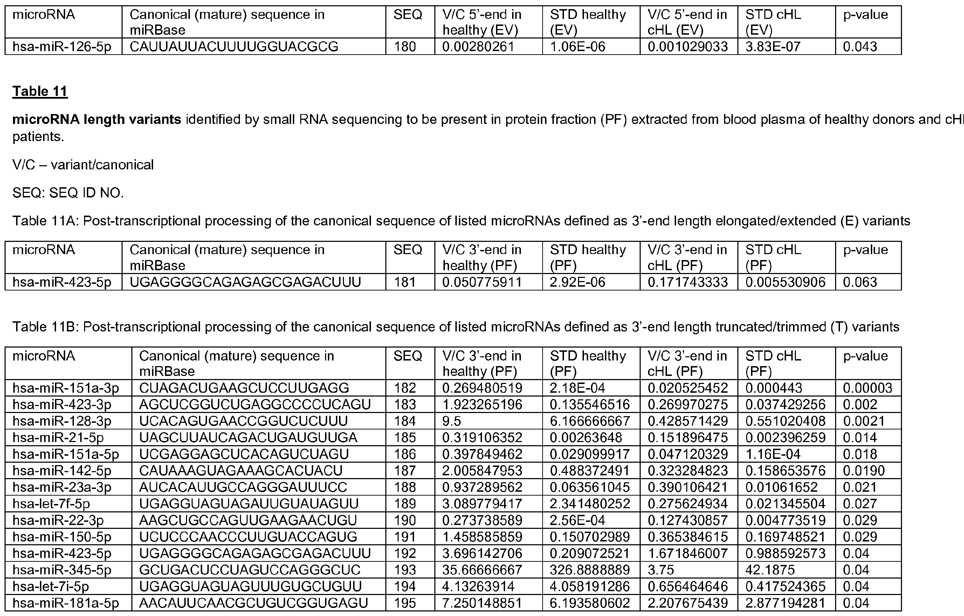

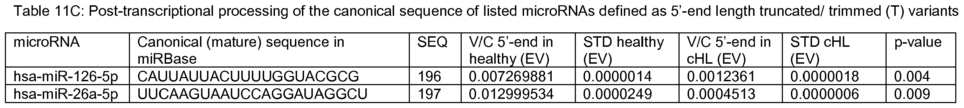

Figure 21 provides further exemplary embodiments of biomarkers of the invention. Table 2 depicts tRNAs biomarker pairs useful in characterizing prostate cancer. Table 2 is understood to disclose that one biomarker is the canonical sequence and the other biomarker is the respective variant. In particular, these biomarker pairs are useful in methods which determine the biomarker pairs in urine. Tables 4 and 10 depict miRNA biomarker pairs useful in characterizing cHL. In particular, these biomarker are useful in methods which determine the biomarker pairs in exosomal vesicles extracted from blood. Tables 4 and 10 are understood to disclose that one biomarker is the canonical sequence and the other biomarker is the respective variant. Tables 5 and 11 depict miRNA biomarker pairs useful in characterizing cHL. In particular, these biomarker pairs are useful in methods which determine the biomarker pairs in the protein fraction extracted from blood. Tables 5 and 11 are understood to disclose that one biomarker is the canonical sequence and the other biomarker is the respective variant. Table 6 depicts miRNA biomarker pairs useful in characterizing breast cancer. In particular, these biomarker pairs are useful in methods which determine the biomarker pairs in blood samples. Table 6 is understood to disclose that one biomarker is the canonical sequence and the other biomarker is the respective variant. Table 7 depicts miRNA biomarker pairs useful in characterizing testicular germ cell tumors. Table 7 is understood to disclose that one biomarker is the canonical sequence and the other biomarker is the respective variant. Table 8 depicts miRNA biomarker pairs useful in colorectal cancer. Table 8 is understood to disclose that one biomarker is the canonical sequence and the other biomarker is the respective variant. Table 9 depicts miRNA biomarker pairs useful in Alzheimer's disease. Table 9 is understood to disclose that one biomarker is the canonical sequence and the other biomarker is the respective variant. In particular, these biomarker pairs are useful in methods which determine the biomarker pairs in blood samples.

In preferred aspects of the methods disclosed herein, the biomarker pair is selected from table 1, 2, or 4-11, preferably from Tables 1, 2, 4, 5, 6, and 9-11.

Preferably, in the methods disclosed herein, the quantity of the two different varieties of said ncRNA are determined from exosomal vesicles purified from the bodily fluid samples. Preferably, in the methods disclosed herein, the quantity of the two different varieties of said ncRNA are determined from the protein fraction purified from the bodily fluid samples. Preferably, the exosomal vesicles or protein fraction are purified by subjecting the bodily fluid samples to size-exclusion chromatography (SEC).

In a further aspect of the invention, a method is provided for characterizing the status of classical Hodgkin's lymphoma (cHL) in an individual, the method comprising determining the quantity of one or more miRNAs from a bodily fluid sample from said individual and comparing the amount of the one or more miRNAs to a reference sample, wherein the difference in the presence or amount of the one or more miRNAs characterizes the status of the individual. A method is also provided for collecting data regarding the health status of an individual comprising determining the quantity of one or more miRNAs from a bodily fluid sample from said individual and comparing the amount of the one or more miRNAs to a reference sample. The data can be used for characterizing the status of classical Hodgkin's lymphoma (cHL) in the individual.

The one or more miRNAs used in these methods are selected table 3.

Preferably, the one or more miRNAs are selected from miR21-5p, let7a-5p, miR127-3p, and miR155-5p.

Table 3 depicts miRNAs useful in characterizing the status of classical Hodgkin's lymphoma (cHL) in an individual. In particular, these biomarker are useful in methods which determine the biomarker pairs in the protein fraction extracted from blood. Preferably, the method characterizes the status of cHL by determining whether an individual is afflicted with cHL.

Preferably, the reference sample is from one or more healthy individuals.

Preferably, the reference sample is from one or more individuals having cHL. Preferably, characterizing the status of cHL comprises determining the treatment efficacy in an individual receiving treatment for cHL. Preferably, the reference sample is from the same individual prior to receiving treatment for cHL.

Preferably, characterizing the status of cHL comprises determining the prognosis of an individual afflicted with cHL. Preferably, the reference sample is from one or more individuals having a good response to treatment for the disorder or from one or more individuals having a poor response to treatment for the disorder.

In preferred embodiments, the methods further comprise purifying exosomal vesicles from the bodily fluid sample and determining the quantity of the one or more miRNAs associated with the purified exosomal vesicles. In preferred embodiments, the methods further comprise purifying the protein fraction from the bodily fluid sample and determining the quantity of the one or more miRNAs associated with the purified exosomal vesicles. Preferably, said purification comprises subjecting the bodily fluid samples to size-exclusion chromatography (SEC). Preferably, the exosomal vesicles are isolated by obtaining the void volume fraction.

In preferred embodiments, the bodily fluid is blood.

Preferably, the methods disclosed herein are performed in vitro, in particular the step of quantifying the relevant biomarkers is performed in vitro.

BRIEF DESCRIPTION OF THE DRAWINGS

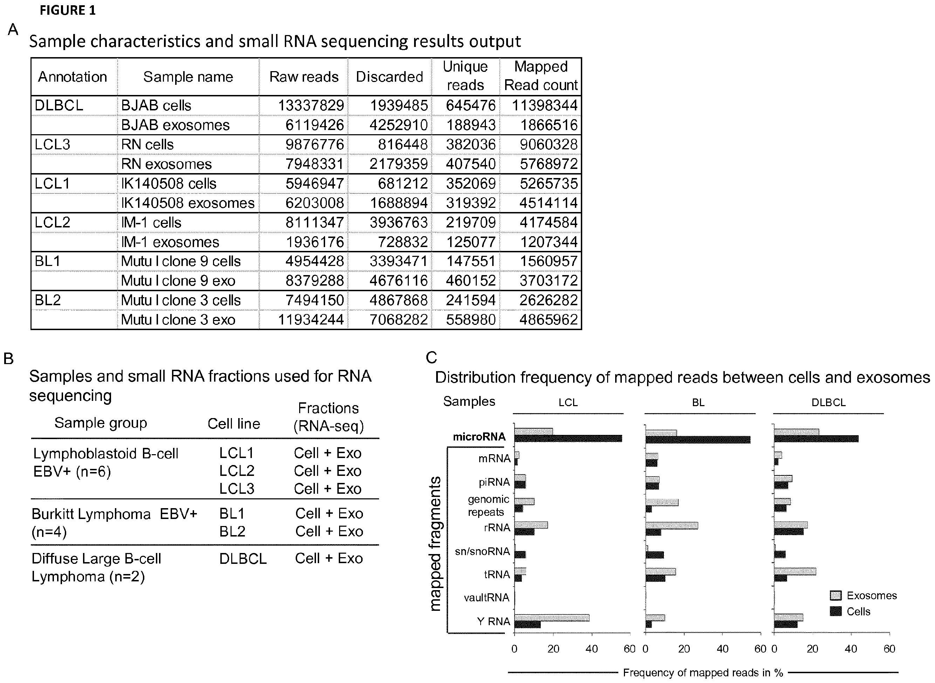

Figure 1. Small RNA repertoire from B cells and their exosomes.

A) Summary of samples characteristics and RNA-seq data. cDNA libraries were generated from cellular and exosomal small RNA fractions. Total number of reads from all libraries before and after mapping to genome of interest (hsg 19; human genome) is specified. To provide annotations to RNA elements that mapped to human genome, all mapped reads were analyzed against currently known databases: 1) mature and pre-miRNA sequences from miRBase version 19, 2) NCBI Reference Sequences human RefSeq genes downloaded 06/02/2013, 3) tRNA sequences from the genomic tRNA database, 4) Repeat Derived sequences detected by means of the RepeatMasker algorithm downloaded from UCSC and 5) piwiRNA (piRNAs) downloaded from the NCBI nucleotide database.

B) cDNA libraries were generated from cellular and exosomal small RNA fractions. Sample groups, cell lines and a type of sample fraction used to generate RNA sequencing libraries are indicated.

C) All mapped reads from cellular and exosomal fractions are grouped by annotation to cellular transcripts from which they originate and presented as distribution frequency of mapped reads in %.

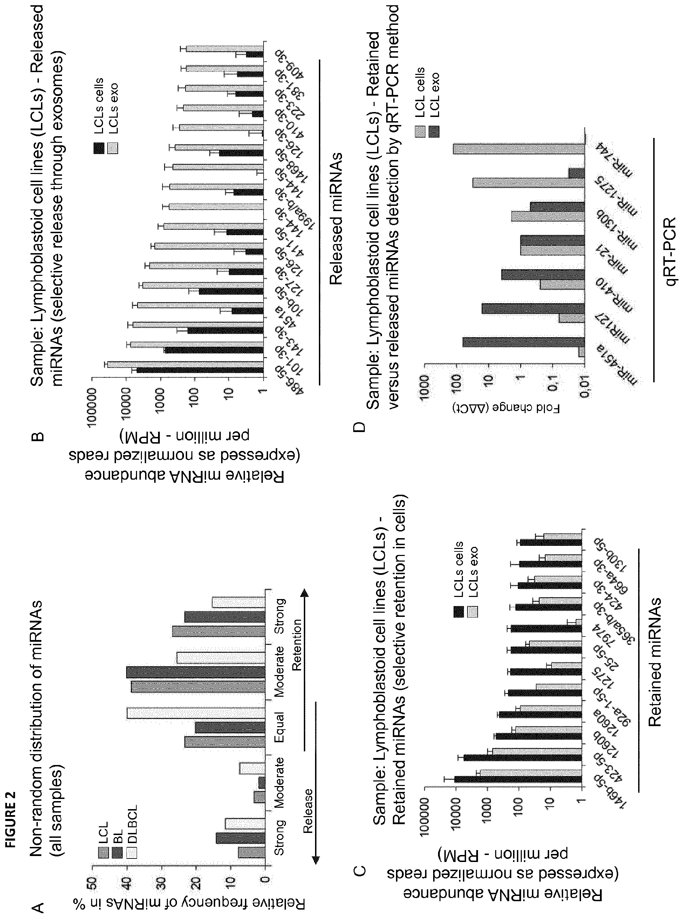

Figure 2. MicroRNAs are non-randomly incorporated into exosomes. A) MiRNAs were classified in five groups indicated at the x-axis according to the ratio between the amount of miRNAs released from the cells and the amount retained in the cell after normalization (reads per million; RPM). The data is plotted as percentage of miRNAs fraction distributed over five groups.

B-C) Significant over-represented miRNAs in exosomes and cells are depicted at the x-axis with their read abundance exceeding 100 reads per million (normalized, RPM) at the y-axis (on logarithmic scale). The error bars are the SD of the mean (LCL 1-3 samples; n=3). D) MiRNA detection in LCL cells and their pared exosomes by stem-loop based qRT-PCR. Data represents ΔΔCt value of technical duplicates normalized to miR-92a Ct value from two independent experiments.

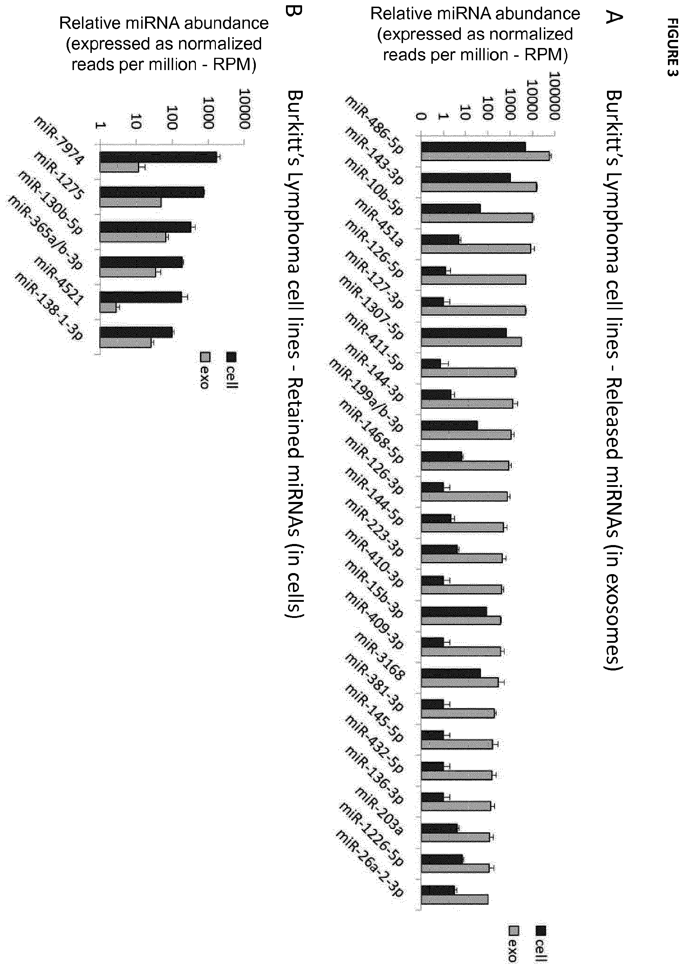

Figure 3. MicroRNAs are non-randomly incorporated into Burkitt's Lymphoma exosomes.

MiRNAs from exosomal and cellular fractions of BLs (BL1 and 2) are analyzed as LCL samples. edgeR analysis yielded statistically significant over-represented miRNAs in (3a) exosomes and (3b) cells with their read abundance exceeding 100 reads per million (normalized, RPM). The y-axis represents the relative abundance of the non-randomly distributed miRNAs. MiRNAs that are significantly expelled or retained are compared with their retained or expelled equivalents. The error bars are the SD; (n=2); y-axis is in logarithmic scale.

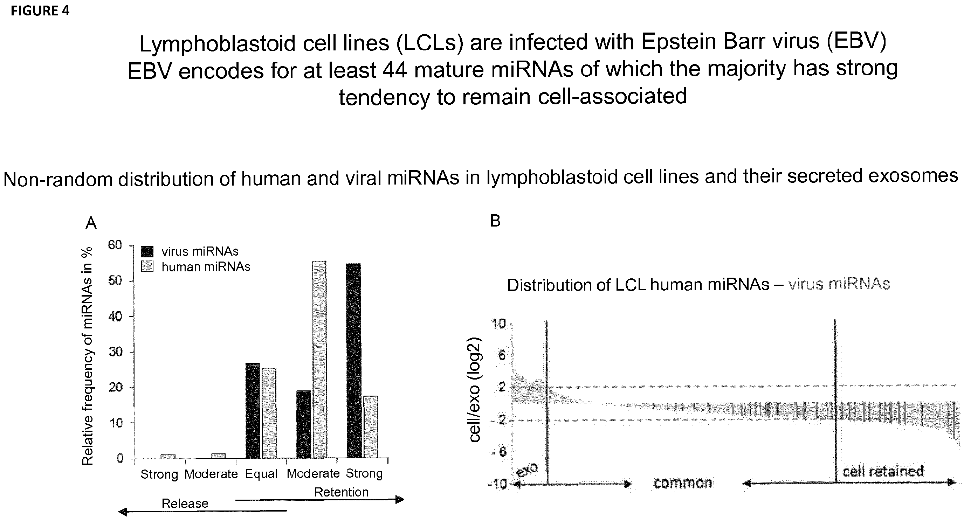

Figure 4. EBV encoded miRNAs exhibit stronger tendency for cellular retention then human miRNAs.

A) Human (LCL) and EBV miRNAs were classified in five groups according to the ratio between the amount of miRNAs released from the cells and the amount retained in the cell after normalization (RPM). The data is plotted as percentage of miRNAs fraction distributed over five groups.
B) Human (LCL1) and EBV miRNAs were classified according to the Fold Change (defined as exo/cell log2) between miRNAs released from the cells and miRNAs retained in the cell after normalization (RPM). Vertical lines indicate miRNA fractions with > 4-fold increase in cell retention or exosome-associated release. Red lines indicate distribution of viral miRNAs among human miRNAs (gray).

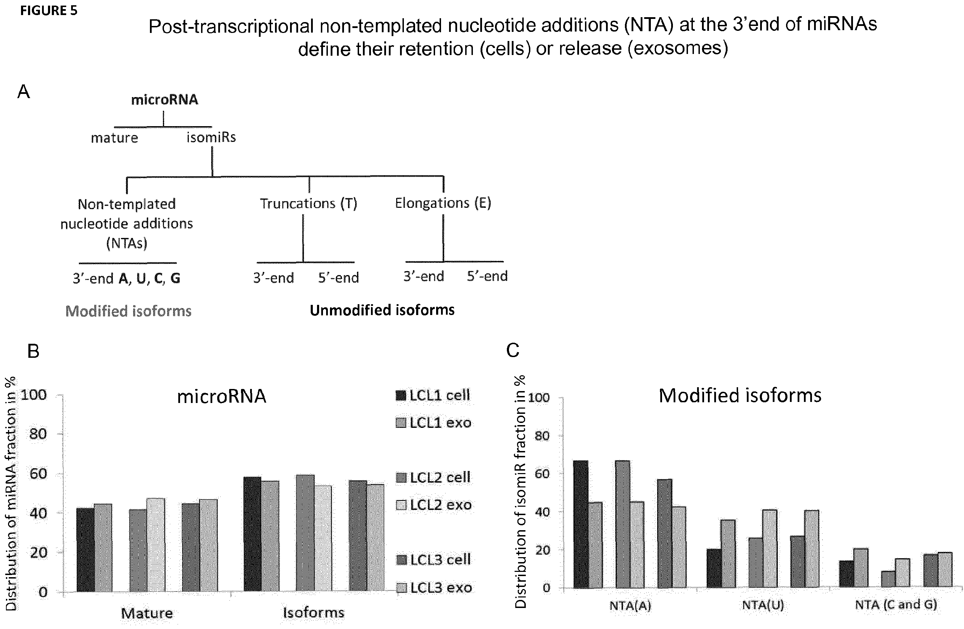

Figure 5. Distribution of mature miRNAs and their isoforms in individual LCL samples (LCL 1, 2 and 3; cells vs. exosomes). A) Sequencing reads for all miRNAs detected in each library that mapped to annotated miRNA sequence are further dissected to mature (canonical) and isoform sequences. Those are further sub-divided into post-transcriptionally modified isoforms (non-templated nucleotide additions; NTAs) and post-transcriptionally unmodified isoforms (truncations and elongations). B) Samples are plotted against the percentage of the sum of all reads assigned to individual miRNAs per sample (for mature vs. isoforms) and C) against the sum of all miRNA reads with the NTAs.

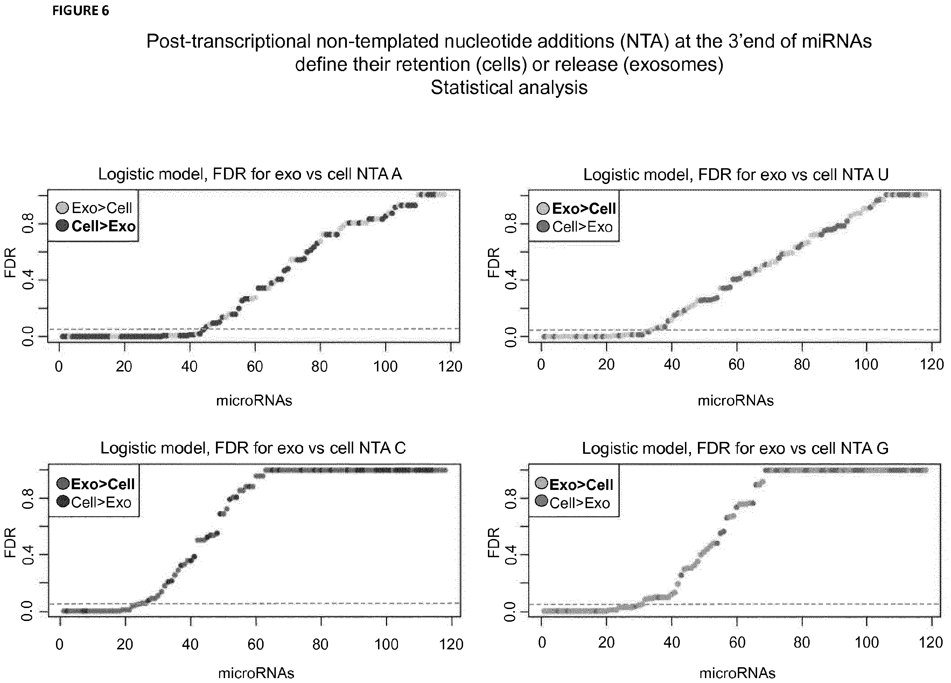

Figure 6. Statistical analysis and the significance of the effect of 3'-end NTA-type on miRNA variants distribution between cells and exosomes. False discovery rate-corrected p-values for 118 miRNAs expressed in all 12 samples. P-values derived from a logistic model comparing counts proportion of each NTA (A, U, C and G) between cell and exosome. In each graph, the circle color indicates the direction of the effect: exo > cell means that the data indicates larger proportions in exosomes compared to cells, and exo < cell means that the data indicates larger proportions in cells compared to exosomes. The results show that 3'end adenylated miRNA isoforms are preferentially retained (chi-square test, p<0.05), whilst all other NTAs are preferentially released (3'end-U: chi-square test, p=0.02; 3'end-C: Fisher's exact test, p=0.04; and 3'end-G: Fisher's exact test, p=0.02).

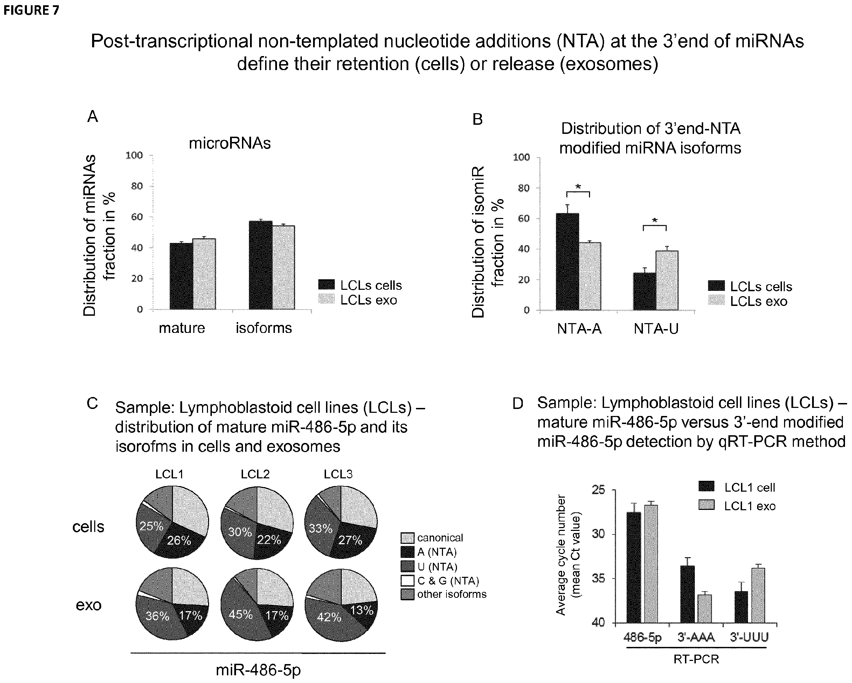

Figure 7. The 3'end posttranscriptional modification defines retention or release of miRNA.

A-B) Distribution of mature miRNAs and their NTA-modified or unmodified isoforms in LCL samples was compared between cells and exosomes. The error bars are the SD of the mean (LCL 1-3 samples, n=3); * P-value = 0.005 (Student's t-test).
C) Distribution of miR-486-5p between individual LCL cells and exosomes libraries. All sequencing reads that mapped to miR-486-5p were summed and the contribution of (iso)form types of miR-486-5p is expressed as percentage.
D) Detection of canonical miR-486-5p, 3'-end adenylated miR-486-5p (3'-AAA) and 3'end uridylated miR-486-5p (3'-UUU) in cellular (LCL) and exosomal RNA fractions by qPCR. Data represents mean cycle threshold (Ct) value of technical duplicates from two independent experiments (error bars, SD).

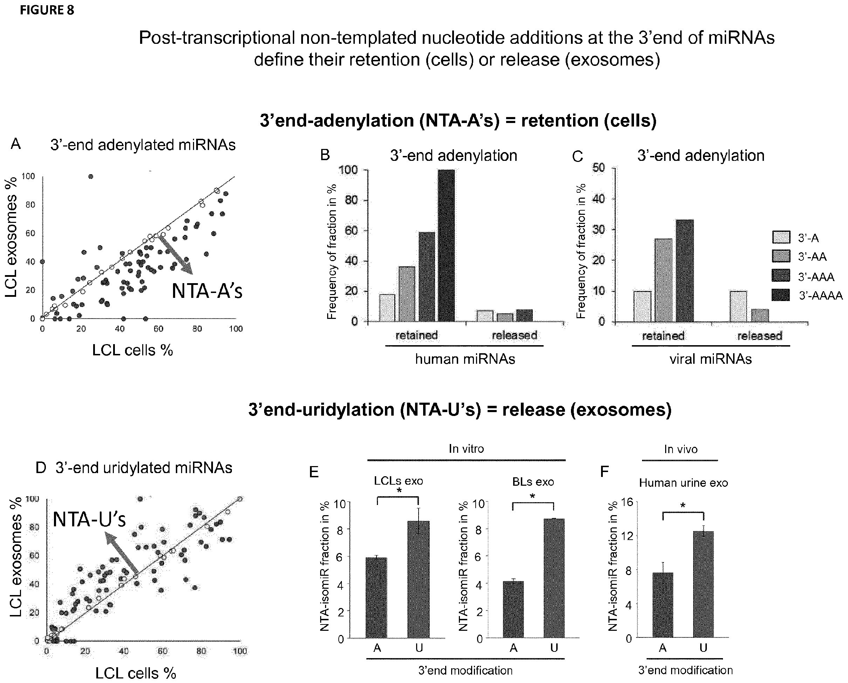

Figure 8. The extent of 3'end adenylation increases retention while 3'end uridylation demarcates exosomal small RNA cargo.

A and D) Frequency plots of a pair-wise analysis of 3'-A and 3'-U isoforms. MiRNAs found to be expressed in both cells and exosomes were selected (at >300 reads, 100 miRNAs) for distribution analysis of their adenylated and uridylated reads expressed as percentage to the total of all NTA-modified reads. Red circles: isoforms with higher percentage in exosomes; Purple circles: isoforms with higher percentage in cells; White circles: equal distribution.

B-C) Fraction of human miRNAs and viral miRNAs for which the adenylated reads (3'end-A's) show retention or release tendency measured against the non-adenylated reads of the same miRNA. Adenylated miRNAs have a higher probability to be retained and it increases with the number of added adenines.

E-F) 3'-end uridylation rather than adenylation is more frequent on miRNA isoforms present in LCLs exosomes, BLs exosomes and in human urine exosomes. The bars represent the weighted mean of isoforms reads per sample (exosomes only) with 3'end NTA; nucleotide type indicated on the x-axis. The error bars are the SD of 3 samples for LCLs (n=3); p=0.005; 2 samples for BLs (n=2); p=0.001; and 6 samples from human urine (n=6); p<0.0001 (Student's t-test).

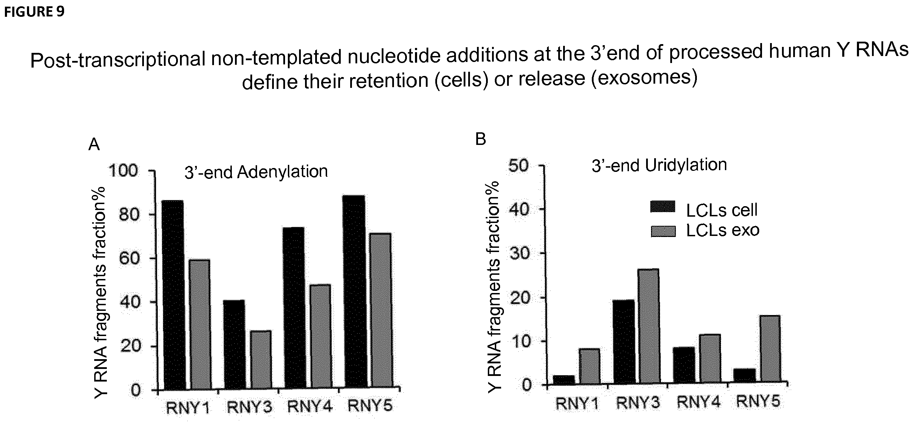

Figure 9. 3'-end post-transcriptional modification affect distribution of processed small ncRNAs. A) Y RNA fragments with 3'end adenylation or B) 3'-end uridylation (right panel) are differentially distributed between cellular and exosomal fractions. RNA fractions that correspond to processed Y RNA fragments that are derived from RNY1, RNY3, RNY4 and RNY5 (x-axis) are plotted against the percentage of RNA fragments with 3'end modifications.

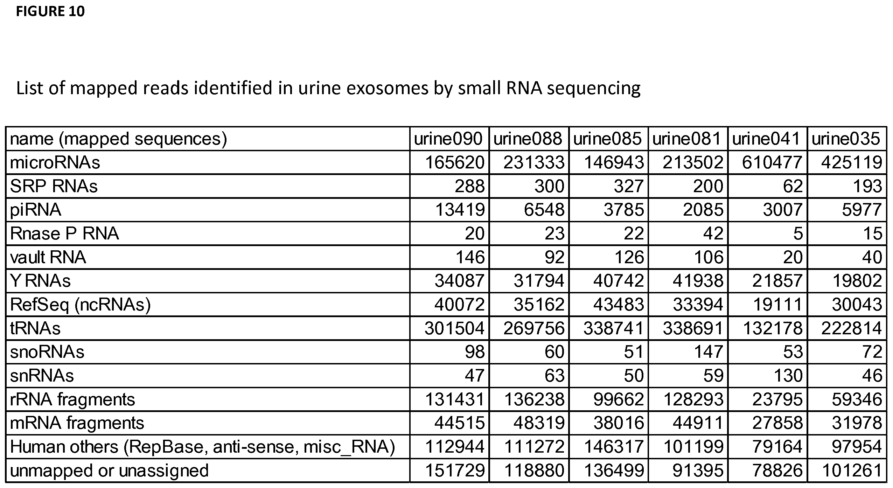

Figure 10. List of mapped reads identified in urine exosomes by small RNA sequencing. Summary of samples characteristics and RNA-seq data from human urine extracellular vesicles. cDNA libraries were generated from exosomal small RNA fractions after purifying urine exosomes. Total number of reads from all libraries before and after mapping to genome of interest (hsg 19/GRCh37, patch 5) is specified. To provide annotations to RNA elements that mapped to human genome, all mapped reads were analyzed against currently known databases: 1) mature and pre-miRNA sequences from miRBase version 19, 2) NCBI Reference Sequences human RefSeq genes downloaded May 2013, 3) tRNA sequences from the genomic tRNA database, 4) Repeat derived sequences detected by means of the RepeatMasker algorithm downloaded from UCSC and 5) piwiRNA (piRNAs) downloaded from the NCBI nucleotide database.

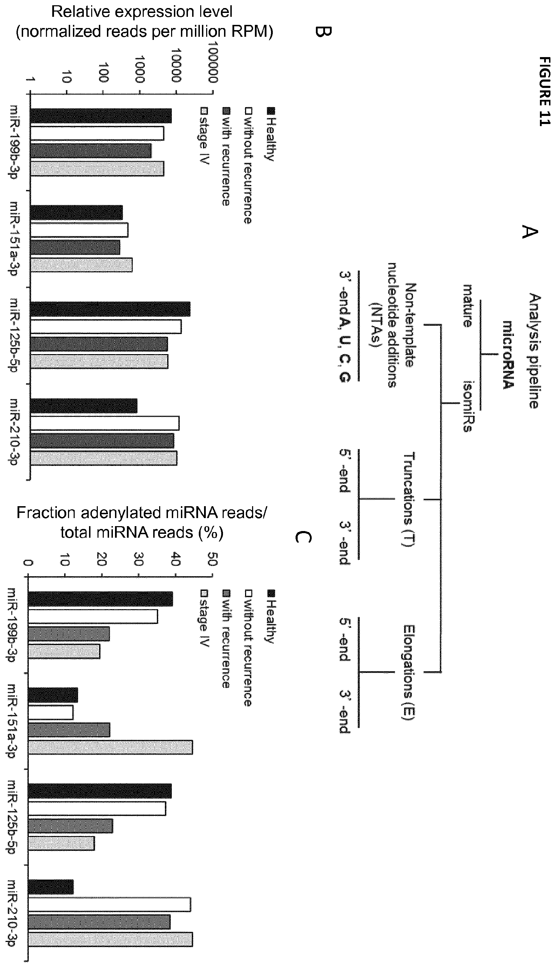

Figure 11 Healthy renal tissue biopsies vs. Clear cell Renal Cell Carcinoma (kidney tissue) biopsies

A) MicroRNA diversification. Sequencing reads detected in each library that mapped to annotated miRNA sequence are grouped and presented as one miRNA. Those mapped reads consist of mature (canonical) and isoform sequences. Isoforms are further sub-divided into enzymatically modified isoforms (3'-end NTA non-templated nucleotide addition) and unmodified isoforms (truncations and elongations occurring at both 3'-end and 5'-end.

B) The analysis of publicly available data from clear cell renal cell carcinoma (CCRC; 4 experimental groups) by RNASeq. Data analysis based on mature (miRBase) sequences shows poor distinction between experimental groups for selected miRNAs.

C) The ratio between isomiRs and mature miRNAs in all 4 experimental groups revealed pronounced differences for 3'-end adenine additions (adenylation) especially between the clinically relevant groups. Significant differences in proportion of adenylated miRNAs permit separation between clinically defined groups.

Figure 12: Post-transcriptional modifications stratifying health versus prostate cancer patients. Although the table states that the data is presented as "C/V", the ratios are in fact presented as "V/C". This change does not affect the STD or p-values.

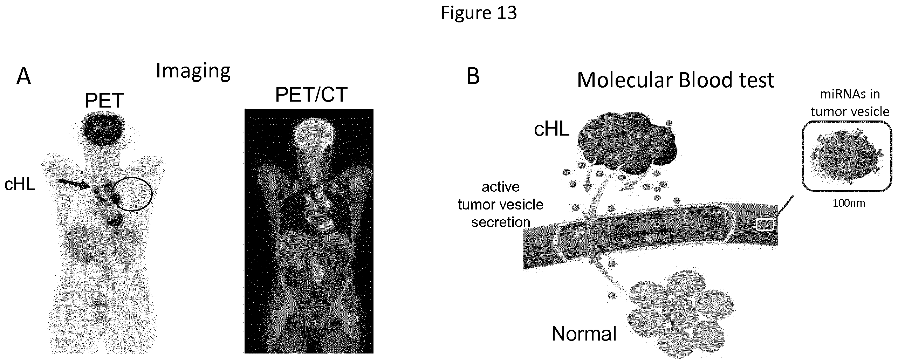

Figure 13: Non-invasive strategies for monitoring vital tumor tissue in malignant Lymphoma patients
A) Left: FDG/PET image of a classical Hodgkin Lymphoma patient before treatment. The black arrow indicates multiple metabolically active tumor masses. Right: fused PET/CT image of the same patient. White arrow indicates a vital tumor mass. B) Lymphoma tumor cells (upper dark brown) and normal cells (lower light brown) actively secrete 100 nanometer vesicles, including MVB-derived exosomes into circulation. These extracellular vesicles (EVs) contain miRNAs that are protected against external RNAses. Besides encapsulated by EVs, miRNAs can be associated with and protected for degradation by proteins and HDL, however these do not reflect vital tumor cells. Moreover, dying cells release biomolecules (ie RNA, DNA, protein) into circulation. The figure is adapted from Hori et al et al., 2013 Science Transl Med.

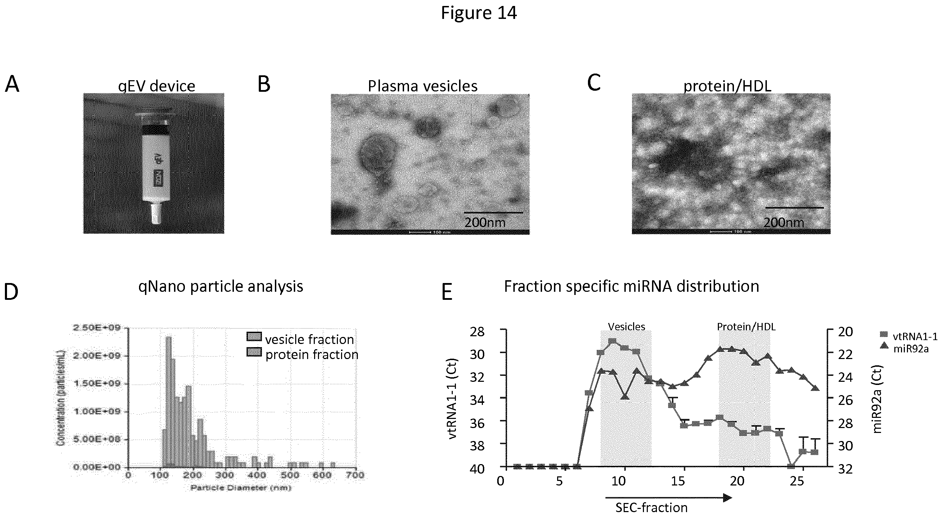

Figure 14: Single step size-exclusion chromatography (SEC) separates circulating extracellular vesicles (EV) from protein/HDL for optimal miRNA detection in patient plasma
A) qEV size exclusion chromatography (SEC) column (recently commercially available from IZONtm). The qEV columns allow single-step reproducible isolation of circulating EVs from 1-1.5 ml plasma, separating them from circulating protein/HDL (Boing et al., JEV 2014). B) EM image of plasma EVs isolated from a cHL patient using sepharose CL-2B SEC. The size bar indicates 200nm and most EVs are in the range of 100nm although larger (200nm) vesicles are present. C) EM image of the protein/HDL fraction of the same patient plasma as in B. Particles that resemble EVs are not observed. D) Particle analysis using qNano (IZONtm), based on Tunable Resistive Pulse Sensing (TRPS). cHL patient plasma EV and protein/HDL fractions are separated using the qEV device. The EV fraction (orange) is highly enriched in particles with a size-distribution that corresponds to the EM image in B. E) RT-PCR analysis of EV and protein/HDL fractions separated by SEC. The EV fractions 9-12 are highly enriched for vtRNA1-1, while protein/HDL fractions (19-22) are enriched for miR92a consistent with (Arroyo et al., PNAS 2011).

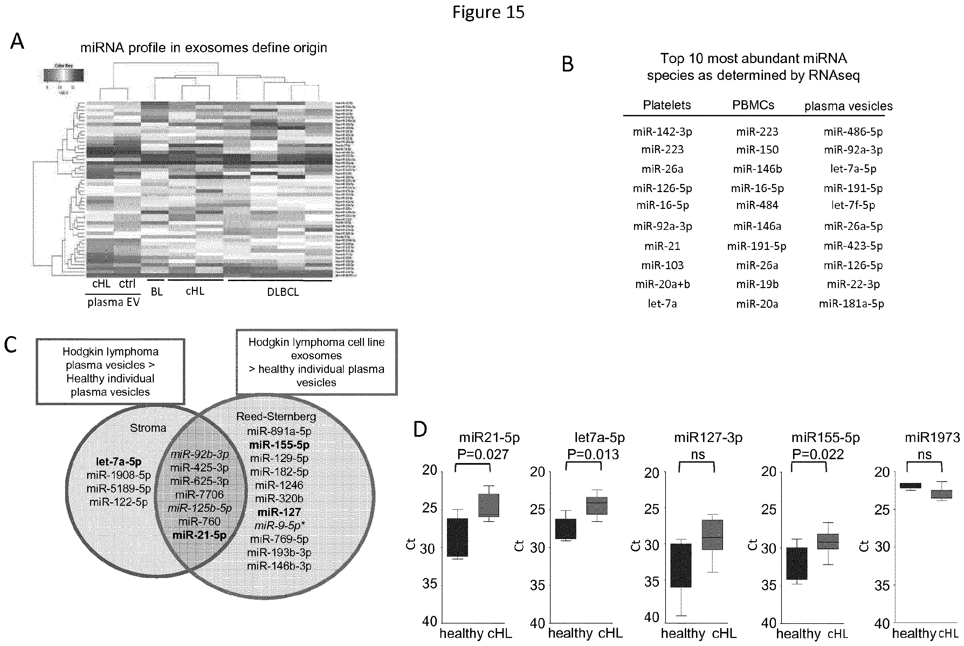

Figure 15: Selection and validation of candidate miRNA biomarkers in plasma EV A) Hierarchical clustering of miRNAs from vesicles. RNAseq analysis of Lymphoma cell line derived exosomes (n=7) and plasma extracellular vesicles from a cHL patient (before treatment) and a healthy control shows clearly distinct profiles. B) List of the 10 most abundant miRNAs in Platelets, PBMCs and EVs (Ple et al., PLoSone 2012). C) Comparison of the level of miRNAs in plasma EV and Hodgkin cell line derived exosomes (L1236) yields potential cHL stroma-derived and tumor-associated miRNA markers. MiRNAs shown differ by log(FC)>1. D) Boxplots showing RT-PCR data from 4 candidate miRNA biomarkers in plasma EVs isolated from cHL patients (n=10) and healthy controls (n=4). miR1973 is abundantly detected in plasma EV from both cHL patients and healthy controls. P values were calculated using a two-tailed student's t-test.

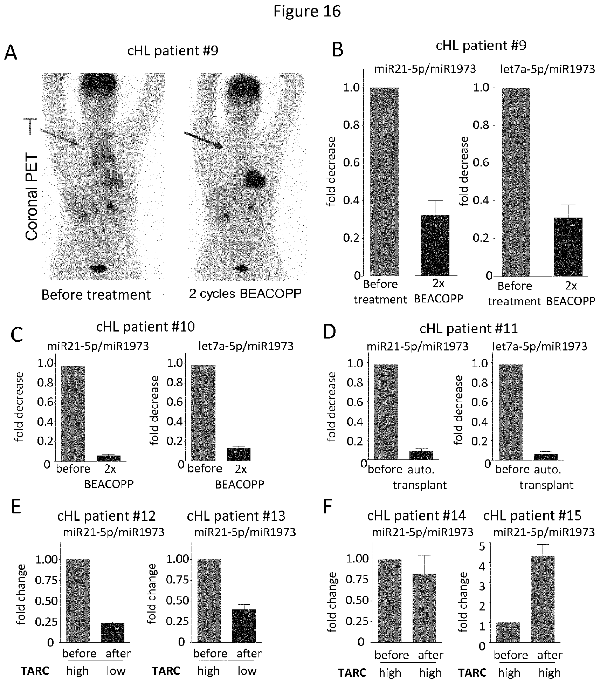

Figure 16: miR-21-5p and let7a-5p levels in plasma EV decrease during successful treatment
A) PET images from a cHL patient before (left) and after (right) 2 cycles of first line treatment (BEACOPP). Arrow indicates metabolically active tumor. B) RT-PCR analysis shows a strong decrease (70%) of EV associated miR-21-5p and let-7a-5p during treatment. MiR-21-5p and let-7a-5p levels are normalized to miR-1973 and shown as fold decrease (error bars, SEM). C+D) MiR21-5p and let7a-5p levels in EVs decrease in both de novo patients during BEACOPP treatment (C) and in relapse patients after treatment with DHAP/ADC followed by autologous stem cell transplantation (D) as determined by RT-PCR. MiR-21-5p and let-7a-5p levels are normalized to miR-1973 and shown as fold decrease (error bars, SEM). E+F) MiR-21-5p and let7a-5p levels in EVs decrease in cHL patients responding to therapy as determined by decreasing serum TARC levels (E) but not in cHL patients where TARC levels remain high (F). MiR21-5p and let7a-5p levels are normalized to miR1973 and shown as fold change (error bars, SEM).

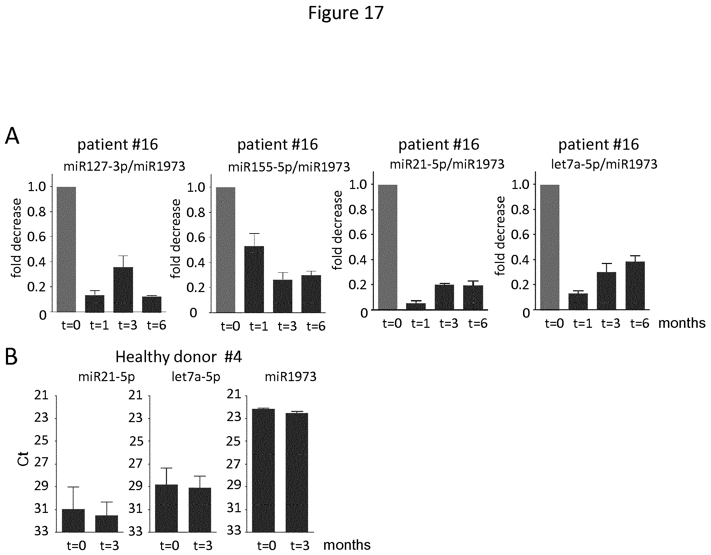

Figure 17: Candidate miRNA biomarker levels in plasma EV remain low during follow up
A) MiR-127-3p, miR-155-5p, miR-21-5p and let-7a-5p levels in cHL patient plasma EV are decreased during (t= 1 month) and after (t=3 months) BEACOPP treatment and remain low up to 6 months after therapy. MiRNA levels are normalized to miR-1973 and shown as fold decrease (error bars, SEM). B) RT-PCR analysis shows that miR-21-5p and let-7a-5p levels in plasma EV of a healthy donor are stable. Data is shown as Ct values (error bars, SEM).

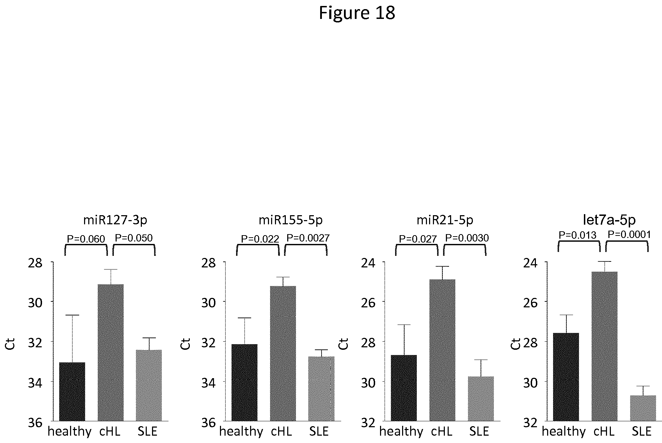

Figure 18: Increased levels of candidate miRNAs in cHL plasma EV are disease specific
MiR-127-3p, miR-155-5p, miR-21-5p and let-7a-5p RT-PCR levels are increased in cHL patient plasma EV (n=10), but not in autoimmune (SLE) patients (n=3) compared to healthy controls (n=4). Data is shown as Ct values (error bars, SEM). P values are calculated using a two-tailed student's t-test.

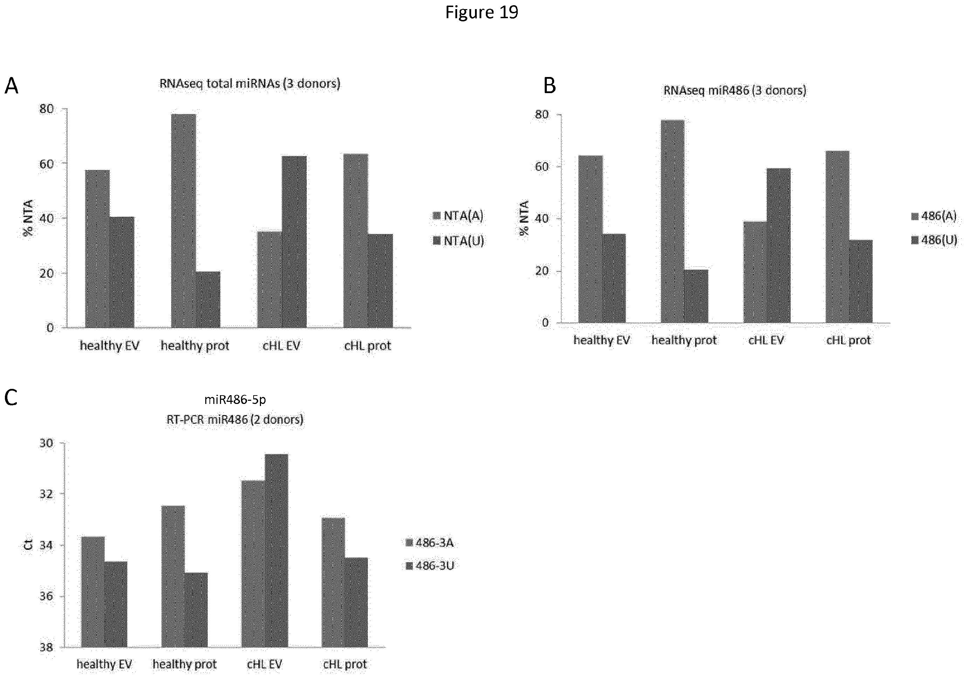

Figure 19: NTA analysis on miRNAs in plasma EVs of Healthy and Hodgkin patients We performed sequencing on multiple plasmas from healthy donors and cHL patients in both protein and vesicle fractions and analyzed the data with sRNAbench (http://arn.ugr.es/srnabench/). A) Graph showing the proportion of all identified miRNAs with the 3'-end NTA defined as A or U. Only the cHL EV fraction has a higher proportion of the 3'-end uridylated miRNAs. B) Same as in A but now the data represents differenced for 3'-end post-transcriptional NTA-based modification of miR-486-5p. C) Taqman RT-PCR as previously described (Koppers-Lalic et al., 2014) for miR486-5p in various fractions of 2 donors confirming RNAseq data as shown in B.

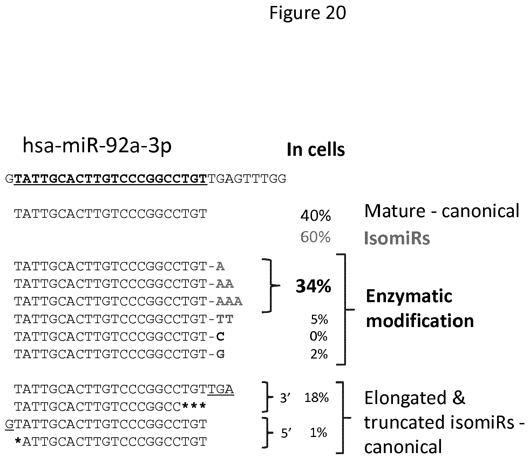

Figure 20: Graphical representation of mapped reads that align to miR-92a genomic sequence. Post-transcriptional modifications (NTA) and elongations/truncations of 5' or 3'-end nucleotides are indicated with underlined letters (for elongation) or with * for truncations.

Figure 21: exemplary biomarkers. Table 2 depicts tRNA biomarker pairs from urine in prostate cancer (PCa) patients. Table 3 depicts miRNAs that characterize cHL. Table 4 and 10 depict ncRNA biomarker pairs in extracellular vesicles extracted from blood plasma of healthy donors and cHL patients. Table 5 and 11 depicts ncRNA biomarker pairs in protein fraction (PF) extracted from blood plasma of healthy donors and cHL patients. Table 6 depicts ncRNA biomarker pairs in blood serum of breast cancer patients. Table 7 depicts ncRNA biomarker pairs in testicular germ cell tumors. Table 8 depicts ncRNA biomarker pairs in colorectal cancer. Table 9 depicts ncRNA biomarker pairs in blood serum of Alzheimer's patients.

DETAILED DESCRIPTION OF THE DISCLOSED EMBODIMENTS

The articles "a" and "an" are used herein to refer to one or to more than one (i.e., to at least one) of the grammatical object of the article. By way of example, "an element" means one element or more than one element.

As used herein, "bodily fluid" refers to a bodily fluid comprising ncRNA including blood (or a fraction of blood such as plasma or serum), lymph, mucus, tears, saliva, sputum, urine, semen, stool, CSF (cerebrospinal fluid), breast milk, and, ascities fluid. Preferably, the bodily fluid is urine. Preferably, the bodily fluid is selected from blood. As used herein, "blood" also includes blood serum and blood plasma. Preferably, the bodily fluid is blood plasma.

"Biomarker" may be used to refer to a biological molecule present in an individual at varying concentrations useful in predicting the health status of an individual.

As used herein, "to comprise" and its conjugations is used in its non-limiting sense to mean that items following the word are included, but items not specifically mentioned are not excluded. In addition the verb "to consist" may be replaced by "to consist essentially of' meaning that a compound or adjunct compound as defined herein may comprise additional component(s) than the ones specifically identified, said additional component(s) not altering the unique characteristic of the invention.

As used herein, "health status" refers to the overall (physical/physiological) condition of an individual at a particular time. Health status includes the presence or absence of disease or disorders, e.g., neurological disorders (such as Alzheimer's disease), cancer/tumors, infectious disease, metabolic diseases (e.g., amyloidosis), cardiovascular diseases, and immunological disorders. Preferably, the disorder is selected from prostate cancer, breast cancer, cervical cancer, lymphoma, colon cancer, glioblastoma and lung cancer.

Health status also includes the risk of developing such disorders (i.e., having a decreased or increased risk over the general population (or individuals of similar age, genetic background, environmental risk factors, etc.). Health status also includes the particular stage of disease/disorder as well as the severity and prognosis (e.g., survival prognosis). Health status also refers to the prognosis of an individual to be effectively treated with a particular agent. "Classifying a health status" includes classifying an individual as healthy; as having or not having a particular disorder; as having an increased, decreased, or normal risk of developing a disorder; as being a good or poor responder to a particular treatment; as having a particular stage or severity of a disease; as have a good or poor survival or recovery prognosis.

As used herein, an "individual" refers to humans and animals, e.g., mammals such as a domestic animal (e.g., dog, cat), a farm animal (e.g., cow, sheep, pig, horse) or a laboratory animal (e.g., monkey, rat, mouse, rabbit, guinea pig). Preferably the individual is a human.

As used herein, "small non-coding RNA" (ncRNA) refers to RNA that is not translated into protein and includes transfer RNA (tRNA), ribosomal RNA (rRNA), snoRNAs, microRNA (miRNA), siRNAs, small nuclear (snRNA), Y RNA, vault RNA, antisense RNA, tiRNA (transcription initiation RNA), TSSa-RNA (transcriptional start-site associated RNA) and piwiRNA (piRNA).Small ncRNA have a length of less than 200 nucleotides. In the embodiments of the invention, the ncRNA is selected from miRNA, tRNA, and Y-RNA.

Preferably, a small ncRNA as used herein is between 16 and 200 nucleotides, more preferably between 16 and 100 nucleotides, even more preferably between 16 and 40 nucleotides. A ncRNA may be of endogenous origin (e.g., a human miRNA) or exogenous origin (e.g., virus, bacteria, parasite). "Canonical" ncRNA refers to the sequence of the RNA as predicted from the genome sequence and is the most abundant sequence identified for a particular RNA. For miRNA, this refers to miRNAs formed via the "canonical miRNA pathway". Precursor miRNA (pre-miRNA) is cleaved into a short hairpin RNA and is then exported into the cytoplasm for processing by a Dicer enzyme. The resulting "canonical" mature miRNAs are usually 21-22 nucleotides in length.

"Trimmed" ncRNA refers to an ncRNA in which exonuclease-mediated nucleotide trimming has removed one or more nucleotides at the 5' and/or 3' end of the molecule. Preferably, the trimming is a 3' trimming. Preferably, one nucleotide is trimmed from the 3' end of a canonical sequence. Trimmed miRNA can be easily detected since the start and stop sites of canonical miRNAs are known. Examples of trimmed ncRNAs are depicted in Figure 20 and Figure 21 (see, e.g., table 11C).

3' non-templated nucleotide addition (3'NTA) refers to post-translational additions of one or more nucleotides to the 3' end of an RNA, usually by RNA nucleotidyl transferases, such as PAPD4, PAPD5, ZCCHC6, and ZCCHC11 (Burroughs et al., 2010; Polikepahad and Corry, 2013). The most common forms are adenylation (3' NTA-A) and uridylation (3' NTA-U), but the addition of cytosine (3' NTA-C) and guanine (3' NTA-G) are also possible. Although generally one or two nucleotides are added, the addition of three or more nucleotides is also possible. Before NTA occurs, the canonical sequence is optionally trimmed at the 5' or 3' end. Preferably, the 3' NTA is a single nucleotide addition selected from 3' NTA-A, 3' NTA-G, 3' NTA-U, and 3' NTA-C. Examples of 3' NTA ncRNAs are depicted in Figure 20 and Figure 21 (see, e.g., table 2A).

"Extended ncRNA" refers to an miRNA that is longer than the canonical miRNA sequence and is a term recognized in the art. The nucleotides making up the extension correspond to nucleotides of the precursor sequence and are therefore encoded by the genome in contrast to non-templated nucleotide addition. While not wishing to be bound by theory, it is thought that extended ncRNAs are the result of differential precursor miRNA processing. In general, such extensions may comprise 1-5, usually 1-3, extra nucleotides as compared to the canonical miRNA sequence. Extended miRNA can be easily detected since the start and stop sites of canonical miRNAs are known. Examples of extended ncRNAs are depicted in Figure 20 and Figure 21 (see, e.g., table 11A).

The ncRNA variants are selected from the canonical sequence of the ncRNA, a 5' or 3' trimmed version of the ncRNA a 5' or 3' extended version of the ncRNA, a 5' or 3' trimmed version of the ncRNA or 5' or 3' extended version of the ncRNA (collectively referred to herein as "length variants") and the ncRNA having a 3' non-templated nucleotide addition (3'NTA). As used herein, "ncRNA variants" refers to a group of sequences that originate from a single ncRNA gene. Each variant differs in sequence from each other, e.g., by 5' or 3' trimming or by the presence or absence of 3' NTAs. The variants may have the same or different biological function.

While not wishing to be bound by theory, it is believed that trimmed ncRNA (i.e., truncated) and extended ncRNA (i.e., elongated) may result for similar inefficiencies in pre-miRNA processing. In some embodiments, the ncRNA variant is a "length variant", i.e., a trimmed or extended ncRNA. For example, table 5B lists biomarker pairs where one variant is the canonical structure and the second variant is a 3' length variant. In some embodiments, it is preferred to separate the length variants into ncRNA extensions and trimmed ncRNAs. Table 11 is an analysis of the same data, but where the length variants are split into extensions (see, table 11A for 3' end extensions) and trimmed ncRNAs (see, table 11B for 3' end trimmed variants).

Quantify and quantification may be used interchangeably, and refer to a process of determining the quantity or abundance of a substance in a sample (e.g., a biomarker), whether relative or absolute. For example, quantification may be determined by methods including but not limited to, micro-array analysis, qRT-PCR, band intensity on a Northern blot, targeting small RNA sequencing or by various other methods know in the art.

The term "treating" includes prophylactic and/or therapeutic treatments. The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

In one aspect, the disclosure provides a method for identifying a small non-coding RNA (ncRNA) biomarker pair. Preferably the ncRNA biomarkers are of at least 16 nucleotides in length. Example 3 describes biomarker pairs for discriminating healthy patients from those having prostate cancer, which were identified using a method as described herein. The top biomarker pairs are disclosed in Figure 12.

The method comprises determining for an ncRNA, the ratio of two different varieties of the ncRNA in a first bodily fluid sample obtained from a first individual reference and in a second bodily fluid sample obtained from a second individual reference, wherein said first individual reference has an altered health status from said second individual reference.

The bodily fluid samples are from one or more individuals or may be the average ratio in a population of individuals. The first individual reference (which may be one more individuals, preferably one) has an altered health status then the second individual reference (which may be one more individuals, preferably one). In some embodiments, the first and second individual reference are from the same individual wherein the health status of the individual has changed, e.g., the samples are obtained from an individual before and after a treatment regime.

Methods of identifying diagnostic and prognostic biomarkers are well-known in the art. The selection of the samples is well within the purview of one skilled in the art and can vary depending on the type of biomarker to be identified.

In the case of biomarkers of disease, an exemplary embodiment provides that the first individual reference is from an individual(s) having a disorder and the second sample from an individual reference (s) not having said disorder. Preferably the disorder is cancer, more preferably selected from cervical cancer, lymphoma, colon cancer, glioblastoma and lung cancer prostate cancer.

In the case of biomarkers of risk, an exemplary embodiment provides the first individual reference from an individual(s) having an increased risk of developing a disorder and the second individual reference from an individual(s) not having an increased risk. For example, the samples could be provided from heavy-smoking individuals before the onset of lung cancer. The ratios are analysed and after a certain amount of time, for example 1 or 2 years, the status of lung cancer in the individuals is monitored.

In the case of prognosis biomarkers, an exemplary embodiment provides the first individual reference from an individual successfully treated and a second sample from an individual not successfully treated.

The ratio of RNA variety may be determined by methods known to a skilled person. Preferably, the ratio is determined by quantifying the two different varieties in each sample and determining the relationship between the two quantities. This is preferably expressed as the quotient of one divided by the other. Preferably, the abundance of each variety is determined. More preferably, the relative abundance of each variety is determined.

In an exemplary embodiment, the ratio is (abundance of a ncRNA variety 1 divided by abundance of the canonical ncRNA) divided by (abundance of a ncRNA variety 2 divided by abundance of the canonical ncRNA).

Methods for quantitating RNA levels are well-known. Several methods exist to add additional sequence to an ncRNA to facilitate priming and detection. In particular, a common sequence can be added to every ncRNA to allow for use of a single universal extension primer. In particular, QPCR-based ncRNA detection methods add additional sequence to the miRNA to increase its length prior to or during reverse transcription. There are also a number of commercially available systems for performing PCR assays on small RNAs (e.g. Applied Biosystems Custom TaqMan® Small RNA Assays and miRCURY LNA™ microRNA PCR System from Exiqon). Preferably, the method for quantitating RNA levels is "Deep sequencing". This refers to methods which provide both the sequence and the frequency of an RNA molecule. (see, e.g., US20120322691for general methods and specific adaptor modifications).

The methods further comprise comparing the ratios from said first and second bodily fluid sample, and identifying the two different varieties of said ncRNA as a biomarker pair when the ratio is altered between said first and second bodily fluid sample. An altered ratio between the first sample and second sample identifies said ncRNA as an ncRNA biomarker. More precisely, the two ncRNA varieties are identified collectively as a biomarker. An altered ratio is a statistically significant difference between the ratios in the two samples. Preferably, an altered ratio is indicated when the ratio from the first sample falls outside of about 1.0 standard deviations, about 1.5 standard deviations, about 2.0 standard deviations, or about 2.5 stand deviations of the second sample.

The ncRNA varieties are selected from the canonical sequence of the ncRNA, a 5' or 3' trimmed version of the ncRNA, a 5' or 3' extended version of the ncRNA, a 5' or 3' trimmed version of the ncRNA or a 5' or 3' extended version of the ncRNA (i.e., "length variant") and the ncRNA having a 3' non-templated nucleotide addition (3'NTA). Preferably, the first variety is selected from the canonical ncRNA and the second variety is the ncRNA with a 3' non-templated nucleotide addition, and when said ncRNA is miRNA, the first variety is selected from canonical ncRNA, the ncRNA trimmed at the 5' or 3' end, and the ncRNA with a 3' non-templated nucleotide addition, optionally trimmed at the 5' or 3' end and the second variety is selected from ncRNA trimmed at the 5' or 3' end and ncRNA with a 3' non-templated nucleotide addition, optionally trimmed at the 5' or 3' end. It is clear that the ratio is for two different varieties of the same ncRNA.

In exemplary embodiments, the ratio is selected from canonical : 3' NTA-A; canonical : 3' NTA-G; canonical: 3' NTA-C; canonical: 3' NTA-U; 3' NTA-G: 3' NTA-C; 3' NTA-G: 3' NTA-U; 3' NTA-G: 3' NTA-A; 3' NTA-C : 3' NTA-U; 3' NTA-C: 3' NTA-A; and 3' NTA-U : 3' NTA-A; canonical : 5' trimmed; canonical : 3' trimmed; 5' trimmed: 3' NTA-C; 5' trimmed: 3' NTA-U; 5' trimmed: 3' NTA-A; 5' trimmed: 3' NTA-G; 3' trimmed: 3' NTA-C; 3' trimmed: 3' NTA-U; 3' trimmed: 3' NTA-A; 3' trimmed: 3' NTA-G; 3' trimmed : 5' trimmed; extended and 3' NTA-A; extended and 3' NTA-G; extended and 3' NTA-C; extended and 3' NTA-U; extended and 5' trimmed; extended and 3' trimmed; 5' or 3' trimmed and 5'or 3'extended; (trimmed and extended) and 3' NTA-U; (trimmed and extended) and 3' NTA-A; (trimmed and extended) and 3' NTA-G; (trimmed and extended) and 3' NTA-C; (trimmed and extended) and canonical. Preferably, the ratio is selected from canonical : a 3' NTA or a 3' NTA : a different 3' NTA. It is clear to a skilled person that the numerator and denominator in the ratios can be reversed. Preferably, the ncRNA is miRNA.

In exemplary embodiments, the ratio is selected from canonical : 3' NTA-A; canonical : 3' NTA-G; canonical: 3' NTA-C; canonical: 3' NTA-U; 3' NTA-G: 3' NTA-C; 3' NTA-G: 3' NTA-U; 3' NTA-G: 3' NTA-A; 3' NTA-C : 3' NTA-U; 3' NTA-C: 3' NTA-A; and 3' NTA-U : 3' NTA-A. It is clear to a skilled person that the numerator and denominator in the ratios can be reversed.

The present disclosure demonstrates that the predictive power of an ncRNA biomarker can be improved by looking at the ratio of two varieties of the ncRNA. Example 2 demonstrates that the canonical sequence of four different miRNAs fails to discriminate between healthy and tissue and renal cancer. However, clinical relevant groups can be discriminated for these same four miRNAs when the ratio of 3'NTA-A : canonical miRNA is used.

Accordingly, the present disclosure also provides methods for classifying the health status of an individual and for collecting data regarding the health status of an individual using a small non-coding RNA (ncRNA) biomarker pair. The ncRNA pair may be based on any known ncRNA biomarker or those identified, e.g., by the methods described herein. Preferably, each ncRNA biomarker is at least 16 nucleotides in length. Various ncRNA molecules (in particular) miRNAs have been suggested for use as biomarkers (see, eg., WO 2009099905 for markers of melanoma, WO2011025919 for markers of lung disease; WO2013003350 for markers of Alzheimer's disease; US2012289420 for markers of airway diseases, etc.). Any of these ncRNA molecues may be used in an embodiment of the methods.

The method for classifying the health status comprise determining the ratio of a biomarker pair in a bodily fluid sample from an individual and comparing the ratio to the ratio of the biomarker pair in a bodily fluid from a reference sample, wherein the presence or absence in a difference in the ratio (i.e., wherein an altered ratio) between the individual and the reference sample classifies the health status of said individual.

The reference sample may have been obtained from a single individual or from the average of a population of individuals. The ratio for the reference sample may be determined as described herein or it may be information previously available. An alteration in the ratio from the individual as compared to the reference sample indicates an altered health status from the reference.

The choice of the appropriate reference sample is well within the purview of a skilled person. In some embodiments, the reference sample is from individual(s) having a particular disorder and an alteration indicates that said individual is not afflicted with the disorder.

In preferred embodiments, the ratio from the individual is compared to two reference samples, the first being a "healthy" control sample and the second from individual(s) having an altered health status (e.g., at risk of developing a particular disease). A skilled person will recognize that when the individual's ratio is closer to the ratio from the "altered health status" ratio, the individual is more likely to also have the altered health status.

The biomarker pair consists of two different varieties of an ncRNA wherein the first variety is selected from the canonical ncRNA and the ncRNA with a 3' non-templated nucleotide addition and the second variety is the ncRNA with a 3' non-templated nucleotide addition, wherein when said ncRNA is miRNA, the first variety is selected from canonical ncRNA, the ncRNA trimmed at the 5' or 3' end, and the ncRNA with a 3' non-templated nucleotide addition, optionally trimmed at the 5' or 3' end and the second variety is selected from ncRNA trimmed at the 5' or 3' end and ncRNA with a 3' non-templated nucleotide addition, optionally trimmed at the 5' or 3' end. The determination of the ratio and the preferred RNA varieties are as previously described herein. Preferably, the biomarker pair consists of two different varieties of an ncRNA wherein the first and second varieties are selected from the canonical ncRNA; the ncRNA trimmed at the 5' or 3' end and/or extended at the 5' or 3' end; and the ncRNA with a 3' non-templated nucleotide addition, optionally trimmed at the 5' or 3' end or extended at the 5'or 3'end;

wherein when said ncRNA is not an miRNA the first variety is selected from the canonical ncRNA and the ncRNA with a 3' non-templated nucleotide addition and the second variety is the ncRNA with a 3' non-templated nucleotide addition.

In preferred embodiments, the methods disclosed herein determine the likelihood that an individual will respond to a particular treatment. Preferably, said methods further comprise the step of treating said individual for said disease or disorder. In preferred embodiments, the methods determine the risk that an individual will develop a particular disorder.

In preferred embodiments, the methods disclosed herein diagnosis the individual with a disease or disorder. In preferred embodiments, the data collected can be used as one factor for assisting a medical practitioner in making a diagnosis or prognosis. Preferably, said methods further comprise the step of treating said individual for said disease or disorder.

In preferred embodiments, the disorder is prostate cancer and the biomarker pair is selected from Table 12 (from Example 3) or table 2. In preferred embodiments, the disorder is cHL and the biomarker pair is selected from table 4 or table 5. In preferred embodiments, the disorder is breast cancer and the biomarker pair is selected from table 6. In preferred embodiments, the disorder is Alzheimer's disease and the biomarker pair is selected from table 9.

The disclosure further provides for methods for characterizing the status of classical Hodgkin's lymphoma (cHL) in an individual and for collecting data regarding the health status of an individual. The methods comprise determining the quantity of one or more miRNAs from a bodily fluid sample from said individual and comparing the amount of the one or more miRNAs to a reference sample. The quantity of said miRNAs may be determined as already disclosed herein.

Example 6 describes the identification of miRNAs useful in classifying cHL. Accordingly, one or more miRNAs useful in these methods are selected from table 3. Preferably the one or more miRNAs are selected from let-7a-5p, miR-1908-5p, miR-5189-5p, miR-92b-3p, miR-425-3p, miR-625-3p, miR-7706, miR-125b-5p, miR-760, miR-21-5p, miR-122-5p, more preferably from miR-21-5p, let-7a-5p, miR-127-3p, and miR-155-5p.

The reference sample may have been obtained from a single individual or from the average of a population of individuals.

In some embodiments, the reference sample is from one or more healthy individuals (i.e, individuals not afflicted with cHL), one or more individuals having cHL, the same individual prior to receiving treatment for cHL, one or more individuals having a good response to treatment for the disorder, or from one or more individuals having a poor response to treatment for the disorder. For example, the quantity of one or more miRNAs as compared to reference levels obtained from heathy or afflicted individuals may be useful to characterize whether the individual is afflicted with cHL. The quantity of one or more miRNAs after treatment as compared to the levels in the individual prior to treatment may be useful to characterize the response to treatment, for example if the treatment was effective or if the patient has re-lapsed. The quantity of one or more miRNAs after treatment as compared to reference levels obtained from patients known to be either good or poor responders to treatment may be useful to characterize whether the individual has a good prognosis for treatment.

In a further aspect of the invention, the identification of biomarkers/biomarker pairs and well as to the characterization of a health status said biomarkers/biomarker pairs could be improved by purifying the bodily fluid, i.e., separating the fluid into different biochemical fractions. The majority of extracellular ncRNAs in blood are associated with soluble biochemical fractions including protein-complexes, lipid vesicles (LDL and HDL) and extracellular vesicles. In preferred embodiments, the bodily fluid is enriched for either extracellular vesicles (i.e., exosomal vesicles) or for a protein rich (protein/HDL) fraction, preferably using size exclusion chromatography. Figure 14E depicts the separation of exosomal vesicles and the protein rich fraction using size exclusion chromatography and an example of the difference in miRNA distribution between these two fractions. Table 4 further discloses biomarker pairs that can distinguish the exosomal vesicle fraction in blood between healthy donors and cHL patients. Table 5 discloses biomarker pairs that can distinguish the protein fraction in blood between healthy donors and cHL patients.

The invention is further explained in the following examples. These examples do not limit the scope of the invention, but merely serve to clarify the invention.

EXAMPLES

**Example 1**

Epstein Barr Virus (EBV)-transformed lymphoblastoid B cells (LCLs), constitutively secrete large quantities of endosome-derived extracellular vesicles (EV) called exosomes that incorporate human and viral miRNAs. Copy number measurements demonstrated that exosomes mediate cell-cell transmission of miRNAs leading to accumulation in recipient cells and target mRNA repression (Pegtel et al., 2010). Increasing in vitro and in vivo data suggest that miRNA sorting at endosomal membranes supports the function of exosomes in cell-cell communication (van Balkom et al., 2013; Kosaka et al., 2010; Mittelbrunn et al., 2011; Montecalvo et al., 2012; Pegtel et al., 2010; Umezu et al., 2013; Zhang et al., 2010; Zhuang et al., 2012). Notably, interfering with exosomes formation at endosomal membranes reduces functional miRNA secretion (Kosaka et al., 2013; Mittelbrunn et al., 2011). Although secretion of miRNAs through EVs may occur in a non-random fashion (Bellingham et al., 2012; Guduric-Fuchs et al., 2012; Nolte-'t Hoen et al., 2012; Palma et al., 2012), a thorough comparative analysis in purified exosomes populations and the producing cells has been lacking.

Small non-coding RNA families are differentially distributed between B cells and exosomes.

To determine whether small RNA variety of less than 200 nucleotides undergo selection for incorporation into exosomes, we generated cellular and corresponding exosomal small RNA libraries for sequencing of EBV-driven LCLs (n=3) and three lymphoma cell lines (n=3). This approach provided a comprehensive dataset to perform high-powered statistical analysis on the intra- and extra-cellular RNA repertoires within a defined cellular background. RNA-Seq yielded >1 million genome-mapped reads per sample (individual reads and alignment statistics in Figure 1) that were aligned to both the human and EBV genomes. Comparison to RNA reference libraries revealed that cellular and exosomal small RNA fractions contained products from diverse classes of RNAs . Besides a large diversity of small RNA sequences, it seems that several RNA classes are enriched in cells (Figure 1; black bars), while others are overrepresented in exosomes (gray bars). In all transformed B cell types, the class of miRNAs (miRBase v19) represents around 50% of the small RNA pool. In exosomes however this percentage was only 20% indicating that miRNAs are retained in cells. RNA elements derived from the other ncRNA classes (i.e. tRNAs, piRNAs, rRNAs, Y RNAs, and vault RNAs) were generally enriched in the exosomes even though the class distribution was distinct between cell types (Figure 1B). Notably, the expression of tRNAs and tRNA-derived fragments are deregulated in diffuse large B cell lymphomas (DLBCL) (Maute et al., 2013) and other tumor tissues (Lee et al., 2009b). In DLBCL-derived exosomes tRNA-derived fragments were highly enriched compared to LCL-derived exosomes (24% vs 7.4%; Figure 1B). The most striking distinction between LCL and lymphoma exosomes was the extreme abundance of human Y RNAs fragments (38%; Figure 1). To investigate whether the observed read abundance of small RNA fragments (Figure 1) reflects the presence of full-length transcripts we performed semi-quantitative stem-loop RT-PCR on the exosomal RNA that was subjected to sequencing. We detected high levels of intact Y RNA variety (i.e. RNY1, RNY3, RNY4 and RNY5) and vault RNA1-1 (Figure 1) in exosomes. Intact Y RNA and vault RNAs were also detected in vesicle fractions isolated from human urine samples (I.V.B., D.K-L., and D.M.P., unpublished data). This data supports a previous suggestion that small cytoplasmic regulatory RNAs may have a non-cell autonomous function (Nolte-'t Hoen et al., 2012). Overall, we uncovered a consistent bias towards exosomal incorporation of RNA polymerase III derived Y RNA transcripts and their fragments that seems most pronounced for transformed B cells. Interestingly, human small RNAs, specifically Y RNAs are packaged in enveloped and non-enveloped viral particles (Garcia et al., 2009; Khan et al., 2007; Routh et al., 2012). The selective recruitment of small Pol III RNAs from the host cell into (retro) viral particles and exosomes implies that common molecular mechanisms drive the packaging process. This realization could aid future characterization of cellular components governing exosomes biogenesis and cargo selection (Koppers-Lalic et al., 2012).

High and low abundant microRNAs are non-randomly incorporated into exosomes While the transcription and biogenesis of miRNAs is well-documented (Ebert and Sharp, 2012), the mechanisms controlling miRNA turnover, subcellular trafficking, and release via exosomes, and how these various factors affect gene-regulatory activity remain incompletely understood (Ameres and Zamore, 2013).

To gain more insight into possible disparities between miRNA distribution in cells versus exosomes, we calculated Spearman correlations between normalized miRNA reads of all cellular samples and paired exosomes (n=12) and determined the variation among datasets. Cluster analysis separated the LCL samples from the lymphoma samples. For all samples Spearman correlations (r) ranged from 0.48 to 0.81. The LCL exosomes (r = 0.76 to 0.8;) and LCL cells (r = 0.72 to 0.76), showed relatively modest sample variability indicating their usefulness as biological replicates. Next, mapped miRNA counts were fitted in a generalized linear model using the R package edgeR (Robinson et al., 2010) and the relative abundance of individual miRNAs in cells and exosomes were compared. The most abundant cellular miRNAs (>10.000 reads per million (RPM)) in general represented the most abundant miRNAs in the exosomes (Table S2, data 1). Although the miRNA distribution in exosomal fractions is clearly influenced by cellular miRNA abundance, we identified a subset of miRNAs that were discordantly distributed between cells and exosomes (false discovery rate FDR=0.05.

To investigate which miRNAs are preferentially retained or released in LCL, Burkitt Lymphoma (BL), and DLBCL backgrounds, all miRNAs were grouped and ranked

according to the fold enrichment in exosomes versus cellular miRNA reads (RPM). We observed that the most discordantly distributed miRNAs were preferentially retained (15 to 42%) rather than preferentially released (8 to 15%) (Figure 2A). The recurrence of retained and released miRNAs in their cellular vs. exosomal fractions demonstrated that the number of released miRNAs shared by all sample groups is significantly different than could be expected from chance (z-score = 26.2). Importantly, many 'released' miRNAs have a high relative abundance of more than 100-1000 RPM (Figure 2B-C) that is sufficient for repressive gene-regulatory activity (Mullokandov et al., 2012).

To assess whether the distribution of miRNAs defined as 'retained' or 'released' is detectable by other methodologies, we prepared new batches of B cells secreted exosomes (biological duplicates) and extracted RNA from cells and exosomes. Next, we performed stem-loop based quantitative RT-PCR analysis and observed that released human miRNAs miR-451, miR-127-3p and miR-410 were highly abundant in exosomes fractions consistent with the RNA-seq approach. Strikingly, miR-451 and 127-3p were barely detectable in cells, in contrast to preferentially "retained" miRNAs miR-1275, miR-744 and miR-130b (Figure 2D). The expulsion of miR-127-3p could be related to its function as a negative regulator of B-cell lymphoma 6 (Bcl6) mRNA, a transcriptional repressor in lymphomagenesis (Lujambio and Esteller, 2007; Parekh et al., 2007). Indeed, miR-127-3p shows tumor suppressive activity (Saito et al., 2006) and its release could favor the growth and survival of B cell lymphoma cells.

Differences in human and viral miRNA distribution

EBV-miRNAs provide essential growth advantages to EBV-infected proliferating B cells and EBV-driven lymphomas (Feederle et al., 2011; Seto et al., 2010; Vereide et al., 2013). We postulated that in contrast to endogenous tumor-suppressor miRNAs, EBV-miRNAs would be preferentially retained and underrepresented in exosomes. We grouped and analyzed the distribution of 44 miRNAs encoded by EBV in the transformed B cells and lymphoma cells (Qiu et al., 2011). In line with previous RNA-seq studies on EBV-infected B cell lines (Riley et al., 2012; Skalsky et al., 2012), we confirmed that EBV-miRNA abundance varies widely and the most abundant ones belonged to the BART-cluster miRNAs. We calculated the ratio between the amount of all miRNAs released from the cells and the amount retained in the cell, after read normalization based on the frequency of their distribution (Figure 4A). Whereas host miRNAs are prone for release (17% are cell-retained), the majority of viral miRNAs are precluded from secretion (54% are strong-retained). This is illustrated in Figure 4B where the viral miRNAs encoded by EBV are indicated in red. This observation reconciles results from Riley et al who found that the abundant EBV BART cluster miRNAs target 132 apoptosis-related cellular mRNAs (Riley et al., 2012), suggesting that EBV miRNAs prevent cell death. Gene ontology analysis indicated that many host miRNAs known to repress the MAPK/PI3K survival pathway are preferentially released. The observation of a coherent, non-random distribution of physiologically relevant miRNAs is consistent with a mechanism that mediates sorting into exosomes.

3'end NTAs define retention or release of miRNAs in vitro and in vivo

The most abundant miRNA sequences from pre-miRNA hairpins are normally used to define the corresponding miRNAs, commonly termed as mature miRNAs (miRBase). However, the analysis of individual miRNA reads and their mappings to the corresponding pre-miRNA sequence, indicated extensive sequence variations at the 3' end of miRNAs in cellular and exosomal RNA fractions. Inefficient Drosha or Dicer-mediated processing of pre-miRNAs produces 3'end length variants (e.g. truncations and elongations). Additionally, miRNA isoforms are generated actively by nucleotidyl transferase-mediated posttranscriptional modifications also referred to as non-templated terminal nucleotide additions (NTA) (Burroughs et al., 2010; Morin et al., 2008; Wyman et al., 2011) (Figure 5A). Global analysis of miRNA 3'end variations showed that 3'end length isoforms are equally distributed between cells and exosomes, while miRNAs with 3'end NTAs exhibited differential distribution. The comparison of individual NTA types (i.e. A, U, C or G) to the sum of all reads with NTAs showed an enrichment of adenylated miRNA isoforms in the cellular fractions. In contrast, 3'end uridylated miRNAs were overrepresented in exosomes of all LCL samples analyzed (Figure 5C). To analyze whether the proportion of NTA-modified reads (A, U, C or G) changes significantly between cell and exosome samples, each miRNA sequence with 3'end NTAs was extracted from all 12 samples (LCLs n=6, BLs n=4, DLBCL n=2) and fitted in a logistic model, which also corrected for the cell line effect (accounting for the paired design). The results suggest that 3'end adenylated miRNA isoforms are preferentially retained (chi-square test, p<0.05), whilst all other NTAs are preferentially released (3'end-U: chi-square test, p=0.02; 3'end-C: Fisher's exact test, p=0.04; and 3'end-G: Fisher's exact test, p=0.02.

Apart from a global NTA distribution analysis, in three LCL samples (i.e. biological triplicates) we observed that the disparity of 3'end adenylated or uridylated miRNA isoforms between cells and exosomes was significant (p=0.005, student t-test) and not observed for mature miRNA (miRBase-based annotation) sequences or unmodified (elongated or truncated) isoforms. To analyze the effect of 3'end NTAs on distribution of individual miRNAs, we selected a 100 abundantly expressed miRNAs in LCL cells and matched exosome fractions for a pair-wise analysis. The results demonstrated that sequences of individual miRNAs with 3'end adenylation are more associated with cellular fractions, whereas the same miRNAs but with 3'end uridylation are more prone to exosomal release. We thus conclude that the type of added nucleotide, i.e. adenine or uridine, influences miRNA isoform distribution between cells and exosomes.

Deeper analysis of individual miRNA sequences allowing more mismatches and longer flanking regions (+6 nt) outside defined and annotated 3'end sequence revealed that 3'ends of many miRNAs are extended with more than one non-template nucleotide. To address whether the number of post-transcriptionally added nucleotides has a cumulative effect on the distribution, we first calculated the ratio of the fold-changes between cells and exosomes (defined as Expel Coefficient; see experimental procedures) for sequence reads with a particular NTA type with non-modified forms, taking into account the number of added nucleotides. 3'end adenylation increases the probability for retention and this probability increases proportionally with the number of added A's (Figure 8B-C). Strikingly, tri-adenylated human miRNAs were 5 times more frequent in LCL cells than in the corresponding exosomes (p=0.03; unpaired t test), whereas tri-adenylated viral miRNAs are only detected in LCL cell fractions (Figure 8C). The association of 3'end adenylation with an increase in cellular retention of viral miRNAs further corroborates our observation that viral miRNAs exhibit strong tendency to remain cell-associated (Figure 4A-B). Importantly, we did not observe extensive 3'end poly-adenylation (more than 4 adenines) that signals for miRNA degradation as recently demonstrated by Backes et al. (Backes et al., 2012).

In contrast to 3'end adenylation, the disproportional occurrence of small RNAs with uridines at the 3'ends seems to define exosomal RNA cargo. To investigate whether this is not a phenomenon restricted to B-cells in culture (Figure 8F), we collected and purified naturally occurring extracellular vesicles from human urine using an exosomes-isolation protocol (Bijnsdorp et al., 2013; Verweij et al., 2013). We sequenced the small RNA content from six urine exosomes samples derived from healthy individuals (Figure 10). In full accordance with 3'end uridylation-mediated miRNA isoforms distribution observed in B cell exosomes (Figure 8F; p-value p<0.005; Student t-test), human urine exosomes are significantly enriched in 3'end uridylated miRNAs (Figure 8F; p-value <0.0001; Student t-test). Collectively these data indicate that in vitro as well as in vivo, 3'end uridylated miRNAs are preferentially released by cells, and that cellular retention of miRNA isoforms depends on the number of adenine nucleotides added to the 3'end of the miRNA.

The impact of 3'end NTAs on exosomal sorting of individual miRNAs

Our results showed that NTAs define miRNA distribution between cells and exosomes. However, the impact of NTAs on individual miRNAs may be distinct. For example 3'end uridylated reads of abundant exosome-enriched miRNAs 486-5p, 143-3p and 101-3p show an above average percentage (38%) of all uridylated isoforms present in exosomal fraction. Yet, miR-486-5p is seemingly equally distributed at the mature miRNA level (Figure 7C; in gray). Notably, 50% of all reads mapping to the mature miR-486-5p sequence are either adenylated or uridylated isoforms (Figure 7C). When using the weighted means of all miRNAs in LCL samples we observed that tri-uridylated reads are 9 times more frequent in LCL exosomes compared to the cellular content (p=0.01; unpaired t-test). To confirm this difference in distribution we developed stem-loop-based primers designed to distinguish miR-486-5p 3'end tri-adenylated and 3'end tri-uridylated isoforms that are three nucleotides longer than the canonical (mature) miRBase-based sequence. As expected, the detection of mature miR-486-5p with no 3'end nucleotide additions shows a virtually equal distribution between cells and exosomes, whereas 3'end tri-adenylated isoforms were enriched in cells (10-fold) and 3'end tri-uridylation (5-fold) in exosomes (Figure 7D). This independent approach supports the prior RNAseq data (Figure 6) and demonstrates that different types of 3'end posttranscriptional modifications are detectable by RT-PCR and reflects a cellular retention and exosomal sorting process. Thus abundant miRNAs and their isoforms represent subpopulations with unique distribution properties depending on the type and the extent of 3'end posttranscriptional modification.

Apart from human and viral miRNAs, we found that adenylation and uridylation can also occur at the 3'ends of processed fragments originating from small cytoplasmic Y RNAs (Figure 9). Importantly, most of the 3'end-modified small RNA molecules followed the same fate as miRNA isoforms in regards to exosomal sorting indicating that NTAs modifications as a signal for sorting is not restricted to the class of miRNAs. These results underscore the relevance of 3'end NTA and expand our knowledge on diversity of small RNA substrates for the nucleotidyl-transferase family of enzymes (Martin and Keller, 2007).

The comparison between cellular and exosomal miRNA repertoire demonstrated conclusive evidence for selective exclusion and release of abundant miRNAs via exosomes. Moreover we observed that the degree of 3'end adenylation seems to impede the incorporation and secretion of miRNA isoforms via exosomes. This finding is consistent with the notion that this particular type of modification increases the stability and activity of certain miRNAs (Burns et al., 2011; D'Ambrogio et al., 2012; Jones et al., 2009). Importantly we found an opposite behaviour for 3'end uridylated miRNA isoforms. We conclude that miRNA trafficking, sorting, and release via exosomes, which is also evident in naturally occurring human urine vesicles, is defined in part by posttranscriptional modification through NTA. Currently the functional significance of these subtle 3'end modifications are becoming unravelled (Baccarini et al., 2011; D'Ambrogio et al., 2012; Jones et al., 2009; Katoh et al., 2009; Rüegger and Großhans, 2012; Scott and Norbury, 2013) although their exact role in selective miRNA turnover, abundance, and activity is far from understood and complex (Ameres and Zamore, 2013). Our study provides a further understanding of exosomal small RNA cargo selection and offers a rationale for studying miRNA processing, modification, and turn-over in connection to exosome biology.

EXPERIMENTAL PROCEDURES

Preparation of RNA samples for deep-sequencing

For each cellular or exosomal sample an equal amount of input RNA (600ng of RNA) was prepared for sequencing using the TruSeq small RNA sample prep following manufacturer's instruction (Illumina San Diego CA USA). Sequence libraries were measured on an Agilent 2100 Bioanalyzer (Agilent, Santa Clara CA USA) and up to 12 samples were equimolarly combined per run. Sequencing was performed on a HiSeq 2000 (Illumina San Diego CA USA) paired end 100 cycle (PE100) run. Detection of RNA reads with non-templated nucleotides additions and statistical analysis

MiRNA isoforms or isomiRs are retrieved by step-wise analysis. Briefly, for detection of NTAs at 3'-termini of mapped RNA elements we compared the read sequence to the aligned pre-miRNA sequence, starting at the nucleotide position 18 of the read. If the algorithm finds a mismatch position between the read and the pre-microRNA after position 18, the read is further analyzed from the mismatch position to the end of the read while all following nucleotides need be equal to the one at the mismatch position. If a different nucleotide is encountered, the search is continued at the next mismatch position or the read is tagged as non-NTA. After detecting all NTA reads, we calculate the weighted mean per sample by dividing the number of reads ending with NTA's of a given nucleobase (A, U, C and G) by total number of reads mapped to miRNAs. Per miRNA and per sample, we computed the total number of reads ending with NTAs of a given nucleobase, in addition to the total number of reads mapped to that miRNA. So, for each nucleobase, we could use the number of reads, in relation to the total, in a logistic regression model that also included the sample type (cell or exosome) as well as the cell line (accounting for the paired design). Based upon this model, we extracted p-values for the difference between proportions of miRNA counts with NTA of a given nucleotide, between cells and exosomes. After fitting this model per miRNA, Benjamini-Hochberg's false discovery rate (FDR) is applied to the p-values in order to correct for multiple testing.

Expel tendency of small RNAs with non-templated nucleotides additions

The assessment of the distribution of post-transcriptionally modified reads (NTAs) between cells and exosomes was performed as following. To test if miRNA-related reads have a tendency either to be incorporated into exosomes or to be retained within cells we compared those to the baseline distribution tendency of the given miRNA (defined by all other reads except the ones that belong to the analysed isomiR type). We define the expel coefficient (EC) by computing the ratio of the fold-changes between exosomes and cells for reads ending with a certain type of non-templated nucleotide (defined as NTA) and those without terminal modification (defined as noNTA). The coefficient will be close to 0 for which the NTA-reads and the non-NTA reads of a small ncRNA sequence have the same tendency to be released or retained. It will be positive if the NTA-reads have a stronger tendency to get released compared to the non-NTA reads. Finally, a negative coefficient indicates that NTA-reads have a stronger tendency to remain within the cells compared to non-NTA reads.

$$EC = \log_2 \left( \frac{RPM^{NTA}_{exosome}}{RPM^{NTA}_{cell}} \Bigg/ \frac{RPM^{noNTA}_{exosome}}{RPM^{noNTA}_{cell}} \right)$$

Exosomes collection procedures

For exosomes collection, each cell line was cultured in RPMI-1640 supplemented with 10% exosome-depleted FBS for three consecutive rounds followed by harvesting exosome-containing supernatant (one collection round represents one exosomes-containing preparation; 100x10^6 cells in 200 ml of medium). Cell death was analyzed routinely by trypan-blue exclusion, and cultures with ≤90%viability were not considered for exosome collection. Exosomes were isolated and purified from the supernatants using the differential centrifugation protocol as described (Verweij et al., 2013). Briefly, the final step involved pelleting of exosomes at 70,000 × g for 1 h and pellet washing with PBS prior to last ultracentrifugation step (70,000xg). Pelleted exosomes were dissolved in 100 μL PBS and stored at -80C. All exosomal preparations were analyzed by immunoblotting to confirm the presence and purity of exosomes (data not shown) as previously described (Pegtel et al., 2010; Verweij et al., 2011). For extraction of urine exosomes, fresh urine samples (50 ml volume) were collected from healthy males (n=6) after signed informed consent. Those healthy males are control group of patients that participate in a study unrelated to this project at the department of Urology (VU University Medical Center, Amsterdam, the Netherlands). Exosomes were isolated by differential centrifugation. First, urine was centrifuged at 500 x g for 20 min, at 2000 x g for 20 min, 10,000 x g for 30 min and 100,000 x g for 1.5 h with one additional washing with PBS. Intact exosomes were collected in PBS, and treated with RNAse A (400 ng/ml, Sigma-Aldrich Chemicals, Zwijndrecht, The Netherlands) before RNA isolation.

RNA extraction, quality assessment and semi-quantitative PCR assay

The RNA was extracted from cells and from paired exosome pellets. Exosomes preparations were pretreated with 400 ng/ul RNase A (Sigma) at 37°C for 1 hour. Total RNA was extracted from all exosomal preparations by TRIzol reagent (Life Technologies). When low yields were expected, 5 μl of glycogen (Roche) was added before the isopropanol precipitation step. To enable small RNA profile comparison between cellular samples and paired exosomes (known to contain the majority of small RNA varieties of <200 nt in size), cellular RNA was extracted by using glass fiber filter-based method (mirVana miRNA Isolation kit; Life Technologies) to enrich for small RNA varieties present in cellular samples. After extraction all cellular RNA samples comprised of small RNA varieties (<200 nt). Small cellular and exosomal RNA profiles exhibited similar patterns in size distribution. The quality and quantity of extracted small RNA (cellular and exosomal) was assayed using a small RNA chip platform (Agilent 2100 Bioanalyzer).

Presence of full length small non-coding RNAs in cellular and exosomal samples was detected by stem-loop based semi-quantitative reverse -transcriptase PCR (RT-PCR) with SYBR Green detection and analyzed by LightCycler480 (Roche). This method makes use of a stem-loop primer for RT reaction. Stem-loop primers are designed to complement a stretch of last eight nucleotides at the 3'-end of small RNA of interest and used at the final concentration of 12.5 nM. TaqMan MicroRNA RT kit (Applied Biosystems) was used, and the reactions were incubated according to manufacturer's instructions. Products were amplified by RNA-specific forward primers and by a reverse primer specific for the overlapping region of target and stem-loop RT primer. The analysis of amplified products was performed by LightCycler480 Software (Roche). Cellular and exosomal miRNAs were detected by TaqMan microRNA assays and Custom Taq-Man small RNA assays (Life Technologies) following manufacturer's instructions. All miRNA data is obtained from samples analyzed by Applied Biosystems 7500 Fast Real-Time PCR Systems and the analysis software provided. For all RT-PCR reactions an equal amount of RNA was used The results are presented as averaged cycle threshold (Ct) values (mean Ct values) of two technical replicates from duplicate experiments in figure 4B, except for figure 2D where Ct values were normalized for miR-92a using the $\Delta\Delta$Ct method. Samples with a Ct greater than 40 are regarded as negative.

Analysis of deep sequencing data and Expression profiling of small RNAs

The expression profiling of small RNAs was performed with a pipeline based on the miRanalyzer program (Hackenberg et al., 2011), including additional analysis steps and customized scripts. Briefly, the preprocessing of the reads in fastq format includes: i) adapter trimming (at least 10 nt of the adapter need to be detected allowing 1 mismatch between the read and the adapter sequence), ii) delete cleaned reads shorter than 15 nt, and iii) collapse all reads with identical sequences into one entry (unique reads). The read count of a unique read is the number that represents how many times the corresponding RNA molecule has been sequenced.

After adapter trimming and unique reads grouping, the reads are aligned by means of the Bowtie algorithm (Langmead et al., 2009) to a pooled index of the human (GRCh37 patch release 5; downloaded as hg19 from UCSC) and EBV genome (Gene Bank Accession number NC_007605). We use an alignment seed length of 19 bp allowing 1 mismatch retrieving only those alignments that map to less than 40 positions in the genome. In order to receive only the best alignments out of all those that satisfy the criterion, we applied a seed extension method as explained by Hackenberg et al. (Hackenberg et al., 2011). Note that the Bowtie seed alignment method allows aligning only the first L bases of a read. Non-genome-templated nucleotide additions (NTA) of adenine (A), uracil (U), cytosine (C) and guanine (G) are often detected by RNA sequencing at the 3'-ends of small RNAs. The post-transcriptionally added bases are not present in the genome sequence and therefore would be detected as mismatches in the alignments. The seed alignment allows detection of those reads as mismatches outside the seed region and will not be accounted for the allowed mismatches. After the alignment to the genome (human and EBV genomes combined), several annotations are used for the expression profiling of small RNA varieties. In general, the read position must be completely positioned within a region defined by the small RNA annotation: [chromosome start - 3 nt: chromosome end + 6 nt]. We allow flanking regions in order to be able to detect length variants and NTAs. The expression value of a given small RNA is simply the sum of all reads that map within the corresponding genome region. Furthermore, we calculate an adjusted read count (RC) by dividing the RC of each read by the number of mapped chromosome positions. Finally we calculate for each small RNA element the Read Per Million expression value (RPM) normalizing by means of the total number of reads mapped to a given RNA varieties. The RPM is the most common type of normalization making the expression value independent of the total number of reads.

To provide annotations to RNA elements that mapped to human and EBV genomes, all mapped reads were analyzed against currently known databases: 1) mature and pre-miRNA sequences from miRBase version 19 (Kozomara and Griffiths-Jones, 2011), 2) NCBI Reference Sequences human RefSeq genes downloaded 06/02/2013 (Pruitt et al., 2012), 3) tRNA sequences from the genomic tRNA database (Chan and Lowe, 2009) Repeat Derived sequences detected by means of the RepeatMasker algorithm downloaded from UCSC and 5) piwiRNA (piRNAs) downloaded from the NCBI nucleotide database. For those annotations in *fasta* format, we obtained the genome coordinates by mapping the sequences to the genome.

Statistical analysis for comparing cell and exosome samples

The RNA-seq libraries involved one pair of libraries per cell line, corresponding to either RNA coming from whole cells or RNA from exosomes only. To compare abundance for each miRNA between cell and exosome samples, the observed counts were fitted in a generalized linear model using the R package edgeR (Robinson et al., 2010), which included the cell line accounting for the paired design, as well as the sample type (either cell or exosome). The model included not only common and trend dispersion, but also tagwise dispersion estimation, allowing thus for extra variability due to interlibrary fluctuation. The p-values corresponding to the likelihood-ratio test statistic for the sample type effect were corrected for multiple testing using Benjamini-Hochberg's false discovery rate (Benjamini, 2010). In addition we also compare the observed intersection between 6 samples to the random expectation. Briefly, we i) calculate the log2 ratios of expression values between exosomes and cells for all 6 samples, ii) extract all expelled (log2 >= 2) miRNAs, iii) extract the shared miRNAs for a given group, i.e. those that are expelled in all samples of this group, iv) extract 10,000 times randomly X miRNAs for each condition (X is the number of observed, i.e. expelled miRNAs), v) for each of the 10,000 random runs, we detect the number of shared miRNAs, vi) from the 10,000 random runs we calculate the mean and standard deviation of the randomly shared RNAs which gives as the expectation value and its fluctuation under random assumption, vii) by means of the observed number of shared RNAs and the random mean and standard deviation we calculate a z-score to assess the statistical significance. We consider z-scores of 1.645 or higher, which corresponds to $p \leq 0.05$, as significant.

**Example 2**

We selected an experiment on clear cell Renal Cell Carcinoma (ccRCC) that consists of 11 healthy (non-tumoral renal cortex -NRC) and 22 clear cell Renal Cell Carcinoma samples (Osanto, S. et al. PLoS One 2012). Furthermore, the 22 ccRCCs patients belonged to 3 prognostic sub-groups, i.e. without disease recurrence, with recurrence and with metastatic disease at diagnosis (stage IV). The data with SRA accession SRP012546 was downloaded from the Short Read Archive. To profile the miRNA expression pattern and to determine the degree of isomiRs we extended our previously developed widely used program, miRanalyzer that annotates complex (small) RNAseq data (Hackenberg, M. et al. Nucleic Acid Res. 2009 & 2011). After adapter trimming the unique reads are mapped to the human genome. To detect all sequence variants, we allow fluctuations compared to the canonical sequence: 3 nt fluctuation at the 5' end and 5 nt at the 3' end.

After detecting all reads that belong to a given miRNA the reads are hierarchically classified (assigned to an isomiR type): i) the canonical miRNA sequence, ii) non-templated additions (NTA) and iii) 5' and/or 3' sequence length variants (e.g. truncations or elongations) from the canonical sequence. The isomiR ratios are calculated for each miRNA and isomiR type. We define them as the number of reads that belong to a given isomiR type divided by the total number of reads mapped to a given miRNA (canonical read count plus all isomiRs).

After analyzing the data with our pipeline, we compared the observed isomiR ratios between all 4 experimental groups (healthy, without recurrence, with recurrence, stage IV). In order to obtain those miRNAs for which statistically significant differences exist between two groups we applied a standard t-test. We found pronounced differences for adenine additions (adenylation) between the clinically relevant groups. We extracted 4 miRNAs that showed a very high percentage of adenylated reads in at least one group (>= 35%) and at least one significant comparison. The 4 mature miRNAs are: miR-199b-3p, miR-125b-5p, miR-210-3p and miR-151a-3p. Figure 11 depicts the isomiR ratios of these four miRNAs for all 4 different patients groups.

Brief summary of preliminary results

1. Distinct miRNAs show a strong correlation between adenylation frequency and expression level including miR-210-3p which corresponds to stabilization of the mature miRNA sequence (D'Ambrogio et al. Cell Reports 2012). MiR-210-3p levels are increased during RCC progression which might be a consequence of miRNA stabilization through post-transcriptional modification in a form of 3'-end adenylation.

2. Between stage IV and healthy individuals, mature miR-199b-3p is not differentially expressed (see Figure 11) however highly significant differences (p-value: 3.83E-06) exist in the adenylation pattern. Furthermore, miR-199b-3p targets 4 highly relevant genes for renal carcinogenesis (MED6, KRT7, MET, JUNB). This miRNA is thus putatively involved in oncogenesis, but would not be detected by traditional analysis pipelines stressing the importance to independently determine isomiR content.

3. Individuals with ccRRC that do not show recurrence after treatment manifests miR-199b-3p adenylation ratios closer to healthy individuals (35%, p-value = 0.13) while those individuals with recurrence do have ratios closer to stage IV individuals (22%, pvalue = 4.72*10-4). For miR-125b-5p a very similar pattern can be observed (see Figure 11). This suggests that isomiRs have unexpected prognostic biomarker potential.

**Example 3:** IsomiRs in biofluids discriminate cancer subtypes and healthy patients Urine was collected from 4 healthy volunteers and 9 patients diagnosed with prostate cancer. Exosomes were collected and RNA was extracted as described in Example 1. Expression profiling and data analysis of small ncRNAs was performed as in Examples 1 and 2.

After detecting all reads that belong to a given miRNA the reads are hierarchically classified (assigned to an isomiR type): i) the canonical miRNA sequence, ii) non-templated additions (NTA) and iii) 5' and/or 3' trimmed variants. The isomiR ratios are calculated for each miRNA and isomiR type. The most statistically significant ratios (determined by p-value) are depicted in Figure 12. The ratio of variant to canonical is significant between healthy patients and those having prostate cancer.

**Example 4:** Size-exclusion chromatography (SEC) for single step isolation of vesicles from human plasma

Measuring circulating ncRNAs, for example (miRNAs), in the blood maybe useful for early cancer detection, prognosis and monitoring. Exhaustive RNA sequencing on tissues and cultured cells reveals a complex and dynamic repertoire of ncRNAs. Over 2000 mature miRNA species have been identified of which many have been detected in circulation and other biofluids such as saliva and urine. However a single genomic locus can produce many, functionally distinct ncRNA variants.

The majority of extracellular ncRNAs in blood are associated with soluble biochemical fractions including protein-complexes, lipid vesicles (LDL and HDL) and extracellular vesicles. Comprehensive ncRNA profiling in total serum or plasma is tedious due to technical restraints. Small RNA library preparation of circulating small RNAs and their variants requires a high-enough enrichment and purity of intact ncRNA species that are available for adapter-ligation.

In an effort to increase the signal to noise ratio for measuring ncRNAs relevant to an individual's heath status, we measured miRNAs in purified exosomal vesicles (EVs) and in the protein fraction as follows.

Pre-treatment Sepharose CL-2B

- Pour 20 mL of sepharose CL-2B (per column) in a beaker and let the sepharose CL-2B settle for at least 15 minutes.
- Discard supernatant
- Add 15 mL buffer and mix gently by swirling
- Let the sepharose settle for at least 15 minutes
- Repeat wash twice
- Discard the supernatant and add 10 mL of buffer

Column preparation

- Use a 10 mL syringe
- Place press the nylon panty hose into the outlet of the syringe
- Pipet the washed sepharose into the column (use plastic Pasteur pipet)
- Let the sepharose settle, avoid column from running dry; column should be nicely vertical, no angle (use level)
- Add sepharose until 10 mL of stacked sepharose is reached
- Avoid column from running dry. Add buffer until use or close syringe outlet wit plug (use column within few hours)

Sample loading

Let the buffer run almost completely into the sepharose column (but prevent column from running dry)

- Load 1.5 mL of plasma on the column
- Immediately start collecting samples of 0.5 mL
- When the plasma sample has almost completely entered the sepharose, carefully add buffer until the syringe is completely filled.
- Repeat the addition of buffer until all fractions are collected (up to 26 fractions)

Sequencing circulating small RNA from clinical samples

- Isolate total RNA from single fractions using the Trizol isolation method
- Measure RNA on bioanlazer and use RT-PCR for quality control
- Prepare small RNA library using manufacturer's guidelines (Illumina Truseq)
- Sequence library using manufacturers instructions (Illumina HiSeq)

Plasma obtained from a patient with classic Hodgkin's lymphoma was subjected to SEC as described above. As shown in figure 14, SEC allows the single-step isolation of circulating EVs from plasma. The miRNA distribution differs between the EV fraction and the protein/HDL fraction (figure 14E).

Using the above method, we discovered multiple vesicle-associated as well as protein fraction associated miRNAs that allow therapy response monitoring in Hodgkin's patients. These results are discussed in Example 5

**Example 5:** EVs isolated from the plasma of Hodgkin's lymphoma

Plasma obtained from a patient with Hodgkin's lymphoma was subjected to SEC as described in Example 4. The results are presented in Figures 16-18.

**Example 6:** IsomiRs in biofluids discriminate healthy individuals from Hodgkin's lymphoma patients. Isolation of extracellular vesicles and protein-bound RNA fractions has been carried out as described in Example 4 and 5. Expression profiling and data analysis of small ncRNAs was performed as in Examples 1 and 2.

After detecting all reads that belong to a given miRNA the reads are hierarchically classified (assigned to an isomiR type): i) the canonical miRNA sequence, ii) non-templated additions (NTA) and iii) 5' and/or 3' sequence variants (e.g. truncations or elongations) from the canonical sequence. The isomiR ratios are calculated for each miRNA and isomiR type. We define them as the number of reads that belong to a given isomiR type divided by the total number of reads mapped to a given miRNA (canonical read count plus all isomiRs). The most statistically significant ratios (determined by p-value) are depicted in Figure 15. The ratio of variant to canonical is significant between healthy individuals and those having Hodgkin's lymphoma.

**Example 7:** IsomiRs in biofluids discriminate breast cancer stage II from stage III of disease and breast cancer patients with disease relapse from no-relapse. Data source and experimental procedures are publicly available and accessible at http://www.ncbi.nlm.nih.gov/sra?term=SRP027589 and http://www.ncbi.nlm.nih.gov/bioproject?LinkName=sra_bioproject&from_uid=455584 Publicly available sequence data of ncRNA in sera was analysed to determine whether the ratio of ncRNA variants could be used to classify health status. Expression profiling and data analysis of small ncRNAs was performed as in Example 2.

After detecting all reads that belong to a given miRNA the reads are hierarchically classified (assigned to an isomiR type): i) the canonical miRNA sequence, ii) non-templated additions (NTA) and iii) 5' and/or 3' sequence variants (e.g. truncations or elongations) from the canonical sequence. The isomiR ratios are calculated for each miRNA and isomiR type. We define them as the number of reads that belong to a given isomiR type divided by the total number of reads mapped to a given miRNA (canonical read count plus all isomiRs). The most statistically significant ratios (determined by p-value) are depicted in table 6. The ratio of variant to canonical is significant between stage II patients and those having stage III breast cancer.

**Example 8:** IsomiRs in tissue discriminate non-tumor adjacent tissue from testicular germ cell tumors Data source and experimental procedures are publicly available and accessible at http://www.ncbi.nlm.nih.gov/sra?term=SRP007946 and http://www.ncbi.nlm.nih.gov/bioproject/154993. Publicly available sequence data of ncRNA in sera was analysed to determine whether the ratio of ncRNA variants could be used to classify health status.

Expression profiling and data analysis of small ncRNAs was performed as in Example 2.

After detecting all reads that belong to a given miRNA the reads are hierarchically classified (assigned to an isomiR type): i) the canonical miRNA sequence, ii) non-templated additions (NTA) and iii) 5' and/or 3' sequence variants (e.g. truncations or elongations) from the canonical sequence. The isomiR ratios are calculated for each miRNA and isomiR type. We define them as the number of reads that belong to a given isomiR type divided by the total number of reads mapped to a given miRNA (canonical read count plus all isomiRs). The most statistically significant ratios (determined by p-value) are depicted in table 7. The ratio of variant to canonical is significant between adjacent tissue and tissue from testicular germ cell tumors.

**Example 9:** IsomiRs in tissue discriminate colorectal normal tissue from colorectal tumor tissue and from colorectal cancer-related metastasis tissues Data source and experimental procedures are publicly available and accessible at http://www.ncbi.nlm.nih.gov/Traces/sra/?study=SRP022054 and http://www.ncbi.nlm.nih.gov/bioproject/ PRJNA201245 and Röhr C et al., "High-throughput miRNA and mRNA sequencing of paired colorectal normal, tumor and metastasis tissues and bioinformatic modeling of miRNA-1 therapeutic applications.", PLoS One, 2013 Jul 2;8(7):e67461. Publicly available sequence data of ncRNA in sera was analysed to determine whether the ratio of ncRNA variants could be used to classify health status.

Expression profiling and data analysis of small ncRNAs was performed as in Example 2.

After detecting all reads that belong to a given miRNA the reads are hierarchically classified (assigned to an isomiR type): i) the canonical miRNA sequence, ii) non-templated additions (NTA) and iii) 5' and/or 3' sequence variants (e.g. truncations or elongations) from the canonical sequence. The isomiR ratios are calculated for each miRNA and isomiR type. We define them as the number of reads that belong to a given isomiR type divided by the total number of reads mapped to a given miRNA (canonical read count plus all isomiRs). The most statistically significant ratios (determined by p-value) are depicted in table 8. The ratio of variant to canonical is significant between normal colon tissue and tissue from colorectal tumors, as well as between normal colon tissue and colorectal tumor-related metastasis as well as between colorectal tumor tissue and colorectal tumor-related metastasis.

**Example** 10: IsomiRs in biofluids discriminate healthy individuals from patients diagnosed with Alzheimer's disease

Data source and experimental procedures are publicly available and accessible at: http://www.ncbi.nlm.nih.gov/sra?term=SRP022043 and http://www.ncbi.nlm.nih.gov/bioproject?LinkName=sra_bioproject&from_uid=387967 and Leidinger P et al., "A blood based 12-miRNA signature of Alzheimer disease patients.", Genome Biol, 2013 Jul 29;14(7):R78.

Publicly available sequence data of ncRNA in sera was analysed to determine whether the ratio of ncRNA variants could be used to classify health status. Expression profiling and data analysis of small ncRNAs was performed as in Example 2.

After detecting all reads that belong to a given miRNA the reads are hierarchically classified (assigned to an isomiR type): i) the canonical miRNA sequence, ii) non-templated additions (NTA) and iii) 5' and/or 3' sequence variants (e.g. truncations or elongations) from the canonical sequence. The isomiR ratios are calculated for each miRNA and isomiR type. We define them as the number of reads that belong to a given isomiR type divided by the total number of reads mapped to a given miRNA (canonical read count plus all isomiRs). The most statistically significant ratios (determined by p-value) are depicted in table 9. The ratio of variant to canonical is significant between heathy individuals and those having Alzheimer's disease.

REFERENCES

Amaral, P.P., Dinger, M.E., Mercer, T.R., and Mattick, J.S. (2008). The eukaryotic genome as an RNA machine. Science 319, 1787-1789.

Ameres, S.L., and Zamore, P.D. (2013). Diversifying microRNA sequence and function. Nat. Rev. Mol. Cell Biol. 14, 475-488.

Ameres, S.L., Martinez, J., and Schroeder, R. (2007). Molecular basis for target RNA recognition and cleavage by human RISC. Cell 130, 101-112.

Ameres, S.L., Horwich, M.D., Hung, J.-H., Xu, J., Ghildiyal, M., Weng, Z., and Zamore, P.D. (2010). Target RNA-directed trimming and tailing of small silencing RNAs. Science 328, 1534-1539.

Baccarini, A., Chauhan, H., Gardner, T.J., Jayaprakash, A.D., Sachidanandam, R., and Brown, B.D. (2011). Kinetic analysis reveals the fate of a microRNA following target regulation in mammalian cells. Curr. Biol. 21, 369-376.

Backes, S., Shapiro, J.S., Sabin, L.R., Pham, A.M., Reyes, I., Moss, B., Cherry, S., and tenOever, B.R. (2012). Degradation of host microRNAs by poxvirus poly(A) polymerase reveals terminal RNA methylation as a protective antiviral mechanism. Cell Host Microbe 12, 200-210.

Balaj, L., Lessard, R., Dai, L., Cho, Y.-J., Pomeroy, S.L., Breakefield, X.O., and Skog, J. (2011). Tumour microvesicles contain retrotransposon elements and amplified oncogene sequences. Nat. Commun. 2, 180.

Van Balkom, B.W.M., de Jong, O.G., Smits, M., Brummelman, J., den Ouden, K., de Bree, P.M., van Eijndhoven, M.A.J., Pegtel, D.M., Stoorvogel, W., Würdinger, T., et al. (2013). Endothelial cells require miR-214 to secrete exosomes that suppress senescence and induce angiogenesis in human and mouse endothelial cells. Blood 121, 3997-4006, S1-15.

Bellingham, S.A., Coleman, B.M., and Hill, A.F. (2012). Small RNA deep sequencing reveals a distinct miRNA signature released in exosomes from prion-infected neuronal cells. Nucleic Acids Res. 40, 10937-10949.

Bijnsdorp, I. V, Geldof, A.A., Lavaei, M., Piersma, S.R., van Moorselaar, R.J.A., and Jimenez, C.R. (2013). Exosomal ITGA3 interferes with non-cancerous prostate cell functions and is increased in urine exosomes of metastatic prostate cancer patients. J. Extracell. Vesicles 2.

Burns, D.M., D'Ambrogio, A., Nottrott, S., and Richter, J.D. (2011). CPEB and two poly(A) polymerases control miR-122 stability and p53 mRNA translation. Nature 473, 105-108.

Burroughs, A.M., Ando, Y., de Hoon, M.J.L., Tomaru, Y., Nishibu, T., Ukekawa, R., Funakoshi, T., Kurokawa, T., Suzuki, H., Hayashizaki, Y., et al. (2010). A comprehensive survey of 3' animal miRNA modification events and a possible role for 3' adenylation in modulating miRNA targeting effectiveness. Genome Res. 20, 1398-1410.

Chairoungdua, A., Smith, D.L., Pochard, P., Hull, M., and Caplan, M.J. (2010). Exosome release of β-catenin: a novel mechanism that antagonizes Wnt signaling. J. Cell Biol. 190, 1079-1091.

Chitwood, D.H., and Timmermans, M.C.P. (2010). Small RNAs are on the move. Nature 467, 415-419.

Chuma, S., and Pillai, R.S. (2009). Retrotransposon silencing by piRNAs: ping-pong players mark their sub-cellular boundaries. PLoS Genet. 5, e1000770.

Cullen, B.R. (2009). Viral and cellular messenger RNA targets of viral microRNAs. Nature 457, 421-425.

D'Ambrogio, A., Gu, W., Udagawa, T., Mello, C.C., and Richter, J.D. (2012). Specific miRNA stabilization by Gld2-catalyzed monoadenylation. Cell Rep. 2, 1537-1545. Ebert, M.S., and Sharp, P.A. (2012). Roles for microRNAs in conferring robustness to biological processes. Cell 149, 515-524.

Feederle, R., Haar, J., Bernhardt, K., Linnstaedt, S.D., Bannert, H., Lips, H., Cullen, B.R., and Delecluse, H.-J. (2011). The members of an Epstein-Barr virus microRNA cluster cooperate to transform B lymphocytes. J. Virol. 85, 9801-9810. Fernandez-Valverde, S.L., Taft, R.J., and Mattick, J.S. (2010). Dynamic isomiR regulation in Drosophila development. RNA 16, 1881-1888.

Garcia, E.L., Onafuwa-Nuga, A., Sim, S., King, S.R., Wolin, S.L., and Telesnitsky, A. (2009). Packaging of host mY RNAs by murine leukemia virus may occur early in Y RNA biogenesis. J. Virol. 83, 12526-12534.

Gibbings, D.J., Ciaudo, C., Erhardt, M., and Voinnet, O. (2009). Multivesicular bodies associate with components of miRNA effector complexes and modulate miRNA activity. Nat. Cell Biol. 11, 1143-1149.

Guasparri, I., Bubman, D., and Cesarman, E. (2008). EBV LMP2A affects LMP1-mediated NF-kappaB signaling and survival of lymphoma cells by regulating TRAF2 expression. Blood 111, 3813-3820.

Guduric-Fuchs, J., O'Connor, A., Camp, B., O'Neill, C.L., Medina, R.J., and Simpson, D.A. (2012). Selective extracellular vesicle-mediated export of an overlapping set of microRNAs from multiple cell types. BMC Genomics 13, 357.

Guo, L., Yang, Q., Lu, J., Li, H., Ge, Q., Gu, W., Bai, Y., and Lu, Z. (2011). A comprehensive survey of miRNA repertoire and 3' addition events in the placentas of patients with pre-eclampsia from high-throughput sequencing. PLoS One 6, e21072.

Jones, M.R., Quinton, L.J., Blahna, M.T., Neilson, J.R., Fu, S., Ivanov, A.R., Wolf, D.A., and Mizgerd, J.P. (2009). Zcchc11-dependent uridylation of microRNA directs cytokine expression. Nat. Cell Biol. 11, 1157-1163.

Kai, Z.S., and Pasquinelli, A.E. (2010). MicroRNA assassins: factors that regulate the disappearance of miRNAs. Nat. Struct. Mol. Biol. 17, 5-10.

Katoh, T., Sakaguchi, Y., Miyauchi, K., Suzuki, T., Kashiwabara, S.-I., Baba, T., and Suzuki, T. (2009). Selective stabilization of mammalian microRNAs by 3' adenylation mediated by the cytoplasmic poly(A) polymerase GLD-2. Genes Dev. 23, 433-438. Khan, M.A., Goila-Gaur, R., Opi, S., Miyagi, E., Takeuchi, H., Kao, S., and Strebel, K. (2007). Analysis of the contribution of cellular and viral RNA to the packaging of APOBEC3G into HIV-1 virions. Retrovirology 4, 48.

Koppers-Lalic, D., Hogenboom, M.M., Middeldorp, J.M., and Pegtel, D.M. (2012). Virus-modified exosomes for targeted rna delivery; A new approach in nanomedicine. Adv. Drug Deliv. Rev.

Kosaka, N., Iguchi, H., Yoshioka, Y., Takeshita, F., Matsuki, Y., and Ochiya, T. (2010). Secretory mechanisms and intercellular transfer of microRNAs in living cells. J. Biol. Chem. 285, 17442-17452.

Kosaka, N., Iguchi, H., Hagiwara, K., Yoshioka, Y., Takeshita, F., and Ochiya, T. (2013). Neutral sphingomyelinase 2 (nSMase2)-dependent exosomal transfer of angiogenic microRNAs regulate cancer cell metastasis. J. Biol. Chem. 288, 10849-10859.

Lee, Y.S., Pressman, S., Andress, A.P., Kim, K., White, J.L., Cassidy, J.J., Li, X., Lubell, K., Lim, D.H., Cho, I.S., et al. (2009a). Silencing by small RNAs is linked to endosomal trafficking. Nat. Cell Biol. 11, 1150-1156.

Lee, Y.S., Shibata, Y., Malhotra, A., and Dutta, A. (2009b). A novel class of small RNAs: tRNA-derived RNA fragments (tRFs). Genes Dev. 23, 2639-2649.

Lujambio, A., and Esteller, M. (2007). CpG island hypermethylation of tumor suppressor microRNAs in human cancer. Cell Cycle 6, 1455-1459.

Martens-Uzunova, E.S., Olvedy, M., and Jenster, G. (2013). Beyond microRNA--novel RNAs derived from small non-coding RNA and their implication in cancer. Cancer Lett. 340, 201-211.

Martin, G., and Keller, W. (2007). RNA-specific ribonucleotidyl transferases. RNA 13, 1834-1849.

Maute, R.L., Schneider, C., Sumazin, P., Holmes, A., Califano, A., Basso, K., and Dalla-Favera, R. (2013). tRNA-derived microRNA modulates proliferation and the DNA damage response and is downregulated in B cell lymphoma. Proc. Natl. Acad. Sci. U. S. A. 110, 1404-1409.

Mittelbrunn, M., and Sanchez-Madrid, F. (2012). Intercellular communication: diverse structures for exchange of genetic information. Nat. Rev. Mol. Cell Biol. 13, 328-335. Mittelbrunn, M., Gutiérrez-Vázquez, C., Villarroya-Beltri, C., Gonzalez, S., Sanchez-Cabo, F., Gonzalez, M.A., Bernad, A., and Sanchez-Madrid, F. (2011). Unidirectional transfer of microRNA-loaded exosomes from T cells to antigen-presenting cells. Nat. Commun. 2, 282.

Montecalvo, A., Larregina, A.T., Shufesky, W.J., Stolz, D.B., Sullivan, M.L.G., Karlsson, J.M., Baty, C.J., Gibson, G.A., Erdos, G., Wang, Z., et al. (2012). Mechanism of transfer of functional microRNAs between mouse dendritic cells via exosomes. Blood 119, 756-766.

Morin, R.D., O'Connor, M.D., Griffith, M., Kuchenbauer, F., Delaney, A., Prabhu, A.-L., Zhao, Y., McDonald, H., Zeng, T., Hirst, M., et al. (2008). Application of massively parallel sequencing to microRNA profiling and discovery in human embryonic stem cells. Genome Res. 18, 610-621.

Mullokandov, G., Baccarini, A., Ruzo, A., Jayaprakash, A.D., Tung, N., Israelow, B., Evans, M.J., Sachidanandam, R., and Brown, B.D. (2012). High-throughput assessment of microRNA activity and function using microRNA sensor and decoy libraries. Nat. Methods 9, 840-846.

Nolte-'t Hoen, E.N.M., Buermans, H.P.J., Waasdorp, M., Stoorvogel, W., Wauben, M.H.M., and't Hoen, P.A.C. (2012). Deep sequencing of RNA from immune cell-derived vesicles uncovers the selective incorporation of small non-coding RNA biotypes with potential regulatory functions. Nucleic Acids Res. 40, 9272-9285. Palma, J., Yaddanapudi, S.C., Pigati, L., Havens, M.A., Jeong, S., Weiner, G.A., Weimer, K.M.E., Stern, B., Hastings, M.L., and Duelli, D.M. (2012). MicroRNAs are exported from malignant cells in customized particles. Nucleic Acids Res. 40, 9125-9138.

Parekh, S., Polo, J.M., Shaknovich, R., Juszczynski, P., Lev, P., Ranuncolo, S.M., Yin, Y., Klein, U., Cattoretti, G., Dalla Favera, R., et al. (2007). BCL6 programs lymphoma cells for survival and differentiation through distinct biochemical mechanisms. Blood 110, 2067-2074.

Pegtel, D.M., Cosmopoulos, K., Thorley-Lawson, D.A., van Eijndhoven, M.A.J., Hopmans, E.S., Lindenberg, J.L., de Gruijl, T.D., Würdinger, T., and Middeldorp, J.M. (2010). Functional delivery of viral miRNAs via exosomes. Proc. Natl. Acad. Sci. U. S. A. 107, 6328-6333.

Pegtel, D.M., van de Garde, M.D.B., and Middeldorp, J.M. (2011). Viral miRNAs exploiting the endosomal-exosomal pathway for intercellular cross-talk and immune evasion. Biochim. Biophys. Acta 1809, 715-721.

Polikepahad, S., and Corry, D.B. (2013). Profiling of T helper cell-derived small RNAs reveals unique antisense transcripts and differential association of miRNAs with argonaute proteins 1 and 2. Nucleic Acids Res. 41, 1164-1177.

Qiu, J., Cosmopoulos, K., Pegtel, M., Hopmans, E., Murray, P., Middeldorp, J., Shapiro, M., and Thorley-Lawson, D.A. (2011). A novel persistence associated EBV miRNA expression profile is disrupted in neoplasia. PLoS Pathog. 7, e1002193.

Riley, K.J., Rabinowitz, G.S., Yario, T.A., Luna, J.M., Darnell, R.B., and Steitz, J.A. (2012). EBV and human microRNAs co-target oncogenic and apoptotic viral and human genes during latency. EMBO J. 31, 2207-2221.

Robinson, M.D., McCarthy, D.J., and Smyth, G.K. (2010). edgeR: a Bioconductor package for differential expression analysis of digital gene expression data. Bioinformatics 26, 139-140.

Routh, A., Domitrovic, T., and Johnson, J.E. (2012). Host RNAs, including transposons, are encapsidated by a eukaryotic single-stranded RNA virus. Proc. Natl. Acad. Sci. U. S. A. 109, 1907-1912.

Rüegger, S., and Großhans, H. (2012). MicroRNA turnover: when, how, and why. Trends Biochem. Sci. 37, 436-446.

Saito, Y., Liang, G., Egger, G., Friedman, J.M., Chuang, J.C., Coetzee, G.A., and Jones, P.A. (2006). Specific activation of microRNA-127 with downregulation of the proto-oncogene BCL6 by chromatin-modifying drugs in human cancer cells. Cancer Cell 9, 435-443.

Scott, D.D., and Norbury, C.J. RNA decay via 3' uridylation. Biochim. Biophys. Acta 1829, 654-665.

Scott, D.D., and Norbury, C.J. (2013). RNA decay via 3' uridylation. Biochim. Biophys. Acta - Gene Regul. Mech. 1829, 654-665.

Seto, E., Moosmann, A., Grömminger, S., Walz, N., Grundhoff, A., and Hammerschmidt, W. (2010). Micro RNAs of Epstein-Barr virus promote cell cycle progression and prevent apoptosis of primary human B cells. PLoS Pathog. 6, e1001063.

Sim, S., Weinberg, D.E., Fuchs, G., Choi, K., Chung, J., and Wolin, S.L. (2009). The subcellular distribution of an RNA quality control protein, the Ro autoantigen, is regulated by noncoding Y RNA binding. Mol. Biol. Cell 20, 1555-1564.

Skalsky, R.L., Corcoran, D.L., Gottwein, E., Frank, C.L., Kang, D., Hafner, M.,

Nusbaum, J.D., Feederle, R., Delecluse, H.-J., Luftig, M.A., et al. (2012). The viral and cellular microRNA targetome in lymphoblastoid cell lines. PLoS Pathog. 8, e1002484. Song, L., Lin, C., Gong, H., Wang, C., Liu, L., Wu, J., Tao, S., Hu, B., Cheng, S.-Y., Li, M., et al. (2013). miR-486 sustains NF-κB activity by disrupting multiple NF-κB-negative feedback loops. Cell Res. 23, 274-289.

Umezu, T., Ohyashiki, K., Kuroda, M., and Ohyashiki, J.H. (2013). Leukemia cell to endothelial cell communication via exosomal miRNAs. Oncogene 32, 2747-2755. Vereide, D.T., Seto, E., Chiu, Y.-F., Hayes, M., Tagawa, T., Grundhoff, A., Hammerschmidt, W., and Sugden, B. (2013). Epstein-Barr virus maintains lymphomas via its miRNAs. Oncogene.

Verweij, F.J., van Eijndhoven, M.A.J., Middeldorp, J., and Pegtel, D.M. (2013). Analysis of viral microRNA exchange via exosomes in vitro and in vivo. Methods Mol. Biol. 1024, 53-68.

Villarroya-Beltri, C., Gutiérrez-Vázquez, C., Sanchez-Cabo, F., Pérez-Hernández, D., Vázquez, J., Martin-Cofreces, N., Martinez-Herrera, D.J., Pascual-Montano, A., Mittelbrunn, M., and Sanchez-Madrid, F. (2013). Sumoylated hnRNPA2B1 controls the sorting of miRNAs into exosomes through binding to specific motifs. Nat. Commun. 4, 2980.

Wee, L.M., Flores-Jasso, C.F., Salomon, W.E., and Zamore, P.D. (2012). Argonaute divides its RNA guide into domains with distinct functions and RNA-binding properties. Cell 151, 1055-1067.

Wyman, S.K., Knouf, E.C., Parkin, R.K., Fritz, B.R., Lin, D.W., Dennis, L.M., Krouse, M.A., Webster, P.J., and Tewari, M. (2011). Post-transcriptional generation of miRNA variants by multiple nucleotidyl transferases contributes to miRNA transcriptome complexity. Genome Res. 21, 1450-1461.

Yao, B., La, L.B., Chen, Y.-C., Chang, L.-J., and Chan, E.K.L. (2012). Defining a new role of GW182 in maintaining miRNA stability. EMBO Rep. 13, 1102-1108.

Zhang, Y., Liu, D., Chen, X., Li, J., Li, L., Bian, Z., Sun, F., Lu, J., Yin, Y., Cai, X., et al. (2010). Secreted monocytic miR-150 enhances targeted endothelial cell migration. Mol. Cell 39, 133-144.

Zhuang, G., Wu, X., Jiang, Z., Kasman, I., Yao, J., Guan, Y., Oeh, J., Modrusan, Z., Bais, C., Sampath, D., et al. (2012). Tumour-secreted miR-9 promotes endothelial cell migration and angiogenesis by activating the JAK-STAT pathway. EMBO J. 31, 3513-3523.

**Claims**

1. A method for identifying a small non-coding RNA (ncRNA) biomarker pair, the method comprising

   - determining the quantity of two different varieties of said ncRNA in a first bodily fluid sample obtained from a first individual reference and in a second bodily fluid sample obtained from a second individual reference, wherein said first individual reference has an altered health status from said second individual reference,
   - determining the ratio of the two different varieties in the first bodily fluid sample,
   - determining the ratio of the two different varieties in the second bodily fluid sample,
   - comparing the ratios from said first and second bodily fluid sample, and
   - identifying the two different varieties of said ncRNA as a biomarker pair when the ratio is altered between said first and second bodily fluid sample, wherein the first and second varieties are selected from the canonical ncRNA; the ncRNA trimmed at the 5' or 3' end and/or extended at the 5' or 3' end; and the ncRNA with a 3' non-templated nucleotide addition, optionally trimmed at the 5' or 3' end or extended at the 5' or 3' end;
   wherein when said ncRNA is not an miRNA the first variety is selected from the canonical ncRNA and the ncRNA with a 3' non-templated nucleotide addition and the second variety is the ncRNA with a 3' non-templated nucleotide addition,
   wherein the ncRNA is selected from miRNA, tRNA, and Y-RNA.

2. The method of claim 1, wherein said first individual reference is from one or more healthy individuals and said second individual reference is from one or more individuals having a disorder.

3. The method of claim 2 wherein said disorder is prostate cancer.

4. The method of claim 1, wherein said first individual reference is from an individual having a disorder and said second individual reference is from the same individual following treatment of the disorder.

5. A method for classifying the health status of an individual using a small non-coding RNA (ncRNA) biomarker pair, the method comprising determining the quantity of the biomarker pair in a bodily fluid sample from said individual,
   determining the ratio of the biomarker pair in the bodily fluid sample,
   and comparing the ratio to the ratio of the biomarker pair in a bodily fluid from a reference sample,
   wherein the presence or absence in a difference in the ratio between the individual and the reference sample classifies the health status of said individual,
   wherein the biomarker pair consists of two different varieties of an ncRNA
   wherein the first and second varieties are selected from the canonical ncRNA; the ncRNA trimmed at the 5' or 3' end and/or extended at the 5' or 3' end; and the ncRNA with a 3' non-templated nucleotide addition, optionally trimmed at the 5' or 3' end or extended at the 5' or 3' end;
   wherein when said ncRNA is not an miRNA the first variety is selected from the canonical ncRNA and the ncRNA with a 3' non-templated nucleotide addition and the second variety is the ncRNA with a 3' non-templated nucleotide addition, wherein the ncRNA is selected from miRNA, tRNA, and Y-RNA.

6. A method for collecting data on the health status of an individual using a small non-coding RNA (ncRNA) biomarker pair, the method comprising determining the quantity of the biomarker pair in a bodily fluid sample from said individual and determining the ratio of the biomarker pair in the bodily fluid sample,
   wherein the biomarker pair consists of two different varieties of an ncRNA
   wherein the first and second varieties are selected from the canonical ncRNA; the ncRNA trimmed at the 5' or 3' end and/or extended at the 5' or 3' end; and the ncRNA with a 3' non-templated nucleotide addition, optionally trimmed at the 5' or 3' end or extended at the 5' or 3' end;
   wherein when said ncRNA is not an miRNA the first variety is selected from the canonical ncRNA and the ncRNA with a 3' non-templated nucleotide addition and the second variety is the ncRNA with a 3' non-templated nucleotide addition, wherein the ncRNA is selected from miRNA, tRNA, and Y-RNA.

7. The method according to claim 4 or 5, wherein the reference sample is from one or more healthy individuals or wherein the reference sample is from one or more individuals having a disorder.

8. The method according to claim 4 or 5, wherein the reference sample is from one or more individuals having a good response to treatment for a disorder.

9. The method according to any one of the preceding claims, wherein the two different varieties are selected from canonical and 3' NTA-A; canonical and 3' NTA-G; canonical and 3' NTA-C; canonical and 3' NTA-U; 3' NTA-G and 3' NTA-C; 3' NTA-G and 3' NTA-U; 3' NTA-G and 3' NTA-A; 3' NTA-C and 3' NTA-U; 3' NTA-C and 3' NTA-A; and 3' NTA-U and 3' NTA-A; canonical and 5' trimmed; canonical and 3' trimmed; 5' trimmed and 3' NTA-C; 5' trimmed and 3' NTA-U; 5' trimmed and 3' NTA-A; 5' trimmed and 3' NTA-G; 3' trimmed and 3' NTA-C; 3' trimmed: 3' NTA-U; 3' trimmed and 3' NTA-A; 3' trimmed and 3' NTA-G; and 3' trimmed and 5' trimmed; extended and 3' NTA-A; extended and 3' NTA-G; extended and 3' NTA-C; extended and 3' NTA-U; extended and 5' trimmed; extended and 3' trimmed; 5' or 3' trimmed and 5'or 3'extended; (trimmed and/or extended) and 3' NTA-U; (trimmed and/or extended) and 3' NTA-A; (trimmed and/or extended) and 3' NTA-G; (trimmed and/or extended) and 3' NTA-C; (trimmed and/or extended) and canonical.

10. The method according to any one of the preceding claims, wherein each ncRNA variety of said biomarker pair comprises at least 16 nucleotides.

11. The method according to any one of the preceding claims, wherein the biomarker pair is selected from one of the biomarker pairs depicted in Tables 1, 2, 4, 5, 6, and 9.

12. The method according to any one of the preceding claims, wherein the varieties are quantified using deep sequencing (RNA-seq).

13. The method of any one of the proceeding claims, further comprising isolating exosomal vesicles from the bodily fluid samples and determining the quantity of the two different varieties of said ncRNA associated with the isolated exosomal vesicles.

14. The method of any one of the proceeding claims, further comprising isolating the protein fraction from the bodily fluid samples and determining the quantity of the two different varieties of said ncRNA associated with the protein fraction.

15. The method of claim 13 or 14, wherein the exosomal vesicles or the protein fraction is isolated by subjecting the bodily fluid samples to size-exclusion chromatography (SEC).

**Patentansprüche**

1. Verfahren zum Identifizieren eines kleinen nichtkodierenden RNA (ncRNA) -Biomarkerpaars, das Verfahren umfassend

- Bestimmen der Menge von zwei verschiedenen Sorten der ncRNA in einer ersten Körperflüssigkeitsprobe, erhalten aus einer ersten individuellen Referenz, und in einer zweiten Körperflüssigkeitsprobe, erhalten aus einer zweiten individuellen Referenz, wobei die erste individuelle Referenz einen geänderten Gesundheitszustand gegenüber der zweiten individuellen Referenz hat,
- Bestimmen des Verhältnisses der beiden verschiedenen Sorten in der ersten Körperflüssigkeitsprobe,
- Bestimmen des Verhältnisses der beiden verschiedenen Sorten in der zweiten Körperflüssigkeitsprobe,
- Vergleichen der Verhältnisse der ersten und zweiten Körperflüssigkeitsprobe, und
- Identifizieren der zwei verschiedenen Sorten der ncRNA als ein Biomarkerpaar, wenn das Verhältnis zwischen der ersten und der zweiten Körperflüssigkeitsprobe geändert ist, wobei die ersten und die zweiten Sorten ausgewählt sind aus der kanonischen ncRNA; der ncRNA getrimmt an dem 5'- oder 3'-Ende und/oder verlängert an dem 5'- oder 3'-Ende; und der ncRNA mit einer 3'-nicht-Matrizen Nukleotidaddition, optional getrimmt am 5'- oder 3'-Ende oder verlängert an dem 5'- oder 3'-Ende; wobei, wenn die ncRNA keine miRNA ist, die erste Sorte ausgewählt ist aus der kanonischen ncRNA und der ncRNA mit einer 3'-nicht-Matrizen Nukleotidaddition und die zweite Sorte die ncRNA mit einer 3'-nicht-Matrizen Nukleotidaddition ist, wobei die ncRNA ausgewählt ist aus miRNA, tRNA, und Y-RNA.

2. Verfahren nach Anspruch 1, wobei die erste individuelle Referenz von einem oder mehreren gesunden Individuen ist und die zweite individuelle Referenz von einem oder mehreren Individuen mit einer Störung ist.

**3.** Verfahren nach Anspruch 2, wobei die Störung Prostatakrebs ist.

**4.** Verfahren nach Anspruch 1, wobei die erste individuelle Referenz von einem Individuum mit einer Störung ist und die zweite individuelle Referenz von derselben Person nach Behandlung der Störung ist.

**5.** Verfahren zum Klassifizieren des Gesundheitszustands eines Individuums unter Verwendung eines kleinen nichtkodierenden RNA (ncRNA)

- Biomarkerpaars, das Verfahren umfassend Bestimmen der Menge des Biomarkerpaars in einer Körperflüssigkeitsprobe von dem Individuum; Bestimmen des Verhältnisses des Biomarkerpaars in der Körperflüssigkeitsprobe, und Vergleichen des Verhältnisses mit dem Verhältnis des Biomarkerpaars in einer Körperflüssigkeit aus einer Referenzprobe, wobei die Anwesenheit oder Abwesenheit eines Unterschied in dem Verhältnis zwischen dem Individuum und der Referenzprobe den Gesundheitszustand des Individuums klassifiziert, wobei das Biomarkerpaar aus zwei verschiedenen Sorten einer ncRNA besteht wobei die erste und zweite Sorte ausgewählt sind aus der kanonischen ncRNA; der ncRNA getrimmt an dem 5'- oder 3'-Ende und/oder verlängert an dem 5'- oder 3'-Ende; und der ncRNA mit einer 3'-nicht-Matrizen Nukleotidaddition, optional getrimmt an dem 5'- oder 3'-Ende oder verlängert an dem 5'- oder 3'-Ende; wobei, wenn die ncRNA keine miRNA ist, die erste Sorte ausgewählt ist aus der kanonischen ncRNA und der ncRNA mit einer 3'-nicht-Matrizen Nukleotidaddition und die zweite Sorte die ncRNA mit einer 3'-nicht-Matrizen Nukleotidaddition ist, wobei die ncRNA ausgewählt ist aus miRNA, tRNA, und Y-RNA.

**6.** Verfahren zum Sammeln von Daten über den Gesundheitszustand eines Individuums unter Verwendung eines kleinen nichtkodierenden RNA (ncRNA) -Biomarkerpaars, das Verfahren umfassend Bestimmen der Menge des Biomarkerpaars in einer Körperflüssigkeitsprobe von dem Individuum und Bestimmen des Verhältnisses des Biomarkerpaars in der Körperflüssigkeitsprobe, wobei das Biomarkerpaar aus zwei verschiedenen Sorten einer ncRNA besteht wobei die erste und die zweite Sorte ausgewählt sind aus der kanonischen ncRNA; der ncRNA getrimmt an dem 5'- oder 3'-Ende und/oder verlängert an dem 5'- oder 3'-Ende; und der ncRNA mit einer 3'-nicht Matrizen Nukleotidaddition, optional getrimmt an dem 5'- oder 3'-Ende oder verlängert an dem 5'- oder 3'-Ende;

wobei, wenn die ncRNA keine miRNA ist, die erste Sorte ausgewählt ist aus der kanonischen ncRNA und der ncRNA mit einer 3'-nicht Matrizen Nukleotidaddition und die zweite Sorte die ncRNA mit einer 3'-nicht Matrizen Nukleotidaddition ist, wobei die ncRNA ausgewählt ist aus miRNA, tRNA, und Y-RNA.

**7.** Verfahren nach Anspruch 4 oder 5, wobei die Referenzprobe von einem oder mehreren gesunden Individuen ist oder wobei die Referenzprobe von einem oder mehreren Individuen mit einer Störung ist.

**8.** Verfahren nach Anspruch 4 oder 5, wobei die Referenzprobe von einem oder mehreren Individuen mit guter Antwort auf Behandlung einer Störung ist.

**9.** Verfahren nach einem der vorhergehenden Ansprüche, wobei die zwei verschiedenen Sorten ausgewählt sind aus kanonisch und 3'-NTA-A; kanonisch und 3' NTA-G; kanonisch und 3' NTA-C; kanonisch und 3' NTA-U; 3' NTA-G und 3' NTA-C; 3' NTA-G und 3' NTA-U; 3' NTA-G und 3' NTA-A; 3' NTA-C und 3' NTA-U; 3' NTA-C und 3' NTA-A; und 3' NTA-U und 3' NTA-A; kanonisch und 5' getrimmt; kanonisch und 3' getrimmt; 5' getrimmt und 3' NTA-C; 5' getrimmt und 3' NTA-U; 5' getrimmt und 3' NTA-A; 5' getrimmt und 3' NTA-G; 3' getrimmt und 3' NTA-C; 3' getrimmt; 3' NTA-U; 3' getrimmt und 3' NTA-A; 3' getrimmt und 3' NTA-G; und 3' getrimmt und 5' getrimmt; verlängert und 3' NTA-A; verlängert und 3' NTA-G; verlängert und 3' NTA-C; verlängert und 3' NTA-U; verlängert und 5' getrimmt; verlängert und 3' getrimmt; 5' oder 3' getrimmt und 5' oder 3' verlängert; (getrimmt und/oder verlängert) und 3' NTA-U; (getrimmt und/oder verlängert) und 3' NTA-A; (getrimmt und/oder verlängert) und 3' NTA-G; (getrimmt und/oder verlängert) und 3'-NTA-C; (getrimmt und/oder erweitert) und kanonisch.

**10.** Verfahren nach einem der vorhergehenden Ansprüche, wobei jede ncRNA-Sorte des Biomarkerpaars mindestens 16 Nukleotide umfasst.

**11.** Verfahren nach einem der vorhergehenden Ansprüche, wobei das Biomarkerpaar ausgewählt ist aus einem der Biomarkerpaare, dargestellt in Tabellen 1, 2, 4, 5, 6 und 9.

**12.** Verfahren nach einem der vorhergehenden Ansprüche, wobei die Sorten unter Verwendung von tiefer Sequenzierung (RNA-seq) quantifiziert werden.

**13.** Verfahren nach einem der vorstehenden Ansprüche, ferner umfassend Isolieren exosomaler Vesikel aus den Körperflüssigkeitsproben und Bestimmen der Menge der zwei verschiedenen Sorten der ncRNA, assoziiert mit den isolierten exosomalen Vesikeln.

**14.** Verfahren nach einem der vorstehenden Ansprüche, ferner umfassend Isolieren der Proteinfraktion aus den Körperflüssigkeitsproben und Bestimmen der Menge der zwei verschiedenen Sorten der ncRNA, assoziiert mit der Proteinfraktion.

**15.** Verfahren nach Anspruch 13 oder 14, wobei die exosomalen Vesikel oder die Proteinfraktion durch Aussetzen der Körperflüssigkeitsproben einer Größenausschlusschromatographie (SEC) isoliert werden.

**Revendications**

**1.** Un procédé pour identifier une petite paire de biomarqueurs d'ARN non codant (ARNnc), le procédé comprenant

    - la détermination de la quantité de deux variétés différentes dudit ARNnc dans un premier échantillon de fluide corporel obtenu à partir d'une première référence individuelle et dans un deuxième échantillon de fluide corporel obtenu à partir d'une deuxième référence individuelle, dans lequel ladite première référence individuelle présente un état de santé modifié par rapport à celui de ladite deuxième référence individuelle,
    - la détermination du rapport des deux variétés différentes dans le premier échantillon de fluide corporel,
    - la détermination du rapport des deux variétés différentes dans le deuxième échantillon de fluide corporel,
    - la comparaison des rapports desdits premier et deuxième échantillons de fluide corporel, et
    - l'identification des deux variétés différentes dudit ARNnc en tant que paire de biomarqueurs lorsque le rapport est modifié entre lesdits premier et deuxième échantillon de fluide corporel,

        dans lequel les première et deuxième variétés sont sélectionnées à partir de l'ARNnc canonique; l'ARNnc coupé à l'extrémité 5'ou 3' et/ou allongé à l'extrémité 5'ou 3'; et l'ARNnc avec une addition nucléotidique non-matricée en 3', éventuellement coupé à l'extrémité 5' ou 3' ou allongé à l'extrémité 5' ou 3';
        dans lequel lorsque ledit ARNnc n'est pas un ARNmi, la première variété est sélectionnée parmi l'ARNnc canonique et l'ARNnc avec une addition nucléotidique non-matricée en 3' et la deuxième variété est l'ARNnc avec une addition nucléotidique non-matricée en 3',
        dans lequel l'ARNnc est choisi parmi ARNmi, ARNt et ARN-Y.

**2.** Le procédé selon la revendication 1, dans lequel ladite première référence individuelle provient d'un ou plusieurs individus sains et ladite deuxième référence individuelle provient d'un ou plusieurs individus souffrant d'un trouble.

**3.** Le procédé selon la revendication 2, dans lequel ledit trouble est le cancer de la prostate.

**4.** Le procédé selon la revendication 1, dans lequel ladite première référence individuelle provient d'un individu souffrant d'un trouble et ladite seconde référence individuelle est issue du même individu après le traitement du trouble.

**5.** Un procédé de classification de l'état de santé d'un individu à l'aide d'une petite paire de biomarqueurs d'ARN non codant (ARNnc), le procédé comprenant

    - la détermination de la quantité de la paire de biomarqueurs dans un échantillon de fluide corporel à partir dudit individu,
    - la détermination du rapport de la paire de biomarqueurs dans l'échantillon de fluide corporel, et
    - la comparaison du rapport au rapport de la paire de biomarqueurs dans un fluide corporel d'un échantillon de référence,
    dans lequel la présence ou l'absence dans une différence de rapport entre l'individu et l'échantillon de référence classe l'état de santé dudit individu ,
    dans lequel la paire de biomarqueurs est constituée de deux variétés différentes d'un ARNnc
    dans lequel les première et deuxième variétés sont sélectionnées parmi l'ARNnc canonique; l'ARNnc coupé à l'extrémité 5'ou 3' et/ou allongé à l'extrémité 5'ou 3'; et l'ARNnc avec une addition nucléotidique non-matricée en 3', éventuellement coupé à l'extrémité 5' ou 3' ou allongé à l'extrémité 5' ou 3';
    dans lequel lorsque ledit ARNnc n'est pas un ARNmi, la première variété est sélectionnée parmi l'ARNnc canonique et l'ARNnc avec une addition nucléotidique non-matricée en 3' et la deuxième variété est l'ARNnc avec une addition nucléotidique non-matricée en 3', où l'ARNnc est sélectionné parmi ARNmi, ARNt et ARN-Y.

**6.** Un procédé pour collecter des données sur l'état de santé d'un individu utilisant une petite paire de biomarqueurs d'ARN non codant (ARNnc), le procédé comprenant la détermination de la quantité de la paire de biomarqueurs dans un échantillon de fluide corporel dudit individu et la détermination du rapport de la paire de biomarqueurs dans l'échantillon de fluide corporel,
dans lequel la paire de biomarqueurs consiste en deux variétés différentes d'un ARNnc

dans lequel les première et deuxième variétés sont sélectionnées à partir de l'ARNnc canonique; l'ARNnc coupé à l'extrémité 5' ou 3' et/ou allongé à l'extrémité 5'ou 3'; et l'ARNnc avec une addition nucléotidique non-matricée en 3', éventuellement coupé à l'extrémité 5' ou 3' ou allongé à l'extrémité 5' ou 3';

dans lequel lorsque ledit ARNnc n'est pas un ARNmi, la première variété est sélectionnée parmi l'ARNnc canonique et l'ARNnc avec une addition nucléotidique non-matricée en 3' et la deuxième variété est l'ARNnc avec une addition nucléotidique non-matricée en 3', où l'ARNnc est sélectionné parmi ARNmi, ARNt et ARN-Y.

7.  Le procédé selon la revendication 4 ou 5, dans lequel l'échantillon de référence provient d'un ou plusieurs individus sains ou dans lequel l'échantillon de référence provient d'un ou plusieurs individus présentant un trouble.

8.  Le procédé selon la revendication 4 ou 5, dans lequel l'échantillon de référence provient d'un ou plusieurs individus ayant une bonne réponse au traitement d'un trouble.

9.  Le procédé selon l'une quelconque des revendications précédentes, dans lequel les deux variétés différentes sont choisies parmi

    3' NTA-A et canonique; 3' NTA-G et canonique; 3' NTA-C et canonique;

    3' NTA-U et canonique; 3' NTA-G et 3'NTA-C; 3' NTA-G et 3'NTA-U;

    3' NTA-G et 3'NTA-A; 3' NTA-C et 3'NTA-U; 3' NTA-C et 3'NTA-A; et 3' NTA-U et 3'NTA-A; 5' coupé et canonique; 3' coupé et canonique;

    5' coupé et 3' NTA-C; 5' coupé et 3' NTA-U; 5' coupé et 3'NTA-A;

    5' coupé et 3' NTA-G; 3' coupé et 3' NTA-C; 3' coupé et 3'NTA-U;

    3' coupé et 3' NTA-A; 3' coupé et 3' NTA-G; 3' coupé et 5' coupé;

    3' NTA-A et allongé; 3' NTA-G et allongé; 3' NTA-C et allongé et

    3' NTA-U allongé ; 5' coupé et allongé; 3' coupé et allongé et;

    5' ou 3' coupé et 5' ou 3' allongé; 3' NTA-U et (coupé et/ou allongé);

    3' NTA-A et (coupé et/ou allongé); 3' NTA-G et (coupé et/ou allongé),

    3' NTA-C et (coupé et/ou allongé) et (coupé et/ou allongé) et canonique.

10. Le procédé selon l'une quelconque des revendications précédentes, dans lequel chaque variété d'ARNnc de ladite paire de biomarqueurs comprend au moins 16 nucléotides.

11. Le procédé selon l'une quelconque des revendications précédentes, dans lequel la paire de biomarqueurs est choisie parmi l'une des paires de biomarqueurs décrites dans les tableaux 1, 2, 4, 5, 6 et 9.

12. Le procédé selon l'une quelconque des revendications précédentes, dans lequel les variétés sont quantifiées en utilisant un séquençage profond (RNA-seq).

13. Le procédé selon l'une quelconque des revendications précédentes, comprenant en outre l'isolement des vésicules exosomales des échantillons de fluide corporel et la détermination de la quantité des deux variétés différentes dudit ARNnc associées aux vésicules exosomales isolées.

14. Le procédé selon l'une quelconque des revendications précédentes, comprenant en outre l'isolement de la fraction protéique des échantillons de fluide corporel et la détermination de la quantité des deux variétés différentes dudit ARNnc associées à la fraction protéique.

15. Le procédé selon la revendication 13 ou 14, dans lequel les vésicules exosomales ou la fraction protéique est isolée en soumettant les échantillons de fluide corporel à une chromatographie d'exclusion stérique (SEC).

**FIGURE 1**

A

Sample characteristics and small RNA sequencing results output

| Annotation | Sample name | Raw reads | Discarded | Unique reads | Mapped Read count |
|---|---|---|---|---|---|
| DLBCL | BJAB cells | 13337829 | 1939485 | 645476 | 11398344 |
| | BJAB exosomes | 6119426 | 4252910 | 188943 | 1866516 |
| LCL3 | RN cells | 9876776 | 816448 | 382036 | 9060328 |
| | RN exosomes | 7948331 | 2179359 | 407540 | 5768972 |
| LCL1 | IK140508 cells | 5946947 | 681212 | 352069 | 5265735 |
| | IK140508 exosomes | 6203008 | 1688894 | 319392 | 4514114 |
| LCL2 | IM-1 cells | 8111347 | 3936763 | 219709 | 4174584 |
| | IM-1 exosomes | 1936176 | 728832 | 125077 | 1207344 |
| BL1 | Mutu I clone 9 cells | 4954428 | 3393471 | 147551 | 1560957 |
| | Mutu I clone 9 exo | 8379288 | 4676116 | 460152 | 3703172 |
| BL2 | Mutu I clone 3 cells | 7494150 | 4867868 | 241594 | 2626282 |
| | Mutu I clone 3 exo | 11934244 | 7068282 | 558980 | 4865962 |

B

Samples and small RNA fractions used for RNA sequencing

| Sample group | Cell line | Fractions (RNA-seq) |
|---|---|---|
| Lymphoblastoid B-cell EBV+ (n=6) | LCL1 | Cell + Exo |
| | LCL2 | Cell + Exo |
| | LCL3 | Cell + Exo |
| Burkitt Lymphoma EBV+ (n=4) | BL1 | Cell + Exo |
| | BL2 | Cell + Exo |
| Diffuse Large B-cell Lymphoma (n=2) | DLBCL | Cell + Exo |

C

Distribution frequency of mapped reads between cells and exosomes

## FIGURE 2

A. Non-random distribution of miRNAs (all samples)

B. Sample: Lymphoblastoid cell lines (LCLs) - Released miRNAs (selective release through exosomes)

C. Sample: Lymphoblastoid cell lines (LCLs) - Retained miRNAs (selective retention in cells)

D. Sample: Lymphoblastoid cell lines (LCLs) - Retained versus released miRNAs detection by qRT-PCR method

FIGURE 3

A Burkitt's Lymphoma cell lines - Released miRNAs (in exosomes)

B Burkitt's Lymphoma cell lines - Retained miRNAs (in cells)

FIGURE 4

Lymphoblastoid cell lines (LCLs) are infected with Epstein Barr virus (EBV)
EBV encodes for at least 44 mature miRNAs of which the majority has strong
tendency to remain cell-associated

Non-random distribution of human and viral miRNAs in lymphoblastoid cell lines and their secreted exosomes

A

Relative frequency of miRNAs in %

virus miRNAs
human miRNAs

Strong Moderate Equal Moderate Strong
Release          Retention

B

Distribution of LCL human miRNAs – virus miRNAs

cell/exo (log2)

exo

common          cell retained

FIGURE 5

Post-transcriptional non-templated nucleotide additions (NTA) at the 3'end of miRNAs define their retention (cells) or release (exosomes)

**FIGURE 6**

Post-transcriptional non-templated nucleotide additions (NTA) at the 3'end of miRNAs define their retention (cells) or release (exosomes)

Statistical analysis

Logistic model, FDR for exo vs cell NTA U

Logistic model, FDR for exo vs cell NTA A

Logistic model, FDR for exo vs cell NTA G

Logistic model, FDR for exo vs cell NTA C

microRNAs

FDR

Exo>Cell
Cell>Exo

FIGURE 7

# Post-transcriptional non-templated nucleotide additions (NTA) at the 3'end of miRNAs define their retention (cells) or release (exosomes)

A

microRNAs

B Distribution of 3'end-NTA modified miRNA isoforms

C Sample: Lymphoblastoid cell lines (LCLs) – distribution of mature miR-486-5p and its isorofms in cells and exosomes

D Sample: Lymphoblastoid cell lines (LCLs) – mature miR-486-5p versus 3'-end modified miR-486-5p detection by qRT-PCR method

FIGURE 8

Post-transcriptional non-templated nucleotide additions at the 3'end of miRNAs define their retention (cells) or release (exosomes)

FIGURE 9

Post-transcriptional non-templated nucleotide additions at the 3'end of processed human Y RNAs
define their retention (cells) or release (exosomes)

**FIGURE 10**

List of mapped reads identified in urine exosomes by small RNA sequencing

| name (mapped sequences) | urine090 | urine088 | urine085 | urine081 | urine041 | urine035 |
|---|---|---|---|---|---|---|
| microRNAs | 165620 | 231333 | 146943 | 213502 | 610477 | 425119 |
| SRP RNAs | 288 | 300 | 327 | 200 | 62 | 193 |
| piRNA | 13419 | 6548 | 3785 | 2085 | 3007 | 5977 |
| Rnase P RNA | 20 | 23 | 22 | 42 | 5 | 15 |
| vault RNA | 146 | 92 | 126 | 106 | 20 | 40 |
| Y RNAs | 34087 | 31794 | 40742 | 41938 | 21857 | 19802 |
| RefSeq (ncRNAs) | 40072 | 35162 | 43483 | 33394 | 19111 | 30043 |
| tRNAs | 301504 | 269756 | 338741 | 338691 | 132178 | 222814 |
| snoRNAs | 98 | 60 | 51 | 147 | 53 | 72 |
| snRNAs | 47 | 63 | 50 | 59 | 130 | 46 |
| rRNA fragments | 131431 | 136238 | 99662 | 128293 | 23795 | 59346 |
| mRNA fragments | 44515 | 48319 | 38016 | 44911 | 27858 | 31978 |
| Human others (RepBase, anti-sense, misc_RNA) | 112944 | 111272 | 146317 | 101199 | 79164 | 97954 |
| unmapped or unassigned | 151729 | 118880 | 136499 | 91395 | 78826 | 101261 |

FIGURE 11

FIGURE 12

Table 1
C/V - canonical/variant
SEQ: - SEQ ID NO:

**Table 1A: Post-transcriptional modification to the canonical sequence of listed microRNAs; type of 3'-end NTA is Adenine (A)**

| microRNA | canonical (mature) sequence in miRBase | SEQ: | C/V NTA-A in healthy | STD healthy | C/V NTA-A in cancer | STD cancer | p-value |
|---|---|---|---|---|---|---|---|
| hsa-let-7g-5p | TGAGGTAGTAGTTTGTACAGTT | 1 | 0.135 | 0.001139395 | 0.217 | 0.001657857 | 0.004 |
| hsa-miR-30e-3p | CTTTCAGTCGGATGTTTACAGC | 2 | 0.042 | 3.35E-04 | 0.083 | 3.82E-04 | 0.004 |
| hsa-miR-200a-3p | TAACACTGTCTGGTAACGATGT | 3 | 0.121 | 2.97E-04 | 0.219 | 0.005998844 | 0.013 |
| hsa-let-7e-5p | TGAGGTAGGAGGTTGTATAGTT | 4 | 0.308 | 0.011296606 | 0.468 | 0.00713208 | 0.019 |
| hsa-miR-30a-5p | TGTAAACATCCTCGACTGGAAG | 5 | 1.946 | 0.180317998 | 2.576 | 0.123396748 | 0.022 |
| hsa-miR-204-5p | TTCCCTTTGTCATCCTATGCCT | 6 | 0.038 | 2.02E-04 | 0.021 | 2.98E-05 | 0.024 |
| hsa-miR-26a-5p | TTCAAGTAATCCAGGATAGGCT | 7 | 0.058 | 6.98E-05 | 0.046 | 6.27E-05 | 0.030 |
| hsa-let-7i-5p | TGAGGTAGTAGTTTGTGCTGTT | 8 | 0.353 | 0.003885292 | 0.446 | 0.003851599 | 0.032 |
| hsa-miR-101-3p | TACAGTACTGTGATAACTGAA | 9 | 0.873 | 0.027996513 | 1.397 | 0.252164314 | 0.036 |

**Table 1B: Post-transcriptional modification to the canonical sequence of listed microRNAs; type of 3'-end NTA is Uracil (U)**

| microRNA | canonical (mature) sequence in miRBase | SEQ: | C/V NTA-U in healthy | STD healthy | C/V NTA-U in cancer | STD cancer | p-value |
|---|---|---|---|---|---|---|---|
| hsa-miR-200c-3p | TAATACTGCCGGGTAATGATGGA | 10 | 0.392 | 0.015845068 | 0.218 | 0.005483231 | 0.019 |
| hsa-miR-26a-5p | TTCAAGTAATCCAGGATAGGCT | 11 | 0.194 | 6.53E-04 | 0.141 | 0.00150199 | 0.021 |
| hsa-let-7i-5p | TGAGGTAGTAGTTTGTGCTGTT | 12 | 0.162 | 0.001185664 | 0.109 | 0.00104543 | 0.024 |
| hsa-miR-320a | AAAAGCTGGGTTGAGAGGGCGA | 13 | 1.465 | 0.267284889 | 2.321 | 0.435442895 | 0.034 |
| hsa-miR-182-5p | TTTGGCAATGGTAGAACTCACACT | 14 | 0.104 | 2.44E-04 | 0.084 | 1.60E-04 | 0.043 |

**Table 1C: Post-transcriptional modification to the canonical sequence of listed microRNAs; type of 3'-end NTA is Cytosine (C)**

| microRNA | canonical (mature) sequence in miRBase | SEQ: | C/V NTA-C in healthy | STD healthy | C/ NTA-C in cancer | STD cancer | p-value |
|---|---|---|---|---|---|---|---|

| microRNA | canonical (mature) sequence in miRBase | SEQ: | C/V NTA-C in healthy | STD healthy | C/ NTA-C in cancer | STD cancer | p-value |
|---|---|---|---|---|---|---|---|
| hsa-miR-204-5p | TTCCCTTTGTCATCCTATGCCT | 15 | 0.005 | 5.58E-06 | 0.002 | 7.98E-07 | 0.005 |
| hsa-miR-101-3p | TACAGTACTGTGATAACTGAA | 16 | 0.478 | 5.49E-02 | 0.779 | 2.91E-02 | 0.013 |
| hsa-miR-200c-3p | TAATACTGCCGGGTAATGATGGA | 17 | 0.004 | 3.51E-05 | 0.022 | 2.98E-04 | 0.019 |
| hsa-let-7b-5p | TGAGGTAGTAGGTTGTGTGGTT | 18 | 0.018 | 3.98E-05 | 0.038 | 4.14E-04 | 0.020 |

**Table 1D: Post-transcriptional modification to the canonical sequence of listed microRNAs; type of 3'-end NTA is Guanine (G)**

| microRNA | canonical (mature) sequence in miRBase | SEQ: | C/V NTA-G in healthy | STD healthy | C/V NTA-G in cancer | STD cancer | p-value |
|---|---|---|---|---|---|---|---|
| hsa-let-7f-5p | TGAGGTAGTAGATTGTATAGTT | 19 | 0.009 | 2.40E-06 | 0.006 | 1.78E-06 | 0.005 |
| hsa-miR-10b-5p | TACCCTGTAGAACCGAATTTGTG | 20 | 0.112 | 3.79E-04 | 0.072 | 6.31E-04 | 0.013 |
| hsa-let-7e-5p | TGAGGTAGGAGGTTGTATAGTT | 21 | 0.006 | 3.70E-05 | 0.016 | 2.99E-05 | 0.019 |
| hsa-miR-30b-5p | TGTAAACATCCTACACTCAGCT | 22 | 0.023 | 3.48E-05 | 0.012 | 7.39E-05 | 0.020 |
| hsa-miR-26a-5p | TTCAAGTAATCCAGGATAGGCT | 23 | 0.019 | 4.43E-06 | 0.014 | 1.63E-05 | 0.026 |
| hsa-miR-10a-5p | TACCCTGTAGATCCGAATTTGTG | 24 | 0.124 | 0.001207764 | 0.077 | 5.78E-04 | 0.027 |
| hsa-miR-186-5p | CAAAGAATTCTCCTTTTGGGCT | 25 | 0.036 | 7.91E-05 | 0.018 | 2.25E-04 | 0.035 |
| hsa-miR-320a | AAAAGCTGGGTTGAGAGGGCGA | 26 | 0.041 | 6.29E-04 | 0.094 | 0.002206116 | 0.035 |
| hsa-miR-125a-5p | TCCCTGAGACCCTTTAACCTGTGA | 27 | 0.449 | 0.044536121 | 0.224 | 0.0014339 | 0.037 |

**Table 1E: Post-transcriptional processing of the canonical sequence of listed microRNA defined as 3'-end trimmed variants**

| microRNA | canonical (mature) sequence in miRBase | SEQ: | C/V 3'-end in healthy | STD healthy | C/V 3'-end in cancer | STD cancer | p-value |
|---|---|---|---|---|---|---|---|
| hsa-let-7f-5p | TGAGGTAGTAGATTGTATAGTT | 28 | 0.662 | 0.005028739 | 0.481 | 0.00305487 | 0.001 |
| hsa-miR-181b-5p | AACATTCATTGCTGTCGGTGGGT | 29 | 2.109 | 0.213109599 | 1.372 | 0.080779922 | 0.010 |
| hsa-miR-22-3p | AAGCTGCCAGTTGAAGAACTGT | 30 | 0.056 | 1.99E-04 | 0.111 | 0.001843821 | 0.013 |
| hsa-miR-28-3p | CACTAGATTGTGAGCTCCTGGA | 31 | 0.240 | 0.003029377 | 0.158 | 0.001208369 | 0.014 |
| hsa-miR-320a | AAAAGCTGGGTTGAGAGGGCGA | 32 | 0.757 | 0.062649693 | 1.323 | 0.159792031 | 0.016 |
| hsa-miR-30e-3p | CTTTCAGTCGGATGTTTACAGC | 33 | 0.172 | 0.006654715 | 0.074 | 2.05E-04 | 0.022 |
| hsa-miR-125a-5p | TCCCTGAGACCCTTTAACCTGTGA | 34 | 10.797 | 6.750391196 | 7.273 | 4.116960925 | 0.031 |

| | | | | | | |
|---|---|---|---|---|---|---|
| hsa-let-7d-5p | AGAGGTAGTAGGTTGCATAGTT | 35 | 0.876 | 0.032562757 | 0.660 | 0.010621945 | 0.036 |
| hsa-miR-192-5p | CTGACCTATGAATTGACAGCC | 36 | 0.064 | 2.62E-04 | 0.045 | 8.41E-05 | 0.043 |
| hsa-let-7i-5p | TGAGGTAGTAGTTTGTGCTGTT | 37 | 0.213 | 6.60E-04 | 0.180 | 4.91E-04 | 0.044 |
| hsa-let-7e-5p | TGAGGTAGGAGGTTGTATAGTT | 38 | 0.789 | 0.020725976 | 1.054 | 0.060355799 | 0.049 |

**Table 1F: Post-transcriptional processing of the canonical sequence of listed microRNA defined as 5'-end trimmed variants**

| microRNA | canonical (mature) sequence in miRBase | SEQ: | C/V 5'-end in healthy | STD healthy | C/V 5'-end in cancer | STD cancer | p-value |
|---|---|---|---|---|---|---|---|
| hsa-miR-181b-5p | AACATTCATTGCTGTCGGTGGGT | 39 | 0.126 | 0.006865806 | 0.017 | 2.09E-04 | 0.014 |
| hsa-miR-320a | AAAAGCTGGGTTGAGAGGGCGA | 40 | 0.043 | 1.93E-04 | 0.020 | 1.59E-04 | 0.015 |
| hsa-miR-10b-5p | TACCCTGTAGAACCGAATTTGTG | 41 | 0.289 | 3.50E-04 | 0.248 | 7.83E-04 | 0.015 |

Figure 13

A      Imaging

PET      PET/CT

cHL

B      Molecular Blood test

miRNAs in tumor vesicle

cHL

active tumor vesicle secretion

100nm

Normal

EP 3 137 626 B1

Figure 14

Figure 15

A

miRNA profile in exosomes define origin

B

Top 10 most abundant miRNA
species as determined by RNAseq

| Platelets | PBMCs | plasma vesicles |
|-----------|-------|-----------------|
| miR-142-3p | miR-223 | miR-486-5p |
| miR-223 | miR-150 | miR-92a-3p |
| miR-26a | miR-146b | let-7a-5p |
| miR-126-5p | miR-16-5p | miR-191-5p |
| miR-16-5p | miR-484 | let-7f-5p |
| miR-92a-3p | miR-146a | miR-26a-5p |
| miR-21 | miR-191-5p | miR-423-5p |
| miR-103 | miR-26a | miR-126-5p |
| miR-20a+b | miR-19b | miR-22-3p |
| let-7a | miR-20a | miR-181a-5p |

C

Hodgkin lymphoma
plasma vesicles >
Healthy individual
plasma vesicles

Hodgkin lymphoma cell line
exosomes
> healthy individual plasma
vesicles

Stroma

let-7a-5p
miR-1908-5p
miR-5189-5p
miR-122-5p

miR-92b-3p
miR-425-3p
miR-625-3p
miR-7706
miR-125b-5p
miR-760
miR-21-5p

Reed-Sternberg
miR-891a-5p
miR-155-5p
miR-129-5p
miR-182-5p
miR-1246
miR-320b
miR-127
miR-9-5p*
miR-769-5p
miR-193b-3p
miR-146b-3p

D

miR21-5p
P=0.027

let7a-5p
P=0.013

miR127-3p
ns

miR155-5p
P=0.022

miR1973
ns

EP 3 137 626 B1

Figure 16

Figure 17

Figure 18

Figure 19

## Figure 20

**hsa-miR-92a-3p**                    **In cells**

GTATTGCACTTGTCCCGGCCTGTTGAGTTTGG

TATTGCACTTGTCCCGGCCTGT                40%    Mature - canonical

                                      60%    IsomiRs

TATTGCACTTGTCCCGGCCTGT-A
TATTGCACTTGTCCCGGCCTGT-AA            34%     Enzymatic
TATTGCACTTGTCCCGGCCTGT-AAA                   modification
TATTGCACTTGTCCCGGCCTGT-TT             5%
TATTGCACTTGTCCCGGCCTGT-C              0%
TATTGCACTTGTCCCGGCCTGT-G              2%

TATTGCACTTGTCCCGGCCTGTTGA      3′    18%     Elongated &
TATTGCACTTGTCCCGGCC***                       truncated isomiRs -
GTATTGCACTTGTCCCGGCCTGT        5′    1%      canonical
*ATTGCACTTGTCCCGGCCTGT

Figure 21

**Table 2**

Post-transcriptional modification identified at the 3'-end of processed fragments originating from small non-coding RNA (ncRNAs) that are not of microRNA origin. The list of non-coding RNAs (from which modified fragments originate) identified by small RNA sequencing to be present in extracellular vesicles extracted from urine of healthy donors and prostate cancer (PCa) patients.

MF/TF – modified fragments/total fragments with sequences mapped to full length small ncRNA

Table 2A: Healthy vs Prostate Cancer; Post-transcriptional modification to the small RNA fragments with sequence identified to map to listed human tRNAs; type of 3'-end NTA is Adenine (A)

| The origin of mapped fragments | MF/TF 3'-end in healthy | STD healthy | MF/TF 3'-end in PCa | STD PCa | p-value |
|---|---|---|---|---|---|
| Homo_sapiens_chr6.trna100-LeuCAA | 0.005433993 | 1.73716E-06 | 0.000919334 | 3.38615E-06 | 0.0003 |
| Homo_sapiens_chr6.trna140-LeuCAA | 0.00538329 | 2.1043E-06 | 0.00094207 | 3.58969E-06 | 0.0006 |
| Homo_sapiens_chr6.trna74-LeuCAA | 0.00587606 | 2.29786E-06 | 0.001290749 | 3.8016E-06 | 0.0006 |
| Homo_sapiens_chr1.trna117-GlyTCC | 0.008568215 | 3.13574E-05 | 0.00039968 | 1.27795E-06 | 0.0016 |
| Homo_sapiens_chr1.trna66-LeuCAA | 0.000718331 | 1.93549E-07 | 7.83576E-05 | 4.91193E-08 | 0.0042 |
| Homo_sapiens_chr11.trna12-ProTGG | 0.013247561 | 3.52197E-05 | 0.004255265 | 1.63389E-05 | 0.0063 |
| Homo_sapiens_chr17.trna36-ThrAGT | 0.026963595 | 0.000555546 | 0 | 0 | 0.007 |
| Homo_sapiens_chr19.trna13-ValCAC | 0.014579569 | 0.000105725 | 0.002090942 | 1.00602E-05 | 0.007 |
| Homo_sapiens_chr16.trna18-GlyGCC | 0.000594112 | 2.08521E-08 | 0.000368039 | 1.4217E-08 | 0.009 |
| Homo_sapiens_chr1.trna68-GlyGCC | 0.00059877 | 2.22291E-08 | 0.000370691 | 1.45797E-08 | 0.0096 |
| Homo_sapiens_chr11.trna17-ValTAC | 0.003495544 | 1.03955E-05 | 0 | 0 | 0.0097 |

Table 2B: Prostate Cancer (PCa) vs Prostate Cancer related Metastasis (PCaM); Post-transcriptional modification to the small RNA fragments with sequence identified to map to listed human tRNAs; type of 3'-end NTA is Adenine (A)

| The origin of mapped fragments | MF/TF 3'-end in PCa | STD PCa | MF/TF 3'-end in PCaM | STD PCaM | p-value |
|---|---|---|---|---|---|
| Homo_sapiens_chr15.trna7-GlnCTG | 0.003705204 | 7.64119E-07 | 0.001180306 | 7.07327E-07 | 0.042 |
| Homo_sapiens_chr6.trna10-AlaCGC | 0.001846208 | 7.18883E-07 | 8.05932E-05 | 1.29905E-08 | 0.043 |
| Homo_sapiens_chr6.trna119-AlaCGC | 0.001845559 | 7.18964E-07 | 8.04182E-05 | 1.29342E-08 | 0.043 |

EP 3 137 626 B1

Table 2C: Healthy donors' vs High Risk Prostate Cancer patients (HRPC); Post-transcriptional modification to the small RNA fragments with sequence identified to map to listed human tRNAs; type of 3'-end NTA is Adenine (A)

| The origin of mapped fragments | MF/TF 3'-end in healthy | STD Healthy | MF/TF 3'-end in HRPC | STD HRPC | p-value |
|---|---|---|---|---|---|
| Homo_sapiens_chr6.trna100-LeuCAA | 0.005433993 | 1.73716E-06 | 0 | 0 | 0.00041 |
| Homo_sapiens_chr6.trna140-LeuCAA | 0.00538329 | 2.1043E-06 | 0 | 0 | 0.00076 |
| Homo_sapiens_chr6.trna74-LeuCAA | 0.00587606 | 2.29786E-06 | 0.000896057 | 1.60584E-06 | 0.0035 |

Table 2D: Healthy donors' vs High Risk Prostate Cancer patients (HRPC); Post-transcriptional modification to the small RNA fragments with sequence identified to map to listed human Y RNA; type of 3'-end NTA is Adenine (A)

| The origin of mapped fragments | MF/TF 3'-end in healthy | STD Healthy | MF/TF 3'-end in HRPC | STD HRPC | p-value |
|---|---|---|---|---|---|
| NR_004392: Homo-sapiens Y_RNA (RNY3) | 0.000688287 | 4.08821E-07 | 0.002027774 | 1.30598E-08 | 0.016 |

**Table 3**

SEQ: SEQ ID NO.

List of microRNA sequences presented in figure 15 (cHL)

| microRNA name | canonical (mature) sequence in miRBase | SEQ |
|---|---|---|
| hsa-let-7a-5p | TGAGGTAGTAGGTTGTATAGTT | 42 |
| hsa-miR-1908-5p | CGGCGGGGACGGCGATTGGTC | 43 |
| hsa-miR-5189-5p | TCTGGGCACAGGCGGATGGACAGG | 44 |
| hsa-miR-92b-3p | TATTGCACTCGTCCCGGCCTCC | 45 |
| hsa-miR-425-3p | ATCGGGAATGTCGTGTCCGCCC | 46 |
| hsa-miR-625-3p | GACTATAGAACTTTCCCCCTCA | 47 |
| hsa-miR-7706 | TGAAGCGCCTGTGCTCTGCCGAGA | 48 |
| hsa-miR-125b-5p | TCCCTGAGACCCTAACTTGTGA | 49 |
| hsa-miR-760 | CGGCTCTGGGTCTGTGGGGA | 50 |
| hsa-miR-21-5p | TAGCTTATCAGACTGATGTTGA | 51 |
| hsa-miR-122-5p | TGGAGTGTGACAATGGTGTTTG | 52 |
| hsa-miR-891a-5p | TGCAACGAACCTGAGCCACTGA | 53 |
| hsa-miR-155-5p | TTAATGCTAATCGTGATAGGGGT | 54 |
| hsa-miR-129-5p | CTTTTTGCGGTCTGGGCTTGC | 55 |
| hsa-miR-182-5p | TTTGGCAATGGTAGAACTCACACT | 56 |
| hsa-miR-1246 | AATGGATTTTTGGAGCAGG | 57 |
| hsa-miR-320b | AAAAGCTGGGTTGAGAGGGCAA | 58 |
| hsa-miR-127-3p | TCGGATCCGTCTGAGCTTGGCT | 59 |
| hsa-miR-9-5p | TCTTTGGTTATCTAGCTGTATGA | 60 |
| hsa-miR-769-5p | TGAGACCTCTGGGTTCTGAGCT | 61 |
| hsa-miR-193b-3p | AACTGGCCCTCAAAGTCCCGCT | 62 |
| hsa-miR-146b-3p | TGCCCTGTGGACTCAGTTCTGG | 63 |

**Table 4**

Post-transcriptionally modified microRNAs identified by small RNA sequencing to be present in extracellular vesicles extracted from blood plasma of healthy donors and cHL patients

V/C – variant/canonical

SEQ: SEQ ID NO.

Table 4A: Post-transcriptional modification to the canonical sequence of listed microRNAs; type of 3'-end NTA is Adenine (A)

| microRNA | Canonical (mature) sequence in miRBase | SEQ | V/C 3'-end in healthy | STD healthy | V/C 3'-end in cHL | STD cHL | p-value |
|---|---|---|---|---|---|---|---|
| hsa-let-7f-5p | UGAGGUAGUAGAUUGUAUAGUU | 64 | 0.050906394 | 9.98949E-05 | 0.029019947 | 8.41686E-05 | 0.031 |

Table 4B: Post-transcriptional modification to the canonical sequence of listed microRNAs; type of 3'-end NTA is Uracil (U)

| microRNA | Canonical (mature) sequence in miRBase | SEQ | V/C 3'-end in healthy | STD healthy | V/C 3'-end in cHL | STD cHL | p-value |
|---|---|---|---|---|---|---|---|
| hsa-miR-486-5p | UCCUGUACUGAGCUGCCCCGAG | 65 | 0.223592359 | 0.004284728 | 0.733684288 | 0.047373483 | 0.008 |

Table 4C: Post-transcriptional processing of the canonical sequence of listed microRNAs defined as 3'-end length variants

| microRNA | Canonical (mature) sequence in miRBase | SEQ | V/C 3'-end in healthy | STD healthy | V/C 3'-end in cHL | STD cHL | p-value |
|---|---|---|---|---|---|---|---|
| hsa-miR-122-5p | UGGAGUGUGACAAUGGUGUUUG | 66 | 0.0375 | 0.00421875 | 0.165542416 | 0.001179908 | 0.023 |

Table 4D: Post-transcriptional processing of the canonical sequence of listed microRNAs defined as 5'-end length variants

| microRNA | Canonical (mature) sequence in miRBase | SEQ | V/C 5'-end in healthy | STD healthy | V/C 5'-end in cHL | STD cHL | p-value |
|---|---|---|---|---|---|---|---|
| hsa-miR-92b-3p | UAUUGCACUCGUCCCGGCCUCC | 67 | 0.002392344 | 1.71699E-05 | 0.011509417 | 3.09912E-06 | 0.013 |

## Table 5

Post-transcriptionally modified microRNAs identified by small RNA sequencing to be present in protein fraction (PF) extracted from blood plasma of healthy donors and cHL patients

V/C – variant/canonical

SEQ: SEQ ID NO.

Table 5A: Post-transcriptional modification to the canonical sequence of listed microRNAs; type of 3'-end NTA is Adenine (A)

| microRNA | Canonical (mature) sequence in miRBase | SEQ | V/C 3'-end in healthy (PF) | STD healthy (PF) | V/C 3'-end in cHL (PF) | STD cHL (PF) | p-value |
|---|---|---|---|---|---|---|---|
| hsa-let-7g-5p | UGAGGUAGUAGUUUGUACAGUU | 68 | 0.358532 | 0.0122 | 0.0278 | 0.002314815 | 0.0063 |

Table 5B: Post-transcriptional processing of the canonical sequence of listed microRNAs defined as 3'-end length variants

| microRNA | Canonical (mature) sequence in miRBase | SEQ | V/C 3'-end in healthy (PF) | STD healthy (PF) | V/C 3'-end in cHL (PF) | STD cHL (PF) | p-value |
|---|---|---|---|---|---|---|---|
| hsa-miR-151a-3p | CUAGACUGAAGCUCCUUGAGG | 69 | 0.26948 | 0.00022 | 0.02053 | 0.00044 | 0.000025 |
| hsa-miR-423-3p | AGCUCGGUCUGAGGCCCCUCAGU | 70 | 1.92327 | 0.13555 | 0.26997 | 0.03743 | 0.001296 |
| hsa-miR-128-3p | UCACAGUGAACCGGUCUCUUU | 71 | 9.53030 | 5.95638 | 0.50000 | 0.75000 | 0.002168 |

Table 5C: Post-transcriptional processing of the canonical sequence of listed microRNAs defined as 5'-end length variants

| microRNA | Canonical (mature) sequence in miRBase | SEQ | V/C 5'-end in healthy (PF) | STD healthy (PF) | V/C 5'-end in cHL (PF) | STD cHL (PF) | p-value |
|---|---|---|---|---|---|---|---|
| hsa-miR-126-5p | CAUUAUUACUUUUGGUACGCG | 72 | 0.0072699 | 0.0000014 | 0.0014457 | 0.0000022 | 0.0051 |
| hsa-miR-26a-5p | UUCAAGUAAUCCAGGAUAGGCU | 73 | 0.0129995 | 0.0000249 | 0.0004513 | 0.0000006 | 0.0086 |

## Table 6

Post-transcriptionally modified microRNAs identified by small RNA sequencing to be present in blood serum of breast cancer patients

Publicly available data; source of information: http://www.ncbi.nlm.nih.gov/sra?term=SRP027589

BioProject description: http://www.ncbi.nlm.nih.gov/bioproject?LinkName=sra_bioproject&from_uid=455584

V/C – variant/canonical

SEQ: SEQ ID NO.

Table 6.1: Comparison between samples from patients diagnosed with stage II and samples from patients diagnosed with stage III breast cancer

Table 6.1A: Post-transcriptional modification to the canonical sequence of listed microRNAs; type of 3'-end NTA is Adenine (A)

| microRNA | Canonical (mature) sequence in miRBase | SEQ | V/C 3'-end in stage II | STD stage II | V/C 3'-end in stage III | STD stage III | p-value |
|---|---|---|---|---|---|---|---|
| hsa-miR-190b | UGAUAUGUUUGAUAUUGGGUU | 74 | 0.538961039 | 0.554140665 | 2.900793651 | 4.237843285 | 0.001 |
| hsa-miR-542-5p | UCGGGGAUCAUCAGUCACGAGA | 75 | 0.016189037 | 0.000566183 | 0.000661376 | 8.74836E-06 | 0.008 |
| hsa-miR-200a-3p | UAACACUGUCUGGUAACGAUGU | 76 | 0.058696802 | 0.003456404 | 0.328946527 | 0.09544156 | 0.009 |

Table 6.1B: Post-transcriptional modification to the canonical sequence of listed microRNAs; type of 3'-end NTA is Uracil (U)

| microRNA | Canonical (mature) sequence in miRBase | SEQ | V/C 3'-end in stage II | STD stage II | V/C 3'-end in stage III | STD stage III | p-value |
|---|---|---|---|---|---|---|---|
| hsa-miR-3913-5p | UUUGGGACUGAUCUUGAUGUCU | 77 | 0.266666667 | 0.118181818 | 0.021385747 | 0.002031877 | 0.004 |
| hsa-miR-22-3p | AAGCUGCCAGUUGAAGAACUGU | 78 | 0.008633074 | 6.58976E-06 | 0.006072176 | 4.71662E-06 | 0.007 |

Table 6.1C: Post-transcriptional processing of the canonical sequence of listed microRNAs defined as 3'-end length variants

| microRNA | Canonical (mature) sequence in miRBase | SEQ | V/C 3'-end in stage II | STD stage II | V/C 3'-end in stage III | STD stage III | p-value |
|---|---|---|---|---|---|---|---|
| hsa-miR-190a-5p | UGAUAUGUUUGAUAUAUUAGGU | 79 | 28.67188405 | 362.637024 | 63.51078902 | 1833.272742 | 0.018 |

Table 6.1D: Post-transcriptional processing of the canonical sequence of listed microRNAs defined as 5'-end length variants

| microRNA | Canonical (mature) sequence in miRBase | SEQ | V/C 5'-end in stage II | STD stage II | V/C 5'-end in stage III | STD stage III | p-value |
|---|---|---|---|---|---|---|---|
| hsa-miR-130b-3p | CAGUGCAAUGAUGAAAGGGCAU | 80 | 0.021607478 | 0.000142081 | 0.04436697 | 0.000299859 | 0.0007 |
| hsa-miR-4772-5p | UGAUCAGGCAAAAUUGCAGACU | 81 | 0.212121212 | 0.111570248 | 0.004329004 | 0.000374806 | 0.009 |

Table 6.2: Comparison between samples from patients with breast cancer relapse and samples from patients without relapse

Table 6.2A: Post-transcriptional modification to the canonical sequence of listed microRNAs; type of 3'-end NTA is Adenine (A)

| microRNA | Canonical (mature) sequence in miRBase | SEQ | V/C 3'-end in relapse | STD relapse | V/C 3'-end in no-relapse | STD no-relapse | p-value |
|---|---|---|---|---|---|---|---|
| hsa-miR-424-5p | CAGCAGCAAUUCAUGUUUUGAA | 82 | 0.134256332 | 0.007704542 | 0.325896169 | 0.057635652 | 0.003 |
| hsa-miR-3138 | UGUGGACAGUGAGGUAGAGGGAGU | 83 | 1.629166667 | 10.65826968 | 9.4375 | 116.7148438 | 0.005 |

Table 6.2B: Post-transcriptional modification to the canonical sequence of listed microRNAs; type of 3'-end NTA is Uracil (U)

| microRNA | Canonical (mature) sequence in miRBase | SEQ | V/C 3'-end in relapse | STD relapse | V/C 3'-end in no-relapse | STD no-relapse | p-value |
|---|---|---|---|---|---|---|---|
| hsa-miR-342-3p | UCUCACACAGAAAUCGCACCCGU | 84 | 0.268790118 | 0.015569342 | 0.567696558 | 0.121362044 | 0.002 |
| hsa-miR-6891-5p | UAAGGAGGGGGAUGAGGGG | 85 | 0.023313492 | 0.004068395 | 0.26875 | 0.111835938 | 0.003 |
| hsa-miR-1307-3p | ACUCGGCGUGGCGUCGGUCGUG | 86 | 2.977727863 | 0.481034278 | 3.843716593 | 0.451959336 | 0.006 |
| hsa-miR-125b-2-3p | UCACAAGUCAGGCUCUUGGGAC | 87 | 2.845622896 | 4.045451355 | 5.646329365 | 7.881816254 | 0.006 |
| hsa-miR-6763-5p | CUGGGGAGUGGCUGGGGAG | 88 | 0.027777778 | 0.017746914 | 0.395833333 | 0.346788194 | 0.009 |

Table 6.2C: Post-transcriptional processing of the canonical sequence of listed microRNAs defined as 3'-end length variants

| microRNA | Canonical (mature) sequence in miRBase | SEQ | V/C 3'-end in relapse | STD relapse | V/C 3'-end in no-relapse | STD no-relapse | p-value |
|---|---|---|---|---|---|---|---|
| hsa-miR-6885-5p | AGGGGGGCACUGCGCAAGCAAAGCC | 89 | 0.131944444 | 0.178192515 | 1.125 | 0.961875 | 0.0006 |

Table 6.2D: Post-transcriptional processing of the canonical sequence of listed microRNAs defined as 5'-end length variants

| microRNA | Canonical (mature) sequence in miRBase | SEQ | V/C 5'-end in relapse | STD relapse | V/C 5'-end in no-relapse | STD no-relapse | p-value |
|---|---|---|---|---|---|---|---|
| hsa-miR-3140-3p | AGCUUUUGGGAAUUCAGGUAGU | 90 | 0.011904762 | 0.003259637 | 0.416666667 | 0.354166667 | 0.003 |
| hsa-miR-125b-2-3p | UCACAAGUCAGGCUCUUGGGAC | 91 | 9.904273781 | 43.68473804 | 21.76339286 | 183.645156 | 0.004 |
| hsa-miR-6866-5p | UUAGAGGCUGGAAUAGAGAUUCU | 92 | 0.016666667 | 0.006388889 | 0.395833333 | 0.305121528 | 0.004 |
| hsa-miR-574-3p | CACGCUCAUGCACACACCCACA | 93 | 0.029712394 | 0.000448329 | 0.006994901 | 7.90746E-05 | 0.008 |
| hsa-miR-4786-5p | UGAGACCAGGACUGGAUGCACC | 94 | 0.015046296 | 0.002892179 | 0.133333333 | 0.03 | 0.008 |

## Table 7

Post-transcriptionally modified microRNAs identified by small RNA sequencing to be present in cancer and matched adjacent tissues from testicular germ cell tumors.

Publicly available data; source of information: http://www.ncbi.nlm.nih.gov/sra?term=SRP007946

BioProject description: http://www.ncbi.nlm.nih.gov/bioproject/154993

V/C – variant/canonical

SEQ: SEQ ID NO.

Table 7: Comparison between samples from matched adjacent tissue (MAT) and samples from testicular germ cell tumors (TGCT).

Table 7A: Post-transcriptional modification to the canonical sequence of listed microRNAs; type of 3'-end NTA is Adenine (A)

| microRNA | Canonical (mature) sequence in miRBase | SEQ | V/C 3'-end in MAT | STD MAT | V/C 3'-end in TGCT | STD TGCT | p-value |
|---|---|---|---|---|---|---|---|
| hsa-miR-3605-5p | UGAGGAUGGAUAGCAAGGAAGCC | 95 | 0.428571429 | 0.259007307 | 2.555555556 | 1.061728395 | 0.0045 |
| hsa-miR-326 | CCUCUGGGCCCUUCCUCCAG | 96 | 1.666666667 | 8.285714286 | 10.66666667 | 10.88888889 | 0.0047 |
| hsa-miR-502-3p | AAUGCACCUGGGCAAGGAUUCA | 97 | 0.050357934 | 0.004477513 | 0.358608059 | 0.030093252 | 0.0068 |
| hsa-miR-1268a | CGGGCGUGGUGGUGGGGG | 98 | 0.004959177 | 3.9299E-05 | 0.043611351 | 0.000643095 | 0.0097 |

Table 7B: Post-transcriptional modification to the canonical sequence of listed microRNAs; type of 3'-end NTA is Uracil (U)

| microRNA | Canonical (mature) sequence in miRBase | SEQ | V/C 3'-end in MAT | STD MAT | V/C 3'-end in TGCT | STD TGCT | p-value |
|---|---|---|---|---|---|---|---|
| hsa-miR-409-5p | AGGUUACCCGAGCAACUUUGCAU | 99 | 0.002172732 | 2.83246E-05 | 0.031630033 | 5.14855E-05 | 0.0002 |
| hsa-miR-371a-3p | AAGUGCCGCCAUCUUUUGAGUGU | 100 | 0.002577951 | 2.21826E-05 | 0.022585955 | 1.11613E-05 | 0.0003 |
| hsa-miR-199a-5p | CCCAGUGUUCAGACUACCUGUUC | 101 | 0.124929366 | 0.025715835 | 0.844444444 | 0.048395062 | 0.0009 |
| hsa-miR-181a-3p | ACCAUCGACCGUUGAUUGUACC | 102 | 0.499329846 | 0.231621215 | 1.73452381 | 0.20524093 | 0.0096 |

Table 7C: Post-transcriptional processing of the canonical sequence of listed microRNAs defined as 3'-end length variants

| microRNA | Canonical (mature) sequence in miRBase | SEQ | V/C 3'-end in MAT | STD MAT | V/C 3'-end in TGCT | STD TGCT | p-value |
|---|---|---|---|---|---|---|---|
| hsa-miR-22-3p | AAGCUGCCAGUUGAAGAACUGU | 103 | 0.507196851 | 0.168230816 | 2.269696961 | 0.217292737 | 0.00069 |
| hsa-miR-455-3p | GCAGUCCAUGGGCAUAUACAC | 104 | 1.403043323 | 1.168225589 | 5.161494253 | 3.251471132 | 0.0066 |
| hsa-miR-520a-3p | AAAGUGCUUCCCUUUGGACUGU | 105 | 0.54821091 | 0.287516172 | 2.24311491 | 0.74309101 | 0.0097 |

Table 7D: Post-transcriptional processing of the canonical sequence of listed microRNAs defined as 5'-end length variants

| microRNA | Canonical (mature) sequence in miRBase | SEQ | V/C 5'-end in MAT | STD MAT | V/C 5'-end in TGCT | STD TGCT | p-value |
|---|---|---|---|---|---|---|---|
| hsa-miR-130b-3p | CAGUGCAAUGAUGAAAGGGCAU | 106 | 0.003446228 | 2.00865E-05 | 0.035700044 | 4.83825E-06 | 0.000005 |
| hsa-miR-3605-5p | UGAGGAUGGAUAGCAAGGAAGCC | 107 | 0.031746032 | 0.002519526 | 1.888888889 | 0.913580247 | 0.0018 |
| hsa-miR-92a-1-5p | AGGUUGGGAUCGGUUGCAAUGCU | 108 | 0.000228739 | 1.86729E-07 | 0.002357306 | 1.05838E-06 | 0.0033 |

**Table 8**

Post-transcriptionally modified microRNAs identified by small RNA sequencing to be expressed in paired colorectal normal, tumor and metastasis tissues.

Publicly available data; source of information: http://www.ncbi.nlm.nih.gov/Traces/sra/?study=SRP022054

BioProject description: http://www.ncbi.nlm.nih.gov/bioproject/PRJNA201245

V/C – variant/canonical

SEQ: SEQ ID NO.

Table 8.1: Comparison between samples from paired colorectal normal tissue (CNT) and colorectal tumor tissue (CTT).

Table 8.1A: Post-transcriptional modification to the canonical sequence of listed microRNAs; type of 3'-end NTA is Uracil (U)

| microRNA | Canonical (mature) sequence in miRBase | SEQ | V/C 3'-end in CNT | STD CNT | V/C 3'-end in CTT | STD CTT | p-value |
|---|---|---|---|---|---|---|---|
| hsa-miR-874-3p | CUGCCCUGGCCCGAGGGACCGA | 109 | 0.397582973 | 0.036348183 | 0.149046167 | 0.015221961 | 0.014 |

Table 8.1B: Post-transcriptional processing of the canonical sequence of listed microRNAs defined as 3'-end length variants

| microRNA | Canonical (mature) sequence in miRBase | SEQ | V/C 3'-end in CNT | STD CNT | V/C 3'-end in CTT | STD CTT | p-value |
|---|---|---|---|---|---|---|---|
| hsa-miR-1266-5p | CCUCAGGGCUGUAGAACAGGGCU | 110 | 0.461788212 | 0.087526184 | 0.1625 | 0.017190689 | 0.032 |

Table 8.1C: Post-transcriptional processing of the canonical sequence of listed microRNAs defined as 5'-end length variants

| microRNA | Canonical (mature) sequence in miRBase | SEQ | V/C 5'-end in CNT | STD CNT | V/C 5'-end in CTT | STD CTT | p-value |
|---|---|---|---|---|---|---|---|
| hsa-miR-125b-2-3p | CUGCCCUGGCCCGAGGGACCGA | 111 | 4.571428571 | 17.67346939 | 0.5 | 1.75 | 0.031 |

Table 8.2: Comparison between samples from paired colorectal normal tissue (CNT) and colorectal tumor metastasis (CTM).

Table 8.2A: Post-transcriptional modification to the canonical sequence of listed microRNAs; type of 3'-end NTA is Adenine (A)

| microRNA | Canonical (mature) sequence in miRBase | SEQ | V/C 3'-end in CNT | STD CNT | V/C 3'-end in CTM | STD CTM | p-value |
|---|---|---|---|---|---|---|---|
| hsa-miR-135b-5p | UAUGGCUUUCAUUCCUAUGUGA | 112 | 0.021601547 | 0.001258247 | 0.085917908 | 0.001505237 | 0.011 |

Table 8.2B: Post-transcriptional modification to the canonical sequence of listed microRNAs; type of 3'-end NTA is Uracil (U)

| microRNA | Canonical (mature) sequence in miRBase | SEQ | V/C 3'-end in CNT | STD CNT | V/C 3'-end in CTM | STD CTM | p-value |
|---|---|---|---|---|---|---|---|
| hsa-miR-301a-5p | GCUCUGACUUUAUUGCACUACU | 113 | 0.034094806 | 0.001045648 | 0.112977142 | 0.004842371 | 0.027 |

Table 8.2C: Post-transcriptional processing of the canonical sequence of listed microRNAs defined as 3'-end length variants

| microRNA | Canonical (mature) sequence in miRBase | SEQ | V/C 3'-end in CNT | STD CNT | V/C 3'-end in CTM | STD CTM | p-value |
|---|---|---|---|---|---|---|---|
| hsa-miR-154-3p | AAUCAUACACGGUUGACCUAUU | 114 | 0.150361953 | 0.015317714 | 0.385300931 | 0.03527946 | 0.025 |

Table 8.2D: Post-transcriptional processing of the canonical sequence of listed microRNAs defined as 5'-end length variants

| microRNA | Canonical (mature) sequence in miRBase | SEQ | V/C 5'-end in CNT | STD CNT | V/C 5'-end in CTM | STD CTM | p-value |
|---|---|---|---|---|---|---|---|
| hsa-miR-191-3p | GCUGCGCUUGGAUUUCGUCCCC | 115 | 0.346250684 | 0.123168635 | 1.176190476 | 0.614829932 | 0.036 |

Table 8.3: Comparison between samples from paired colorectal tumor tissue (CTT) and colorectal tumor metastasis (CTM).

Table 8.3A: Post-transcriptional modification to the canonical sequence of listed microRNAs; type of 3'-end NTA is Adenine (A)

| microRNA | Canonical (mature) sequence in miRBase | SEQ | V/C 3'-end in CTT | STD CNT | V/C 3'-end in CTM | STD CTM | p-value |
|---|---|---|---|---|---|---|---|
| hsa-miR-212-5p | ACCUUGGCUCUAGACUGCUUACU | 116 | 0.034779745 | 0.001324047 | 0.096377613 | 0.002490405 | 0.024 |

Table 8.3B: Post-transcriptional modification to the canonical sequence of listed microRNAs; type of 3'-end NTA is Uracil (U)

| microRNA | Canonical (mature) sequence in miRBase | SEQ | V/C 3'-end in CTT | STD CNT | V/C 3'-end in CTM | STD CTM | p-value |
|---|---|---|---|---|---|---|---|
| hsa-miR-30c-1-3p | CUGGGAGAGGGUUGUUUACUCC | 117 | 0.111805556 | 0.010535783 | 0.642393321 | 0.22787036 | 0.014 |

Table 8.3C: Post-transcriptional processing of the canonical sequence of listed microRNAs defined as 3'-end length variants

| microRNA | Canonical (mature) sequence in miRBase | SEQ | V/C 3'-end in CTT | STD CNT | V/C 3'-end in CTM | STD CTM | p-value |
|---|---|---|---|---|---|---|---|
| hsa-miR-30c-1-3p | CUGGGAGAGGGUUGUUUACUCC | 118 | 2.278472222 | 2.807179302 | 5.563388992 | 8.243342134 | 0.024 |

Table 8.3D: Post-transcriptional processing of the canonical sequence of listed microRNAs defined as 5'-end length variants

| microRNA | Canonical (mature) sequence in miRBase | SEQ | V/C 5'-end in CTT | STD CNT | V/C 5'-end in CTM | STD CTM | p-value |
|---|---|---|---|---|---|---|---|
| hsa-miR-324-3p | ACUGCCCCAGGUGCUGCUGG | 119 | 0.425 | 0.214375 | 3.247252747 | 3.993177153 | 0.0032 |

## Table 9

Post-transcriptionally modified microRNAs identified by small RNA sequencing to be present in the blood samples from healthy donors and in the blood samples from Alzheimer's patients.

Publicly available data; source of information: http://www.ncbi.nlm.nih.gov/sra?term=SRP022043

BioProject description: http://www.ncbi.nlm.nih.gov/bioproject?LinkName=sra_bioproject&from_uid=387967

V/C – variant/canonical

SEQ: SEQ ID NO.

Table 9A: Post-transcriptional modification to the canonical sequence of listed microRNAs; type of 3'-end NTA is Adenine (A)

| microRNA | Canonical (mature) sequence in miRBase | SEQ | V/C 3'-end in healthy | STD healthy | V/C 3'-end in Alzheimer | STD Alzheimer | p-value |
|---|---|---|---|---|---|---|---|
| hsa-miR-27b-3p | UUCACAGUGGCUAAGUUCUGC | 120 | 0.411732 | 0.089286 | 0.117006 | 0.008993 | 0.00000006 |
| hsa-miR-378a-3p | ACUGGACUUGGAGUCAGAAGGC | 121 | 1.599882 | 0.344643 | 0.971829 | 0.104322 | 0.0000003 |
| hsa-miR-26a-5p | UUCAAGUAAUCCAGGAUAGGCU | 122 | 0.013759 | 0.000088 | 0.007026 | 0.000005 | 0.00002 |
| hsa-miR-140-3p | UACCACAGGGUAGAACCACGG | 123 | 96.360846 | 10796.7899 | 29.302897 | 103.5106 | 0.00005 |
| hsa-miR-16-5p | UAGCAGCACGUAAAUAUUGGCG | 124 | 0.003587 | 0.000006 | 0.001915 | 0.000001 | 0.00006 |
| hsa-miR-186-5p | CAAAGAAUUCUCCUUUUGGGCU | 125 | 0.021162 | 0.000270 | 0.011080 | 0.000025 | 0.0003 |
| hsa-miR-30e-3p | CUUUCAGUCGGAUGUUUACAGC | 126 | 0.299693 | 0.104876 | 0.113701 | 0.004868 | 0.0004 |
| hsa-miR-25-3p | CAUUGCACUUGUCUCGGUCUGA | 127 | 0.036016 | 0.000094 | 0.029075 | 0.000043 | 0.0009 |
| hsa-miR-532-5p | CAUGCCUUGAGUGUAGGACCGU | 128 | 2.167682 | 1.661605 | 3.223986 | 1.263253 | 0.001 |
| hsa-miR-584-5p | UUAUGGUUUGCCUGGGACUGAG | 129 | 2.926921 | 4.255546 | 1.538687 | 1.593786 | 0.001 |
| hsa-miR-103a-3p | AGCAGCAUUGUACAGGGCUAUGA | 130 | 0.038029 | 0.000411 | 0.025135 | 0.000160 | 0.002 |
| hsa-miR-146a-5p | UGAGAACUGAAUUCCAUGGGUU | 131 | 0.229589 | 0.011971 | 0.161576 | 0.004596 | 0.003 |

Table 9B: Post-transcriptional modification to the canonical sequence of listed microRNAs; type of 3'-end NTA is Uracil (U)

| microRNA | Canonical (mature) sequence in miRBase | SEQ | V/C 3'-end in healthy | STD healthy | V/C 3'-end in Alzheimer | STD Alzheimer | p-value |
|---|---|---|---|---|---|---|---|
| hsa-miR-30e-3p | CUUUCAGUCGGAUGUUUACAGC | 132 | 1.7051821 | 1.3926408 | 0.6879818 | 0.3964491 | 0.0000174 |
| hsa-miR-26a-5p | UUCAAGUAAUCCAGGAUAGGCU | 134 | 0.0167074 | 0.0000738 | 0.0097654 | 0.0000198 | 0.0000416 |
| hsa-miR-18a-3p | ACUGCCCUAAGUGCUCCUUCUGG | 135 | 2.2783845 | 1.7943275 | 1.2244586 | 0.5932116 | 0.0001145 |

EP 3 137 626 B1

| | | | | | | | |
|---|---|---|---|---|---|---|---|
| hsa-miR-22-3p | AAGCUGCCAGUUGAAGAACUGU | 136 | 0.0055188 | 0.0000715 | 0.0005070 | 0.0000002 | 0.0001415 |
| hsa-miR-423-3p | AGCUCGGUCUGAGGCCCCUCAGU | 137 | 0.1311864 | 0.0005699 | 0.1042053 | 0.0007957 | 0.0002756 |
| hsa-miR-151a-3p | CUAGACUGAAGCUCCUUGAGG | 138 | 0.8932042 | 0.1497361 | 1.2017309 | 0.0911402 | 0.0006641 |
| hsa-miR-636 | UGUGCUUGCUCGUCCGCCCGCA | 139 | 0.4686133 | 0.0820729 | 0.2967185 | 0.0182500 | 0.0012733 |
| hsa-miR-28-3p | CACUAGAUUGUGAGCUCCUGGA | 140 | 0.0788520 | 0.0014452 | 0.0567764 | 0.0002994 | 0.0015863 |
| hsa-miR-361-3p | UCCCCCAGGUGUGAUUCUGAUUU | 141 | 0.2222736 | 0.0350978 | 0.5103031 | 0.1481984 | 0.0016134 |
| hsa-miR-484 | UCAGGCUCAGUCCCCUCCGAU | 142 | 0.3331470 | 0.0036495 | 0.2711522 | 0.0061310 | 0.0018272 |
| hsa-miR-378a-3p | ACUGGACUUGGAGUCAGAAGGC | 143 | 0.8906028 | 0.1047794 | 0.6547852 | 0.0670671 | 0.0020061 |
| hsa-miR-328-3p | CUGGCCCUCUCUGCCCUUCCGU | 144 | 2.3156467 | 0.5040896 | 2.9737738 | 0.6961762 | 0.0023580 |

Table 9C: Post-transcriptional processing of the canonical sequence of listed microRNAs defined as 3'-end length variants

| microRNA | Canonical (mature) sequence in miRBase | SEQ | V/C 3'-end in healthy | STD healthy | V/C 3'-end in Alzheimer | STD Alzheimer | p-value |
|---|---|---|---|---|---|---|---|
| hsa-miR-378a-3p | ACUGGACUUGGAGUCAGAAGGC | 145 | 1.36139 | 0.43052 | 0.69854 | 0.09961 | 0.0000004 |
| hsa-miR-15b-5p | UAGCAGCACAUCAUGGUUUACA | 146 | 0.34916 | 0.03394 | 0.18470 | 0.00879 | 0.0000069 |
| hsa-miR-183-5p | UAUGGCACUGGUAGAAUUCACU | 147 | 1.11501 | 0.38349 | 0.64397 | 0.03574 | 0.0000115 |
| hsa-miR-18a-3p | ACUGCCCUAAGUGCUCCUUCUGG | 148 | 29.20176 | 414.25342 | 13.90097 | 38.81686 | 0.0000143 |
| hsa-miR-652-3p | AAUGGCGCCACUAGGGUUGUG | 149 | 22.13222 | 233.07454 | 9.70611 | 44.40786 | 0.0000146 |
| hsa-miR-23b-3p | AUCACAUUGCCAGGGAUUACC | 150 | 13.70508 | 55.58031 | 7.08323 | 19.10053 | 0.0000200 |
| hsa-miR-27b-3p | UUCACAGUGGCUAAGUUCUGC | 151 | 14.22932 | 72.03828 | 7.50809 | 13.29942 | 0.0000215 |
| hsa-miR-182-5p | UUUGGCAAUGGUAGAACUCACACU | 152 | 2.12943 | 0.22529 | 1.61001 | 0.19789 | 0.0000427 |
| hsa-miR-140-3p | UACCACAGGGUAGAACCACGG | 153 | 44.42029 | 609.92163 | 26.79512 | 90.11594 | 0.0000696 |
| hsa-miR-501-3p | AAUGCACCCGGGCAAGGAUUCU | 154 | 1.97822 | 1.26168 | 1.18843 | 0.27984 | 0.0001938 |
| hsa-miR-30e-3p | CUUUCAGUCGGAUGUUUACAGC | 156 | 0.79301 | 0.31273 | 0.34567 | 0.13538 | 0.0002103 |
| hsa-miR-7706 | UGAAGCGCCUGUGCUCUGCCGAGA | 157 | 0.83893 | 0.14441 | 0.55197 | 0.04900 | 0.0002165 |
| hsa-miR-192-5p | CUGACCUAUGAAUUGACAGCC | 158 | 0.08491 | 0.00212 | 0.05356 | 0.00044 | 0.0002584 |
| hsa-miR-502-3p | AAUGCACCUGGGCAAGGAUUCA | 159 | 11.55906 | 90.82373 | 4.87880 | 24.68940 | 0.0003196 |
| hsa-miR-30d-5p | UGUAAACAUCCCCGACUGGAAG | 160 | 61.98171 | 941.7383 | 106.3779 | 2719.06939 | 0.0004995 |
| hsa-miR-324-3p | ACUGCCCCAGGUGCUGCUGG | 161 | 23.27968 | 1110.61079 | 5.59967 | 9.18954 | 0.0006186 |
| hsa-miR-148a-3p | UCAGUGCACUACAGAACUUUGU | 162 | 0.79164 | 0.26883 | 0.48802 | 0.02995 | 0.0006263 |
| hsa-miR-23a-3p | AUCACAUUGCCAGGGAUUUCC | 163 | 3.47593 | 4.37419 | 1.93477 | 2.08603 | 0.0007687 |
| hsa-miR-423-3p | AGCUCGGUCUGAGGCCCCUCAGU | 164 | 1.31323 | 0.51447 | 0.82346 | 0.19659 | 0.0009879 |
| hsa-mir-1273c-3p | CAGAGUCUCGUUCUGUUGCCCA | 165 | 21.08865 | 446.58500 | 43.98982 | 744.30891 | 0.0010086 |
| hsa-miR-941 | CACCCGGCUGUGUGCACAUGUGC | 166 | 3.17051 | 4.85542 | 1.91735 | 0.83898 | 0.0014978 |
| hsa-miR-28-3p | CACUAGAUUGUGAGCUCCUGGA | 167 | 0.12458 | 0.00200 | 0.09833 | 0.00055 | 0.0022918 |

| hsa-let-7b-5p | UGAGGUAGUAGGUUGUGUGGUU | 168 | 1.33049 | 0.06605 | 1.12210 | 0.06223 | 0.0023040 |
| hsa-miR-19b-3p | UGUGCAAAUCCAUGCAAAACUGA | 169 | 14.17067 | 243.07727 | 6.27938 | 23.69134 | 0.0025107 |
| hsa-miR-326 | CCUCUGGGCCCUUCCUCCAG | 170 | 1.63637 | 3.37525 | 0.61730 | 0.96690 | 0.0040963 |

Table 9D: Post-transcriptional processing of the canonical sequence of listed microRNAs defined as 5'-end length variants

| microRNA | Canonical (mature) sequence in miRBase | SEQ | V/C 5'-end in healthy | STD healthy | V/C 5'-end in Alzheimer | STD Alzheimer | p-value |
|---|---|---|---|---|---|---|---|
| hsa-miR-192-5p | CUGACCUAUGAAUUGACAGCC | 171 | 0.453893 | 0.024577 | 0.274961 | 0.004938 | 0.000000011 |
| hsa-miR-181a-5p | AACAUUCAACGCUGUCGGUGAGU | 172 | 0.004378 | 0.000007 | 0.002355 | 0.000003 | 0.000236507 |

## Table 10

**microRNA length variants** identified by small RNA sequencing to be present in extracellular vesicles (EV) extracted from blood plasma of healthy donors and cHL patients

V/C – variant/canonical

SEQ: SEQ ID NO.

Table 10A: Post-transcriptional processing of the canonical sequence of listed microRNAs defined as 3'-end length elongated/extended (E) variants

| microRNA | Canonical (mature) sequence in miRBase | SEQ | V/C 3'-end in healthy (EV) | STD healthy (EV) | V/C 3'-end in cHL (EV) | STD cHL (EV) | p-value |
|---|---|---|---|---|---|---|---|
| hsa-miR-486-5p | UCCUGUACUGAGCUGCCCCGAG | 173 | 2.41E-04 | 1.16E-08 | 0.001798348 | 6.69E-07 | 0.0171 |
| hsa-let-7b-5p | UGAGGUAGUAGGUUGUGUGGUU | 174 | 0.324870615 | 0.014219315 | 0.600290274 | 0.013733156 | 0.0291 |

Table 10B: Post-transcriptional processing of the canonical sequence of listed microRNAs defined as 3'-end length truncated/trimmed (T) variants

| microRNA | Canonical (mature) sequence in miRBase | SEQ | V/C 3'-end in healthy (EV) | STD healthy (EV) | V/C 3'-end in cHL (EV) | STD cHL (EV) | p-value |
|---|---|---|---|---|---|---|---|
| hsa-miR-99b-5p | CACCCGUAGAACCGACCUUGCG | 175 | 0.662985403 | 0.030698002 | 0.351704768 | 0.005615789 | 0.03 |
| hsa-let-7d-5p | UGAGGUAGUAGGUUGUGUGGUU | 176 | 0.227261558 | 0.009220008 | 0.071317471 | 2.96E-04 | 0.033 |
| hsa-miR-92a-3p | UAUUGCACUUGUCCCGGCCUGU | 177 | 0.368502769 | 0.019848131 | 0.126572457 | 0.004359178 | 0.04 |
| hsa-let-7g-5p | UGAGGUAGUAGUUUGUACAGUU | 178 | 0.608337676 | 0.121128655 | 0.085777775 | 8.71E-04 | 0.04 |

Table 10C: Post-transcriptional processing of the canonical sequence of listed microRNAs defined as 5'-end length elongated/extended (E) variants

| microRNA | Canonical (mature) sequence in miRBase | SEQ | V/C 5'-end in healthy (EV) | STD healthy (EV) | V/C 5'-end in cHL (EV) | STD cHL (EV) | p-value |
|---|---|---|---|---|---|---|---|
| hsa-miR-92b-3p | UAUUGCACUCGUCCCGGCCUCC | 179 | 0.002392344 | 1.71699E-05 | 0.009532972 | 9.49554E-06 | 0.054 |

Table 10D: Post-transcriptional processing of the canonical sequence of listed microRNAs defined as 5'-end length truncated/ trimmed (T) variants

| microRNA | Canonical (mature) sequence in miRBase | SEQ | V/C 5'-end in healthy (EV) | STD healthy (EV) | V/C 5'-end in cHL (EV) | STD cHL (EV) | p-value |
|---|---|---|---|---|---|---|---|
| hsa-miR-126-5p | CAUUAUUACUUUUGGUACGCG | 180 | 0.00280261 | 1.06E-06 | 0.001029033 | 3.83E-07 | 0.043 |

**Table 11**

**microRNA length variants** identified by small RNA sequencing to be present in protein fraction (PF) extracted from blood plasma of healthy donors and cHl patients.

V/C – variant/canonical

SEQ: SEQ ID NO.

Table 11A: Post-transcriptional processing of the canonical sequence of listed microRNAs defined as 3'-end length elongated/extended (E) variants

| microRNA | Canonical (mature) sequence in miRBase | SEQ | V/C 3'-end in healthy (PF) | STD healthy (PF) | V/C 3'-end in cHL (PF) | STD cHL (PF) | p-value |
|---|---|---|---|---|---|---|---|
| hsa-miR-423-5p | UGAGGGGCAGAGAGCGAGACUUU | 181 | 0.050775911 | 2.92E-06 | 0.171743333 | 0.005530906 | 0.063 |

Table 11B: Post-transcriptional processing of the canonical sequence of listed microRNAs defined as 3'-end length truncated/trimmed (T) variants

| microRNA | Canonical (mature) sequence in miRBase | SEQ | V/C 3'-end in healthy (PF) | STD healthy (PF) | V/C 3'-end in cHL (PF) | STD cHL (PF) | p-value |
|---|---|---|---|---|---|---|---|
| hsa-miR-151a-3p | CUAGACUGAAGCUCCUUGAGG | 182 | 0.269480519 | 2.18E-04 | 0.020525452 | 0.000443 | 0.00003 |
| hsa-miR-423-3p | AGCUCGGUCUGAGGCCCCUCAGU | 183 | 1.923265196 | 0.135546516 | 0.269970275 | 0.037429256 | 0.002 |
| hsa-miR-128-3p | UCACAGUGAACCGGUCUCUUU | 184 | 9.5 | 6.166666667 | 0.428571429 | 0.551020408 | 0.0021 |
| hsa-miR-21-5p | UAGCUUAUCAGACUGAUGUUGA | 185 | 0.319106352 | 0.00263648 | 0.151896475 | 0.002396259 | 0.014 |
| hsa-miR-151a-5p | UCGAGGAGCUCACAGUCUAGU | 186 | 0.397849462 | 0.029099917 | 0.047120329 | 1.16E-04 | 0.018 |
| hsa-miR-142-5p | CAUAAAGUAGAAAGCACUACU | 187 | 2.005847953 | 0.488372491 | 0.323284823 | 0.158653576 | 0.0190 |
| hsa-miR-23a-3p | AUCACAUUGCCAGGGAUUUCC | 188 | 0.937289562 | 0.063561045 | 0.390106421 | 0.01061652 | 0.021 |
| hsa-let-7f-5p | UGAGGUAGUAGAUUGUAUAGUU | 189 | 3.089779417 | 2.341480252 | 0.275624934 | 0.021345504 | 0.027 |
| hsa-miR-22-3p | AAGCUGCCAGUUGAAGAACUGU | 190 | 0.273738589 | 2.56E-04 | 0.127430857 | 0.004773519 | 0.029 |
| hsa-miR-150-5p | UCUCCCAACCCUUGUACCAGUG | 191 | 1.458585859 | 0.150702989 | 0.365384615 | 0.169748521 | 0.029 |
| hsa-miR-423-5p | UGAGGGGCAGAGAGCGAGACUUU | 192 | 3.696142706 | 0.209072521 | 1.671846007 | 0.988592573 | 0.04 |
| hsa-miR-345-5p | GCUGACUCCUAGUCCAGGGCUC | 193 | 35.66666667 | 326.8888889 | 3.75 | 42.1875 | 0.04 |
| hsa-let-7i-5p | UGAGGUAGUAGUUUGUGCUGUU | 194 | 4.13263914 | 4.058191286 | 0.656464646 | 0.417524365 | 0.04 |
| hsa-miR-181a-5p | AACAUUCAACGCUGUCGGUGAGU | 195 | 7.250148851 | 6.193580602 | 2.207675439 | 2.877194281 | 0.04 |

Table 11C: Post-transcriptional processing of the canonical sequence of listed microRNAs defined as 5'-end length truncated/ trimmed (T) variants

| microRNA | Canonical (mature) sequence in miRBase | SEQ | V/C 5'-end in healthy (EV) | STD healthy (EV) | V/C 5'-end in cHL (EV) | STD cHL (EV) | p-value |
|---|---|---|---|---|---|---|---|
| hsa-miR-126-5p | CAUUAUUACUUUUGGUACGCG | 196 | 0.007269881 | 0.0000014 | 0.0012361 | 0.0000018 | 0.004 |
| hsa-miR-26a-5p | UUCAAGUAAUCCAGGAUAGGCU | 197 | 0.012999534 | 0.0000249 | 0.0004513 | 0.0000006 | 0.009 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011024157 A
- US 20120322691 A
- WO 2009099905 A

- WO 2011025919 A
- WO 2013003350 A
- US 2012289420 A

### Non-patent literature cited in the description

- **LI et al.** *BMC Genomics,* 2012, vol. 13, S1-S13
- **LEUNG et al.** *Oncology Reports,* 2014, vol. 31, 1067-1078
- **HORI et al.** *Science Transl Med,* 2013
- **BOING et al.** *JEV,* 2014
- **ARROYO et al.** *PNAS,* 2011
- **PLE et al.** *PLoSone,* 2012
- **OSANTO, S. et al.** *PLoS One,* 2012
- **HACKENBERG, M. et al.** *Nucleic Acid Res.,* 2009
- **D'AMBROGIO et al.** *Cell Reports,* 2012
- **RÖHR C et al.** High-throughput miRNA and mRNA sequencing of paired colorectal normal, tumor and metastasis tissues and bioinformatic modeling of miRNA-1 therapeutic applications. *PLoS One,* 02 July 2013, vol. 8 (7), e67461
- **LEIDINGER P et al.** A blood based 12-miRNA signature of Alzheimer disease patients. *Genome Biol,* 29 July 2013, vol. 14 (7), R78
- **AMARAL, P.P. ; DINGER, M.E. ; MERCER, T.R. ; MATTICK, J.S.** The eukaryotic genome as an RNA machine. *Science,* 2008, vol. 319, 1787-1789
- **AMERES, S.L. ; ZAMORE, P.D.** Diversifying micro-RNA sequence and function. *Nat. Rev. Mol. Cell Biol.,* 2013, vol. 14, 475-488
- **AMERES, S.L. ; MARTINEZ, J. ; SCHROEDER, R.** Molecular basis for target RNA recognition and cleavage by human RISC. *Cell,* 2007, vol. 130, 101-112
- **AMERES, S.L., HORWICH, M.D., HUNG, J.-H., XU, J., GHILDIYAL, M., WENG, Z. ZAMORE, P.D.** Target RNA-directed trimming and tailing of small silencing RNAs. *Science,* 2010, vol. 328, 1534-1539
- **BACCARINI, A. ; CHAUHAN, H. ; GARDNER, T.J. ; JAYAPRAKASH, A.D. ; SACHIDANANDAM, R. ; BROWN, B.D.** Kinetic analysis reveals the fate of a microRNA following target regulation in mammalian cells. *Curr. Biol.,* 2011, vol. 21, 369-376

- **BACKES, S. ; SHAPIRO, J.S. ; SABIN, L.R. ; PHAM, A.M. ; REYES, I. ; MOSS, B. ; CHERRY, S. ; TENOEVER, B.R.** Degradation of host microRNAs by poxvirus poly(A) polymerase reveals terminal RNA methylation as a protective antiviral mechanism. *Cell Host Microbe,* 2012, vol. 12, 200-210
- **BALAJ, L. ; LESSARD, R. ; DAI, L. ; CHO, Y.-J. ; POMEROY, S.L. ; BREAKEFIELD, X.O. ; SKOG, J.** Tumour microvesicles contain retrotransposon elements and amplified oncogene sequences. *Nat. Commun,* 2011, vol. 2, 180
- **VAN BALKOM, B.W.M. ; DE JONG, O.G. ; SMITS, M. ; BRUMMELMAN, J. ; DEN OUDEN, K. ; DE BREE, P.M. ; VAN EIJNDHOVEN, M.A.J. ; PEG-TEL, D.M. ; STOORVOGEL, W. ; WÜRDINGER, T. et al.** Endothelial cells require miR-214 to secrete exosomes that suppress senescence and induce angiogenesis in human and mouse endothelial cells. *Blood,* 2013, vol. 121 (3997-4006), 1-15
- **BELLINGHAM, S.A. ; COLEMAN, B.M. ; HILL, A.F.** Small RNA deep sequencing reveals a distinct miRNA signature released in exosomes from prion-infected neuronal cells. *Nucleic Acids Res.,* 2012, vol. 40, 10937-10949
- **BIJNSDORP, I. V ; GELDOF, A.A. ; LAVAEI, M. ; PIERSMA, S.R. ; VAN MOORSELAAR, R.J.A. ; JIMENEZ, C.R.** Exosomal ITGA3 interferes with non-cancerous prostate cell functions and is increased in urine exosomes of metastatic prostate cancer patients. *J. Extracell. Vesicles,* 2013, vol. 2
- **BURNS, D.M. ; D'AMBROGIO, A. ; NOTTROTT, S. ; RICHTER, J.D.** CPEB and two poly(A) polymerases control miR-122 stability and p53 mRNA translation. *Nature,* 2011, vol. 473, 105-108

- **BURROUGHS, A.M. ; ANDO, Y. ; DE HOON, M.J.L. ; TOMARU, Y. ; NISHIBU, T. ; UKEKAWA, R. ; FUNAKOSHI, T. ; KUROKAWA, T. ; SUZUKI, H. ; HAYASHIZAKI, Y. et al.** A comprehensive survey of 3' animal miRNA modification events and a possible role for 3' adenylation in modulating miRNA targeting effectiveness. *Genome Res.,* 2010, vol. 20, 1398-1410

- **CHAIROUNGDUA, A. ; SMITH, D.L. ; POCHARD, P. ; HULL, M. ; CAPLAN, M.J.** Exosome release of β-catenin: a novel mechanism that antagonizes Wnt signaling. *J. Cell Biol.,* 2010, vol. 190, 1079-1091

- **CHITWOOD, D.H. ; TIMMERMANS, M.C.P.** Small RNAs are on the move. *Nature,* 2010, vol. 467, 415-419

- **CHUMA, S. ; PILLAI, R.S.** Retrotransposon silencing by piRNAs: ping-pong players mark their sub-cellular boundaries. *PLoS Genet,* 2009, vol. 5, e1000770

- **CULLEN, B.R.** Viral and cellular messenger RNA targets of viral microRNAs. *Nature,* 2009, vol. 457, 421-425

- **D'AMBROGIO, A. ; GU, W. ; UDAGAWA, T. ; MELLO, C.C. ; RICHTER, J.D.** Specific miRNA stabilization by Gld2-catalyzed monoadenylation. *Cell Rep.,* 2012, vol. 2, 1537-1545

- **EBERT, M.S. ; SHARP, P.A.** Roles for microRNAs in conferring robustness to biological processes. *Cell,* 2012, vol. 149, 515-524

- **FEEDERLE, R. ; HAAR, J. ; BERNHARDT, K. ; LINNSTAEDT, S.D. ; BANNERT, H. ; LIPS, H. ; CULLEN, B.R. ; DELECLUSE, H.-J.** The members of an Epstein-Barr virus microRNA cluster cooperate to transform B lymphocytes. *J. Virol.,* 2011, vol. 85, 9801-9810

- **FERNANDEZ-VALVERDE, S.L. ; TAFT, R.J. ; MATTICK, J.S.** Dynamic isomiR regulation in Drosophila development. *RNA,* 2010, vol. 16, 1881-1888

- **GARCIA, E.L. ; ONAFUWA-NUGA, A. ; SIM, S. ; KING, S.R. ; WOLIN, S.L. ; TELESNITSKY, A.** Packaging of host mY RNAs by murine leukemia virus may occur early in Y RNA biogenesis. *J. Virol.,* 2009, vol. 83, 12526-12534

- **GIBBINGS, D.J. ; CIAUDO, C. ; ERHARDT, M. ; VOINNET, O.** Multivesicular bodies associate with components of miRNA effector complexes and modulate miRNA activity. *Nat. Cell Biol.,* 2009, vol. 11, 1143-1149

- **GUASPARRI, I. ; BUBMAN, D. ; CESARMAN, E.** EBV LMP2A affects LMP1-mediated NF-kappaB signaling and survival of lymphoma cells by regulating TRAF2 expression. *Blood,* 2008, vol. 111, 3813-3820

- **GUDURIC-FUCHS, J. ; O'CONNOR, A. ; CAMP, B. ; O'NEILL, C.L. ; MEDINA, R.J. ; SIMPSON, D.A.** Selective extracellular vesicle-mediated export of an overlapping set of microRNAs from multiple cell types. *BMC Genomics,* 2012, vol. 13, 357

- **GUO, L. ; YANG, Q. ; LU, J. ; LI, H. ; GE, Q. ; GU, W. ; BAI, Y. ; LU, Z.** A comprehensive survey of miRNA repertoire and 3' addition events in the placentas of patients with pre-eclampsia from high-throughput sequencing. *PLoS One,* 2011, vol. 6, e21072

- **JONES, M.R. ; QUINTON, L.J. ; BLAHNA, M.T. ; NEILSON, J.R. ; FU, S. ; IVANOV, A.R. ; WOLF, D.A. ; MIZGERD, J.P.** Zcchc11-dependent uridylation of microRNA directs cytokine expression. *Nat. Cell Biol.,* 2009, vol. 11, 1157-1163

- **KAI, Z.S. ; PASQUINELLI, A.E.** MicroRNA assassins: factors that regulate the disappearance of miRNAs. *Nat. Struct. Mol. Biol.,* 2010, vol. 17, 5-10

- **KATOH, T. ; SAKAGUCHI, Y. ; MIYAUCHI, K. ; SUZUKI, T. ; KASHIWABARA, S.-I. ; BABA, T. ; SUZUKI, T.** Selective stabilization of mammalian microRNAs by 3' adenylation mediated by the cytoplasmic poly(A) polymerase GLD-2. *Genes Dev.,* 2009, vol. 23, 433-438

- **KHAN, M.A. ; GOILA-GAUR, R. ; OPI, S. ; MIYAGI, E. ; TAKEUCHI, H. ; KAO, S. ; STREBEL, K.** Analysis of the contribution of cellular and viral RNA to the packaging of APOBEC3G into HIV-1 virions. *Retrovirology,* 2007, vol. 4, 48

- **KOPPERS-LALIC, D. ; HOGENBOOM, M.M. ; MIDDELDORP, J.M. ; PEGTEL, D.M.** Virus-modified exosomes for targeted rna delivery; A new approach in nanomedicine. *Adv. Drug Deliv. Rev.,* 2012

- **KOSAKA, N. ; IGUCHI, H. ; YOSHIOKA, Y. ; TAKESHITA, F. ; MATSUKI, Y. ; OCHIYA, T.** Secretory mechanisms and intercellular transfer of microRNAs in living cells. *J. Biol. Chem.,* 2010, vol. 285, 17442-17452

- **KOSAKA, N. ; IGUCHI, H. ; HAGIWARA, K. ; YOSHIOKA, Y. ; TAKESHITA, F. ; OCHIYA, T.** Neutral sphingomyelinase 2 (nSMase2)-dependent exosomal transfer of angiogenic microRNAs regulate cancer cell metastasis. *J. Biol. Chem.,* 2013, vol. 288, 10849-10859

- **LEE, Y.S. ; PRESSMAN, S. ; ANDRESS, A.P. ; KIM, K. ; WHITE, J.L. ; CASSIDY, J.J. ; LI, X. ; LUBELL, K. ; LIM, D.H. ; CHO, I.S. et al.** Silencing by small RNAs is linked to endosomal trafficking. *Nat. Cell Biol.,* 2009, vol. 11, 1150-1156

- **LEE, Y.S. ; SHIBATA, Y. ; MALHOTRA, A. ; DUTTA, A.** A novel class of small RNAs: tRNA-derived RNA fragments (tRFs). *Genes Dev.,* 2009, vol. 23, 2639-2649

- **LUJAMBIO, A. ; ESTELLER, M.** CpG island hypermethylation of tumor suppressor microRNAs in human cancer. *Cell Cycle,* 2007, vol. 6, 1455-1459

- **MARTENS-UZUNOVA, E.S. ; OLVEDY, M. ; JENSTER, G.** Beyond microRNA--novel RNAs derived from small non-coding RNA and their implication in cancer. *Cancer Lett.,* 2013, vol. 340, 201-211
- **MARTIN, G. ; KELLER, W.** RNA-specific ribonucleotidyl transferases. *RNA,* 2007, vol. 13, 1834-1849
- **MAUTE, R.L. ; SCHNEIDER, C. ; SUMAZIN, P. ; HOLMES, A. ; CALIFANO, A. ; BASSO, K. ; DALLA-FAVERA, R.** tRNA-derived microRNA modulates proliferation and the DNA damage response and is down-regulated in B cell lymphoma. *Proc. Natl. Acad. Sci. U. S. A.,* 2013, vol. 110, 1404-1409
- **MITTELBRUNN, M. ; SANCHEZ-MADRID, F.** Intercellular communication: diverse structures for exchange of genetic information. *Nat. Rev. Mol. Cell Biol.,* 2012, vol. 13, 328-335
- **MITTELBRUNN, M. ; GUTIÉRREZ-VÁZQUEZ, C. ; VILLARROYA-BELTRI, C. ; GONZALEZ, S. ; SANCHEZ-CABO, F. ; GONZALEZ, M.A. ; BERNAD, A. ; SANCHEZ-MADRID, F.** Unidirectional transfer of microRNA-loaded exosomes from T cells to antigen-presenting cells. *Nat. Commun.,* 2011, vol. 2, 282
- **MONTECALVO, A. ; LARREGINA, A.T. ; SHUFESKY, W.J. ; STOLZ, D.B. ; SULLIVAN, M.L.G. ; KARLSSON, J.M. ; BATY, C.J. ; GIBSON, G.A. ; ERDOS, G. ; WANG, Z. et al.** Mechanism of transfer of functional microRNAs between mouse dendritic cells via exosomes. *Blood,* 2012, vol. 119, 756-766
- **MORIN, R.D. ; O'CONNOR, M.D. ; GRIFFITH, M. ; KUCHENBAUER, F. ; DELANEY, A. ; PRABHU, A.-L. ; ZHAO, Y. ; MCDONALD, H. ; ZENG, T. ; HIRST, M. et al.** Application of massively parallel sequencing to microRNA profiling and discovery in human embryonic stem cells. *Genome Res.,* 2008, vol. 18, 610-621
- **MULLOKANDOV, G. ; BACCARINI, A. ; RUZO, A. ; JAYAPRAKASH, A.D. ; TUNG, N. ; ISRAELOW, B. ; EVANS, M.J. ; SACHIDANANDAM, R. ; BROWN, B.D.** High-throughput assessment of microRNA activity and function using microRNA sensor and decoy libraries. *Nat. Methods,* 2012, vol. 9, 840-846
- **NOLTE-'T HOEN, E.N.M. ; BUERMANS, H.P.J. ; WAASDORP, M. ; STOORVOGEL, W. ; WAUBEN, M.H.M. ; T HOEN, P.A.C.** Deep sequencing of RNA from immune cell-derived vesicles uncovers the selective incorporation of small non-coding RNA biotypes with potential regulatory functions. *Nucleic Acids Res.,* 2012, vol. 40, 9272-9285
- **PALMA, J. ; YADDANAPUDI, S.C. ; PIGATI, L. ; HAVENS, M.A. ; JEONG, S. ; WEINER, G.A. ; WEIMER, K.M.E. ; STERN, B. ; HASTINGS, M.L. ; DUELLI, D.M.** MicroRNAs are exported from malignant cells in customized particles. *Nucleic Acids Res.,* 2012, vol. 40, 9125-9138
- **PAREKH, S. ; POLO, J.M. ; SHAKNOVICH, R. ; JUSZCZYNSKI, P. ; LEV, P. ; RANUNCOLO, S.M. ; YIN, Y. ; KLEIN, U. ; CATTORETTI, G. ; DALLA FAVERA, R. et al.** BCL6 programs lymphoma cells for survival and differentiation through distinct biochemical mechanisms. *Blood,* 2007, vol. 110, 2067-2074
- **PEGTEL, D.M. ; COSMOPOULOS, K. ; THORLEY-LAWSON, D.A. ; VAN EIJNDHOVEN, M.A.J. ; HOPMANS, E.S. ; LINDENBERG, J.L. ; DE GRUIJL, T.D. ; WÜRDINGER, T. ; MIDDELDORP, J.M.** Functional delivery of viral miRNAs via exosomes. *Proc. Natl. Acad. Sci. U. S. A.,* 2010, vol. 107, 6328-6333
- **PEGTEL, D.M. ; VAN DE GARDE, M.D.B. ; MIDDELDORP, J.M.** Viral miRNAs exploiting the endosomal-exosomal pathway for intercellular cross-talk and immune evasion. *Biochim. Biophys. Acta,* 2011, vol. 1809, 715-721
- **POLIKEPAHAD, S. ; CORRY, D.B.** Profiling of T helper cell-derived small RNAs reveals unique antisense transcripts and differential association of miRNAs with argonaute proteins 1 and 2. *Nucleic Acids Res.,* 2013, vol. 41, 1164-1177
- **QIU, J. ; COSMOPOULOS, K. ; PEGTEL, M. ; HOPMANS, E. ; MURRAY, P. ; MIDDELDORP, J. ; SHAPIRO, M. ; THORLEY-LAWSON, D.A.** A novel persistence associated EBV miRNA expression profile is disrupted in neoplasia. *PLoS Pathog,* 2011, vol. 7, e1002193
- **RILEY, K.J. ; RABINOWITZ, G.S. ; YARIO, T.A. ; LUNA, J.M. ; DARNELL, R.B. ; STEITZ, J.A.** EBV and human microRNAs co-target oncogenic and apoptotic viral and human genes during latency. *EMBO J.,* 2012, vol. 31, 2207-2221
- **ROBINSON, M.D. ; MCCARTHY, D.J. ; SMYTH, G.K.** edgeR: a Bioconductor package for differential expression analysis of digital gene expression data. *Bioinformatics,* 2010, vol. 26, 139-140
- **ROUTH, A. ; DOMITROVIC, T. ; JOHNSON, J.E.** Host RNAs, including transposons, are encapsidated by a eukaryotic single-stranded RNA virus. *Proc. Natl. Acad. Sci. U. S. A.,* 2012, vol. 109, 1907-1912
- **RÜEGGER, S. ; GROßHANS, H.** MicroRNA turnover: when, how, and why. *Trends Biochem. Sci.,* 2012, vol. 37, 436-446
- **SAITO, Y. ; LIANG, G. ; EGGER, G. ; FRIEDMAN, J.M. ; CHUANG, J.C. ; COETZEE, G.A. ; JONES, P.A.** Specific activation of microRNA-127 with downregulation of the proto-oncogene BCL6 by chromatin-modifying drugs in human cancer cells. *Cancer Cell,* 2006, vol. 9, 435-443
- **SCOTT, D.D. ; NORBURY, C.J.** RNA decay via 3' uridylation. *Biochim. Biophys. Acta,* vol. 1829, 654-665

- **SCOTT, D.D. ; NORBURY, C.J.** RNA decay via 3' uridylation. *Biochim. Biophys. Acta - Gene Regul. Mech.,* 2013, vol. 1829, 654-665
- **SETO, E. ; MOOSMANN, A. ; GRÖMMINGER, S. ; WALZ, N. ; GRUNDHOFF, A. ; HAMMERSCHMIDT, W.** Micro RNAs of Epstein-Barr virus promote cell cycle progression and prevent apoptosis of primary human B cells. *PLoS Pathog,* 2010, vol. 6, e1001063
- **SIM, S. ; WEINBERG, D.E. ; FUCHS, G. ; CHOI, K. ; CHUNG, J. ; WOLIN, S.L.** The subcellular distribution of an RNA quality control protein, the Ro autoantigen, is regulated by noncoding Y RNA binding. *Mol. Biol. Cell,* 2009, vol. 20, 1555-1564
- **SKALSKY, R.L., CORCORAN, D.L., GOTTWEIN, E., FRANK, C.L., KANG, D., HAFNER, M., NUSBAUM, J.D., FEEDERLE, R., DELECLUSE, H.-J., LUFTIG, M.A.** The viral and cellular microRNA targetome in lymphoblastoid cell lines. *PLoS Pathog.,* 2012, vol. 8, e1002484
- **SONG, L. ; LIN, C. ; GONG, H. ; WANG, C. ; LIU, L. ; WU, J. ; TAO, S. ; HU, B. ; CHENG, S.-Y. ; LI, M. et al.** miR-486 sustains NF-κB activity by disrupting multiple NF-κB-negative feedback loops. *Cell Res.,* 2013, vol. 23, 274-289
- **UMEZU, T. ; OHYASHIKI, K. ; KURODA, M. ; OHYASHIKI, J.H.** Leukemia cell to endothelial cell communication via exosomal miRNAs. *Oncogene,* 2013, vol. 32, 2747-2755
- **VEREIDE, D.T. ; SETO, E. ; CHIU, Y.-F. ; HAYES, M. ; TAGAWA, T. ; GRUNDHOFF, A. ; HAMMERSCHMIDT, W. ; SUGDEN, B.** Epstein-Barr virus maintains lymphomas via its miRNAs. *Oncogene,* 2013
- **VERWEIJ, F.J. ; VAN EIJNDHOVEN, M.A.J. ; MIDDELDORP, J. ; PEGTEL, D.M.** Analysis of viral microRNA exchange via exosomes in vitro and in vivo. *Methods Mol. Biol.,* 2013, vol. 1024, 53-68
- **VILLARROYA-BELTRI, C. ; GUTIÉRREZ-VÁZQUEZ, C. ; SANCHEZ-CABO, F. ; PÉREZ-HERNÁNDEZ, D. ; VÁZQUEZ, J. ; MARTIN-COFRECES, N. ; MARTINEZ-HERRERA, D.J. ; PASCUAL-MONTANO, A. ; MITTELBRUNN, M. ; SANCHEZ-MADRID, F.** Sumoylated hnRNPA2B1 controls the sorting of miRNAs into exosomes through binding to specific motifs. *Nat. Commun,* 2013, vol. 4, 2980
- **WEE, L.M. ; FLORES-JASSO, C.F. ; SALOMON, W.E. ; ZAMORE, P.D.** Argonaute divides its RNA guide into domains with distinct functions and RNA-binding properties. *Cell,* 2012, vol. 151, 1055-1067
- **WYMAN, S.K. ; KNOUF, E.C. ; PARKIN, R.K. ; FRITZ, B.R. ; LIN, D.W. ; DENNIS, L.M. ; KROUSE, M.A. ; WEBSTER, P.J. ; TEWARI, M.** Post-transcriptional generation of miRNA variants by multiple nucleotidyl transferases contributes to miRNA transcriptome complexity. *Genome Res.,* 2011, vol. 21, 1450-1461
- **YAO, B. ; LA, L.B. ; CHEN, Y.-C. ; CHANG, L.-J. ; CHAN, E.K.L.** Defining a new role of GW182 in maintaining miRNA stability. *EMBO Rep.,* 2012, vol. 13, 1102-1108
- **ZHANG, Y. ; LIU, D. ; CHEN, X. ; LI, J. ; LI, L. ; BIAN, Z. ; SUN, F. ; LU, J. ; YIN, Y. ; CAI, X. et al.** Secreted monocytic miR-150 enhances targeted endothelial cell migration. *Mol. Cell,* 2010, vol. 39, 133-144
- **ZHUANG, G. ; WU, X. ; JIANG, Z. ; KASMAN, I. ; YAO, J. ; GUAN, Y. ; OEH, J. ; MODRUSAN, Z. ; BAIS, C. ; SAMPATH, D. et al.** Tumour-secreted miR-9 promotes endothelial cell migration and angiogenesis by activating the JAK-STAT pathway. *EMBO J.,* 2012, vol. 31, 3513-3523